# EUROPEAN PATENT SPECIFICATION

(11) **EP 2 322 521 B1**
(45) Date of publication and mention of the grant of the patent: **04.09.2013**
(21) Application number: 10180554.7
(22) Date of filing: 06.02.2003
(51) Int. Cl.: C07D 413/04, C07D 413/14, C07D 403/04, C07D 409/04, C07D 401/04, C07D 235/18, C07D 471/04, C07D 487/04, C07D 491/04, A61K 31/4245, A61K 31/501

(54) **Heteroaryl compounds useful as inhibitors of GSK-3**
Heteroaryl-Verbindungen und Ihre Anwendung als GSK-3-Hemmer
Composés hétéroaryle utiles comme inhibiteurs de GSK-3

(30) Priority: 06.02.2002 US 354843 P
(43) Date of publication of application: 18.05.2011
(62) Divisional of application: 03710903.0
(73) Proprietor: Vertex Pharmaceuticals, Inc., Cambridge, MA 02139 (US)
(72) Inventor: Harbeson, Scott L., Cambridge, MA 02140 (US); Arnost, Michael J., North Andover, MA 01845 (US); Green, Jeremy, Rockville, MD 20850 (US); Savic, Vladimir, Essex, Essex CB10 1XL (GB)
(74) Representative: Cohausz & Florack

(56) References cited:
- EP-A- 1 136 099
- WO-A1-01/00610
- WO-A1-02/50073
- WO-A1-2004/103994
- WO-A2-02/088078
- US-A- 5 496 826
- DATABASE CHEMCATS [Online] XP002244560, retrieved from STN Database accession no. 2001:1365428 & "CATALOG: Screening Collection", 28 March 2000 (2000-03-28), ZELINSKY INSTITUTE, US * Order Number: A1438/0063775 *
- DATABASE CHEMCATS [Online] XP002244561, retrieved from STN Database accession no. 2001:1432097 & "CATALOG: Screening Collection", 28 March 2000 (2000-03-28), ZELINSKY INSTITUTE, US * Order Number: A2222/0093571 *
- DATABASE CHEMCATS [Online] XP002244562, retrieved from STN Database accession no. 2002:206442 & "CATALOG: LaboTest Stock", 2 January 2002 (2002-01-02), LABOTEST, DE * Order Number: LT00631882 *
- DATABASE CHEMCATS [Online] XP002244563, retrieved from STN Database accession no. 2002:1417269 & "CATALOG: LaboTest Stock", 2 January 2002 (2002-01-02), LABOTEST, DE * Order Number: LT01184225 *
- DATABASE CHEMCATS [Online] XP002244564, retrieved from STN Database accession no. 2002:616303 & "CATALOG: ChemBridge Product List", 17 January 2002 (2002-01-17), CHEMBRIDGE CORPORATION, US * Order Number: 6354773 *
- DATABASE CHEMCATS [Online] XP002244565, retrieved from STN Database accession no. 2001:1241574 & "CATALOG: Screening Collection", 28 March 2000 (2000-03-28), ZELINSKY INSTITUTE, US * Order Number: A2160/0090742 *
- DATABASE CHEMCATS [Online] XP002244566, retrieved from STN Database accession no. 2002:186549 & "CATALOG: LaboTest Stock", 2 January 2002 (2002-01-02), LABOTEST, DE * Order Number: LT00015966 *
- DATABASE CHEMCATS [Online] XP002244567, retrieved from STN Database accession no. 2002:2989359 & "CATALOG: ChemBridge Product List", 17 January 2002 (2002-01-17), CHEMBRIDGE CORPORATION, US * Order Number: 6621687 *
- DATABASE CHEMCATS [Online] XP002244568, retrieved from STN Database accession no. 2002:596566 & "CATALOG: Chembridge Product List", 17 January 2002 (2002-01-17), CHEMBRIDGE CORPORATION, US * Order Number: 6053074 *
- DATABASE CHEMCATS [Online] XP002244569, retrieved from STN Database accession no. 2002:613497 & "CATALOG: ChemBridge Product List", 17 January 2002 (2002-01-17), CHEMBRIDGE CORPORATION, US * Order Number: 6268279 *
- DATABASE CHEMCATS [Online] XP002244570, retrieved from STN Database accession no. 2001:1240237 & "CATALOG: Screening Collection", 28 March 2000 (2000-03-28), ZELINSKY INSTITUTE, US * Order Number: A1397/0062473 *
- SERGIEVSKII A V ET AL: "4-AMINOFURAZAN-3-CARBOXYLIC ACID IMINOESTER IN REACTIONS WITH N,O-NUCLEOPHILES", RUSSIAN JOURNAL OF ORGANIC CHEMISTRY, CONSULTANTS BUREAU, US, vol. 38, no. 6, 1 January 2002 (2002-01-01), pages 872-874, XP009018073, ISSN: 1070-4280, DOI: DOI:10.1023/A:1020307708515
- SERGIEVSKII A V ET AL: "REACTIONS OF METHYL 4-AMINOFURAZAN-3-CARBOXIMIDATE WITH NITROGEN-CONTAINING NUCLEOPHILES", RUSSIAN JOURNAL OF ORGANIC CHEMISTRY, CONSULTANTS BUREAU, US, vol. 37, no. 5, 1 January 2001 (2001-01-01), pages 717-720, XP001155338, ISSN: 1070-4280, DOI: DOI:10.1023/A:1012412220493
- SANTRA, S.; KRISHMAMOORTHY, G.; DOGRA, S.K.: "Excited-State Intramolecular Proton Transfer in 2-(2'-Acetamidophenyl)benzimidazole", JOURNAL OF PHYSICAL CHEMISTRY A, vol. 104, 1 January 2000 (2000-01-01), pages 476-482, XP002629934, DOI: 10.1021/jp992678
- KREUTZBERGER A ET AL: "5-Substituierte 4-Aminopyrimidine durch Aminomethinylierung von Acetonitrilen", JUSTUS LIEBIGS ANNALEN DER CHEMIE, VERLAG CHEMIE GMBH, WEINHEIM; DE, no. 4, 1 January 1977 (1977-01-01), pages 537-544, XP002243486, ISSN: 0075-4617, DOI: DOI:10.1002/JLAC.197719770403
- TAKAGI K ET AL: "Synthesis of pyrimidino[4,5-b][1,5]benzodiazepin-2-ones and pyrimidino[1,6-a]benzimidazol-1-ones from 4-[(ethoxycarbonyl)amino]-1 H-1,5- benzodiazpine-3-carbonitrile via 4-(2-aminoanilino)pyrimidin-2 (1H)-one- 5-carbonitriles", JOURNAL OF HETEROCYCLIC CHEMISTRY, WILEY-BLACKWELL PUBLISHING, INC, US, vol. 23, no. 5, 1 January 1986 (1986-01-01), pages 1443-1449, XP002243485, ISSN: 0022-152X, DOI: DOI:10.1002/JHET.5570230539
- HASKELL T H ET AL: "Neuraminidase inhibition and viral chemotherapy", JOURNAL OF MEDICINAL CHEMISTRY, AMERICAN CHEMICAL SOCIETY, WASHINGTON, US, vol. 13, no. 4, 1 July 1970 (1970-07-01), pages 697-704, XP002277728, ISSN: 0022-2623, DOI: DOI:10.1021/JM00298A027

## Description

### TECHNICAL FIELD OF INVENTION

The present invention relates to inhibitors of protein kinases, especially glycogen synthase kinase-3 (GSK-3), a serine/threonine protein kinase and Lck, a member of the Src family of protein kinases. Kinases are implicated in cancer, immune disorders and bone diseases. The invention also provides pharmaceutically acceptable compositions comprising the inhibitors of the invention and methods of utilizing those compositions in the treatment and prevention of various disorders, such as autoimmune diseases, diabetes, Alzheimer's disease, Huntington's Disease, Parkinson's Disease, multiple sclerosis (MS), schizophrenia, rheumatoid arthritis and leukemia.

### BACKGROUND OF THE INVENTION

The search for new therapeutic agents has been greatly aided in recent years by a better understanding of the structure of enzymes and other biomolecules associated with target diseases. One important class of enzymes that has been the subject of extensive study is protein kinases.

Protein kinase mediate intracellular signal transduction. They do this by effecting a phosphoryl transfer from a nucleoside triphosphate to a protein acceptor that is involved in a signaling pathway. There are a number of kinases and pathways through which extracellular and other stimuli cause a variety of cellular responses to occur inside the cell. Examples of such stimuli include environmental and chemical stress signals (e.g., osmotic shock, heat shock, ultraviolet radiation, bacterial endotoxin, and H₂O₂), cytokines (e.g., interleukin-1 (IL-1) and tumor necrosis factor α (TNF-α)), and growth factors (e.g., granulocyte macrophage-colony-stimulating factor (GM-CSF), and fibroblast growth factor (FGF)). An extracellular stimulus may affect one or more cellular responses related to cell growth, migration, differentiation, secretion of hormones, activation of transcription factors, muscle contraction, glucose metabolism, control of protein synthesis and regulation of cell cycle.

Many diseases are associated with abnormal cellular responses triggered by protein kinase-mediated events. These diseases include autoimmune diseases, inflammatory diseases, metabolic diseases, neurological and neurodegenerative diseases, cancer, cardiovascular diseases, allergies and asthma, Alzheimer's disease and hormone-related diseases. Accordingly, there has been a substantial effort in medicinal chemistry to find protein kinase inhibitors that are effective as therapeutic agents.

Glycogen synthase kinase-3 (GSK-3) is a serine/threonine protein kinase comprised of α and β isoforms that are each encoded by distinct genes [Coghlan et aL, Chemistry & Biology, 7, 793-803 (2000); Kim and Kimmel, Curr. Opinion Genetics Dev., 10, 508-514 (2000)]. GSK-3 has been implicated in various diseases including diabetes, Alzheimer's disease, CNS disorders such as manic depressive disorder and neurodegenerative diseases, and cardiomyocyte hypertrophy [see, e.g., WO 99/65897; WO 00/38675; Kaytor and Orr, Curr. Opin. Neurobiol., 12, 275-8 (2000); Haq et al., J. Cell Biol., 151, 117-30 (2000); Eldar-Finkelman, Trends Mol. Med., 8,126-32 (2002)]. These diseases may be caused by, or may result in, the abnormal operation of certain cell signaling pathways in which GSK-3 plays a role.

GSK-3 has been found to phosphorylate and modulate the activity of a number of regulatory proteins. These include glycogen synthase, which is the rate- limiting enzyme required for glycogen synthesis, the microtubule-associated protein Tau, the gene transcription factor β-catenin, the translation initiation factor e1F-2B, as well as ATP citrate lyase, axin, heat shock factor-1, c*-Jun,* c-*myc,* c-*myb,* CREB, and CEPBα. These diverse targets implicate GSK-3 in many aspects of cellular metabolism, proliferation, differentiation and development.

In a GSK-3 mediated pathway that is relevant for the treatment of type II diabetes, insulin-induced signaling leads to cellular glucose uptake and glycogen synthesis. GSK-3 is a negative regulator of the insulin-induced signal in this pathway. Normally, the presence of insulin causes inhibition of GSK-3-mediated phosphorylation and deactivation of glycogen synthase. The inhibition of GSK-3 leads to increased glycogen synthesis and glucose uptake [Klein et al., PNAS, 93, 8455-9 (1996); Cross et al., Biochem. J., 303, 21-26 (1994); Cohen, Biochem. Soc. Trans., 21, 555-567 (1993); and Massillon et aL, Biochem J. 299, 123-128 (1994); Cohen and Frame, Nat. Rev. Mol. Cell. Biol., 2, 769-76 (2001)]. However, where the insulin response is impaired in a diabetic patient, glycogen synthesis and glucose uptake fail to increase despite the presence of relatively high blood levels of insulin. This leads to abnormally high blood levels of glucose with acute and chronic effects that may ultimately result in cardiovascular disease, renal failure and blindness. In such patients, the normal insulin-induced inhibition of GSK-3 fails to occur. It has also been reported that GSK-3 is overexpressed in patients with type II diabetes [WO 00/38675]. Therapeutic inhibitors of GSK-3 are therefore useful for treating diabetic patients suffering from an impaired response to insulin.

GSK-3 activity has also been associated with Alzheimer's disease. This disease is characterized by the presence of the well-known β-amyloid peptide and the formation of intracellular neurofibrillary tangles. The neurofibrillary tangles contain hyperphosphorylated Tau protein, in which Tau is phosphorylated on abnormal sites. GSK-3 has been shown to phosphorylate these abnormal sites in cell and animal models. Furthermore, inhibition of GSK-3 has been shown to prevent hyperphosphorylation of Tau in cells [Lovestone et al., Curr. Biol., 4,1077-86 (1994); and Brownlees et aL, Neuroreport 8, 3251-55 (1997); Kaytor and Orr, Curr. Opin. Neurobiol., 12, 275-8 (2000)]. In transgenic mice overexpressing GSK3, significant increased Tau hyperphosphorylation and abnormal morphology of neurons were observed [Lucas et al., EMBO J, 20:27-39 (2001)]. Active GSK3 accumulates in cytoplasm of pretangled neurons, which can lead to neurofibrillary tangles in brains of patients with AD [Pei et al., J Neuropathol Exp Neurol 58,1010-19 (1999)].Therefore, inhibition of GSK-3 may be used to slow or halt the generation of neurofibrillary tangles and thus treat or reduce the severity of Alzheimer's disease.

Another substrate of GSK-3 is β-catenin, which is degraded after phosphorylation by GSK-3. Reduced levels of β-catenin have been reported in schizophrenic patients and have also been associated with other diseases related to increase in neuronal cell death [Zhong et al., Nature, 395, 698-702 (1998); Takashima et al., PNAS, 90, 7789-93 (1993); Pei et al., J. Neuropathol. Exp, 56, 70-78 (1997); and Smith et al., Bio-org. Med. Chem. 11, 635-639 (2001)].

GSK-3 activity has also been associated with stroke [Wang et al., Brain Res, 859,381-5 (2000); Sasaki et al., Neurol Res, 23, 588-92 (2001); Hashimoto et al., J. Biol. Chem, July 2, In Press (2002)].

Another protein kinase family of particular interest is the Src family of kinases. These kinases are implicated in cancer, immune system dysfunction and bone remodeling diseases. For general reviews, see Thomas and Brugge, Annu. Rev. Cell Dev. Biol. (1997) 13, 513; Lawrence and Niu, Pharmacol. Ther. (1998) 77, 81; Tatosyan and Mizenina, Biochemistry (Moscow) (2000) 65, 49; Boschelli et al., Drugs of the Future 2000, 25(7), 717, (2000).

Members of the Src family include the following eight kinases in mammals: Src, Fyn, Yes, Fgr, Lyn, Hck, Lck, and Blk. These are nonreceptor protein kinases that range in molecular mass from 52 to 62 kD. All are characterized by a common structural organization that is comprised of six distinct functional domains: Src homology domain 4 (SH4), a unique domain, SH3 domain, SH2 domain, a catalytic domain (SH1), and a C-terminal regulatory region. Tatosyan et al. Biochemistry (Moscow) 65, 49-58 (2000).

Based on published studies, Src kinases are considered as potential therapeutic targets for various human diseases. Lck plays a role in T-cell signaling. Mice that lack the Lck gene have a poor ability to develop thymocytes. The function of Lck as a positive activator of T-cell signaling suggests that Lck inhibitors may be useful for treating autoimmune disease such as rheumatoid arthritis. Molina et al., Nature, 357, 161 (1992). Hck, Fgr and Lyn have been identified as important mediators of integrin signaling in myeloid leukocytes. Lowell et al., J. Leukoc. Biol., 65, 313 (1999). Inhibition of these kinase mediators may therefore be useful for treating inflammation. Boschelli et aL, Drugs of the Future 2000, 25(7), 717, (2000).

Accordingly, there is a high unmet medical need to develop new therapeutic agents that are useful in treating the aforementioned conditions associated with protein kinases, especially GSK-3 and Lck, considering the currently available, relatively inadequate treatment options for the majority of these conditions.

WO 02/088078 and WO 02/050073 disclose compounds with pyrazolo [3,1-c]pyridine and pyrazolo[3,1-c]pyridine cores as GSK-3 inhibitors.

### SUMMARY OF THE INVENTION

It has now been found that compounds of this invention, and pharmaceutically acceptable compositions thereof, are effective as inhibitors of GSK-3 and Lck protein kinases. These compounds fall under the formula **I**: 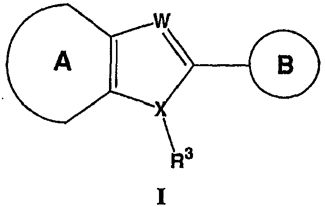 or a pharmaceutically acceptable salt thereof, wherein Ring A, Ring B, W, X, and R³ are as defined herein and wherein W is selected from nitrogen or CR⁴ and X is selected from nitrogen or CH, wherein at least one of W and X is nitrogen;

These compounds, and pharmaceutically acceptable compositions thereof, are useful for treating or lessening the severity of a variety of disorders such as autoimmune diseases, inflammatory diseases, metabolic, neurological and neurodegenerative diseases, cardiovascular diseases, allergy, asthma, diabetes, Alzheimer's disease, Huntington's Disease, Parkinson's Disease, AIDS-associated dementia, amyotrophic lateral sclerosis (AML, Lou Gehrig's Disease), multiple sclerosis (MS), schizophrenia, cardiomyocyte hypertrophy, reperfusion/ischemia, rheumatoid arthritis, baldness and leukemia.

The compounds of the present invention are also useful in methods for enhancing glycogen synthesis and/or lowering blood levels of glucose and therefore are especially useful for diabetic patients. The present compounds are also useful in methods for inhibiting the production of hyperphosphorylated Tau protein, which is useful in halting or slowing the progression of Alzheimer's disease. Another embodiment of this invention relates to a method for inhibiting the phosphorylation of β-catenin, which is useful for treating schizophrenia.

### DETAILED DESCRIPTION OF THE INVENTION

The present invention relates to a compound of formula **Ia** or **Ib**: 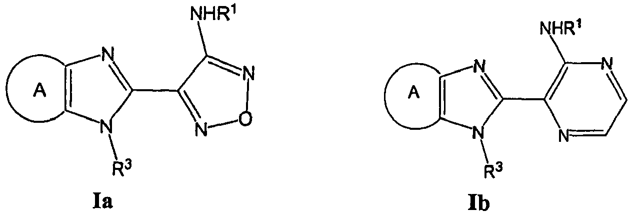 or a pharmaceutically acceptable salt thereof, wherein:
Ring A is a 5-7 membered, partially unsaturated or fully unsaturated ring having 0-3 heteroatoms independently selected from nitrogen, oxygen or sulfur, and wherein Ring A is optionally fused to an saturated, partially unsaturated or fully unsaturated 5-8 membered ring having 0-3 heteroatoms independently selected from nitrogen, oxygen or sulfur, wherein said Ring A and the ring optionally fused to Ring A are optionally substituted on the unsaturated carbon atom of an aryl, heteroaryl, aralkyl, or heteroaralkyl group with halogen, oxo, N₃, -R^{o}, -OR^{o}, SR^{o}, 1,2-methylene-dioxy, 1,2-ethylenedioxy, phenyl (Ph), Ph substituted with R^{o}, -O(Ph), O-(Ph) substituted with R^{o}, -CH₂(Ph), -CH₂(Ph) substituted with R^{o}, -CH₂CH₂ (Ph), -CH₂CH₂ (Ph) substituted with R^{o}, NO₂, -CN, -N(R^{o})₂, -NR^{o}C(O)R^{o}, -NR^{o}C(O)N(R^{o})₂, -NR^{o}CO₂R^{o}, - NR^{o}NR^{o}C(O)R^{o}, -NR^{o}NR^{o}C(O)N(R^{o})2, -NR^{o}NR^{o}CO₂R^{o}, -C(O)C(O)R^{o}, - C(O)CH₂C(O)R^{o}, -CO₂R^{o}, -C(O)R^{o}, -C(O)N(R^{o})₂, -OC(O)N(R^{o})₂, -S(O)₂R^{o}, - SO₂N(R^{o})₂, -S(O)R^{o}, -NR^{o}SO₂N(R^{o})₂, -NR^{o}SO₂R^{o}, -C(=S)N(R^{o})₂, -C(=NH)-N(R^{o})₂, or -(CH₂)_{Y}NHC(O)R^{o}, wherein y is 0-4, each R^{o} is independently selected from hydrogen, optionally substituted C₁₋₆ aliphatic, an unsubstituted 5-6 membered heteroaryl or heterocyclic ring having 0-4 heteroatoms independently selected from nitrogen, oxygen or sulfur, phenyl (Ph), -O(Ph), or -CH₂(Ph)-CH₂(Ph), and wherein each optional substituent on the aliphatic group of R^{o} is independently selected from NH₂, NH(C₁₋₄ aliphatic), N(C₁₋₄ aliphatic)₂, halogen, C₁₋₄ aliphatic, OH, O-(C₁₋₄ aliphatic), NO₂, CN, CO₂H, CO₂(C₁₋₄ aliphatic), -O(haloC₁₋₄ aliphatic), or halo C₁₋₄ aliphatic; and wherein said Ring A and the ring optionally fused to Ring A are optionally substituted on the saturated carbon atom of an aliphatic group or of a non-aromatic heterocyclic ring with the substituents being selected from those listed above for the unsaturated carbon of an aryl or heteroaryl group and =O, =S, =NNHR*, NN(R*)₂, =N-, =NNHC(O)R*, =NNHCO₂ (alkyl), =NNHSO₂ (alkyl), or =NR*, where each R*is independently selected from hydrogen or an optionally substituted C1-6 aliphatic with the substituents on the aliphatic group of R* are selected from NH₂, NH(C₁₋₄ aliphatic), N(C₁₋₄ aliphatic)₂, halogen, C₁₋₄ aliphatic, OH, O-(C₁₋₄ aliphatic), NO₂, CN, CO₂H, CO₂ (C₁₋₄ aliphatic)-O(haloC₁₋₄ aliphatic, or haloC₁₋₄ aliphatic;
R¹ is selected from R or R²;
R² is selected from -SO₂R, -SO₂N(R)₂, -CN, -C(O)R, -CO₂F, or -CON(R)₂;
R is independently selected from hydrogen or an optionally substituted group selected from C₁₋₆ aliphatic, a 3-6 membered saturated, partially unsaturated, or aryl ring having 0-4 heteroatoms independently selected from nitrogen, oxygen, or sulfur, or:
two R groups on the same nitrogen are taken together with the nitrogen bound thereto to form a 3-7 membered heterocyclic or heteroaryl ring having 1-4 heteroatoms independently selected from nitrogen, oxygen, or sulfur, wherein the optional substituent is selected as listed above;
R³ is selected from T-CN or L-R;
T is a valence bond or an optionally substituted C₁₋₆ alkylidene chain with the optional substituent being selected as listed before for the substituents on the saturated carbon of an aliphatic group, ; and
L is a valence bond or a C₁₋₄ alkylidene chain, wherein up to two methylene units of L are optionally, and independently, replaced by, -S-, -NR-, -NRC(O)-, -NRC(O)NR-, -OC(O)NR-, , -CO₂-, -NRCO₂-, -C(O)NR-, -SO₂NR-, -NRSO₂-, or -NRSO₂NR-;
provided that said compound is other than one of the group consisting of:
   4-[1-(4-Chloro-benzyl)-1H-benzoimidazol-2-yl]-furazan-3-ylamine (**I**-1);
   4-(1-Prop-2-ynyl-1H-benzoimidazol-2-yl)-furazan-3-ylamine (**I**-2);
   4-(5-Methyl-1H-benzoimidazol-2-yl)-furazan-3-ylamine (**I**-3);
   4-[1-(2-Chloro-6-fluoro-benzyl)-1H-benzoimidazol-2-yl]-furazan-3-ylamine (**I**-4);
   [2-(4-Amino-furazan-3-yl)-benzoimidazol-1-yl]-acetic acid (**I**-5);
   2-[2-(4-Amino-furazan-3-yl)-benzoimidazol-1-yl]-acetamide (**I**-6);
   4-(1-Propyl-1H-benzoimidazol-2-yl)-furazan-3-ylamine (**I**-7);
   4-[1-(2,6-Dichloro-benzyl)-1H-benzoimidazol-2-yl]-furazan-3-ylamine (**I**-8);
   4-(1-Allyl-1H-benzoimidazol-2-yl)-furazan-3-ylamine (**I**-9);
   4-[1-(4-Methyl-benzyl)-1H-benzoimidazol-2-yl]-furazan-3-ylamine (**I**-10);
   4-(1-Isopropyl-1H-benzoimidazol-2-yl)-furazan-3-ylamine (**I**-11);
   4-[1-(2-Methyl-benzyl)-1H-benzoimidazol-2-yl]-furazan-3-ylamine (**I**-12);
   [2-(4-Amino-furazan-3-yl)-benzoimidazol-1-yl]-acetonitrile (**I**-13);
   4-[1-(1H-Tetrazol-5-ylmethyl)-1H-benzoimidazol-2-yl]-krazan-3-ylamine (**I**-14);
   4-[1-(2,4-Dichlorobenzyl)-1H-benzoimidazol-2-yl]-furazan-3-ylamine (**I**-15);
   4-[1-(3,4-Dichloro-benzyl)-1H-benzoimidazol-2-yl]-furazan-3-ylamine (**I**-16);
   2-[2-(4-Amino-furazan-3-yl)-benzoimidazol-1-yl]-N-(3,4-dimethoxy-phenyl)-acetamide (**I-**17);
   4-(1H-Benzoimidazol-2-yl)-furazan-3-ylamine (**I**-18);
   2-[2-(4-Amino-furazan-3-yl)-benzoimidazol-1-yl]-N-(3,4-difluoro-phenyl)-acetamide (**I-**19);
   2-[2-(4-Amino-furazan-3-yl)-benzoimidazol-1-ylmethyl]-benzonitrile (I**-**20);
   2-[2-(4-Amino-furazan-3-yl)-benzoimidazol-1-yl]-N-(2-trifluoromethyl-phenyl)-acetamide (1-21);
   4-[1-(3-Bromo-benzyl)-1H-benzoimidazol-2-yl]-furazan-3-ylamine (**I**-22);
   4-[2-(4-Amino-furazan-3-yl)-benzoimidazol-1-yl]-butyronitrile (**I**-23);
   4-(1-Ethyl-1H-benzoimidazol-2-yl)-furazan-3-ylamine (**I**-55);
   2-[2-(4-Amino-furazan-3-yl)-benzoimidazol-1-yl]-N-(2-fluoro-phenyl)-acetamide (**I**-61);
   4-(1-Methyl-1H-benzoimidazol-2-yl)-furazan-3-ylamine (**I**-62);
   2-[2-(4-Amino-furazan-3-yl)-benzoimidazol-1-yl]-N-biphenyl-2-yl-acetamide (**I**-63);
   2-[2-(4-Amino-furazan-3-yl)-benzoimidazol-1-yl]-N-(2,6-dimethyl-phenyl)-acetamide (**I-**64);
   2-[2-(4-Amino-furazan-3-yl)-benzoimidazol-1-yl]-N-tert-butyl-acetamide (**I**-65);
   2-[2-(4-Amino-furazan-3-yl)-benzoimidazol-1-yl]-N-(3-fluoro-phenyl)-acetamide (**I**-66);
   2-[2-(4-Amino-furazan-3-yl)-benzoimidazol-1-yl]-N-(2-fluoro-phenyl)-acetamide (**I**-70);
   N-[4-(1-Ethyl-1H-benzoimidazol-2-yl)-furazan-3-yl]-2,2,2-trifluoro-acetamide (**I**-72);
   N-[4-(1-Cyanomethyl-1H-benzoimidazol-2-yl)-furazan-3-yl]-acetamide (**I**-76);
   2-(4-Amino-1,2,5-oxadiazol-3-yl)-1H-benzimidazole-1-acetonitrile;
   2-(4-Amino-1,2,5-oxadiazol-3-yl)-N-(2,4-difluorophenyl)-1H-benzimidazole-1-acetamide;
   4-[1-(Cyclopropylmethyl)-1H-benzimidazol-2-yl-1,2,5-oxadiazol-3-amine;
   N-[4-[1-(Cyclopropylmethyl)-1H-benzimidazol-2-yl]-1,2,5-oxadiazol-3-yl]-acetamide;
   4-[1-(2-Methylpropyl)-1H-benzimidazol-2-yl]-1,2,5-oxadiazol-3-amine;
   4-[1-(Phenylmethyl)-1H-benzimidazol-2-yl-1,2,5-oxadiazol-3-amine;
   2-(4-Amino-1,2,5-oxadiazol-3-yl)-N-[4-(trifluoromethoxy)phenyl]-1H-benzimidazole-1-acetamide;
   2-(4-Amino-1,2,5-oxadiazol-3-yl)-1H-benzimidazole-1-acetic acid ethyl ester;
   N-[4-[1-[(5-Methyl-1,3,4-oxadiazol-2yl)-methyl]-1H-benzimidazol-2-yl]-1,2,5-oxadiazol-3yl-acetamide;
   4-(1-Methyl-5-nitro-1H-benzimidazol-2-yl)-1,2,5-oxadiazol-3-amine[,] ;
   3-Amino-4-(1H-benzimidazol-2-yl)-1,2,5-oxadiazole;
   3-Amino-4-(1-ethyl-1H-benzimidazol-2-yl)-1,2,5-oxadiazole;
   4-(1H-benzo[d]imidazol-2-yl)-1,2,5-oxadiazol-3-amine; and
   Methyl-2-[2-(4-amino-1,2,5-oxadiazol-3 yl)-(1H-benzimidazol-1-yl)]-acetate wherein each compound number corresponds to the compound numbers of Table 1.

As used herein, the following definitions shall apply unless otherwise indicated. The phrase "optionally substituted" is used interchangeably with the phrase "substituted or unsubstituted." Unless otherwise indicated, an optionally substituted group may have a substituent at each substitutable position of the group, and each substitution is independent of the other.

The term "aliphatic" or "aliphatic group" as used herein means a straight-chain or branched C₁-C₁₂ hydrocarbon chain that is completely saturated or that contains one or more units of unsaturation, or a monocyclic C₃-C₈ hydrocarbon or bicyclic C₈-C₁₂ hydrocarbon that is completely saturated or that contains one or more units of unsaturation, but which is not aromatic (also referred to herein as "carbocycle" or "cycloalkyl"), that has a single point of attachment to the rest of the molecule wherein any individual ring in said bicyclic ring system has 3-7 members. For example, suitable aliphatic groups include, but are not limited to, linear or branched or alkyl, alkenyl, alkynyl groups and hybrids thereof such as (cycloalkyl)alkyl, (cycloalkenyl)alkyl or (cycloalkyl)alkenyl.

The terms "alkyl", "alkoxy", "hydroxyalkyl", "alkoxyalkyl", and "alkoxycarbonyl", used alone or as part of a larger moiety includes both straight and branched chains containing one to twelve carbon atoms. The terms "alkenyl" and "alkynyl" used alone or as part of a larger moiety shall include both straight and branched chains containing two to twelve carbon atoms.

The terms "haloalkyl", "haloalkenyl" and "haloalkoxy" means alkyl, alkenyl or alkoxy, as the case may be, substituted with one or more halogen atoms. The term "halogen" means F, Cl, Br, or L

The term "heteroatom" means nitrogen, oxygen, or sulfur and includes any oxidized form of nitrogen and sulfur, and the quaternized form of any basic nitrogen. Also the term "nitrogen" includes a substitutable nitrogen of a heterocyclic ring. As an example, in a saturated or partially unsaturated ring having 0-4 heteroatoms selected from oxygen, sulfur or nitrogen, the nitrogen may be N (as in 3,4-dihydro-2*H*-pyrrolyl), NH (as in pyrrolidinyl) or NR⁺ (as in N-substituted pyrrolidinyl).

The term "aryl" used alone or as part of a larger moiety as in "aralkyl", "aralkoxy", or "aryloxyalkyl", refers to monocyclic, bicyclic and tricyclic ring systems having a total of five to fourteen ring members, wherein at least one ring in the system is aromatic and wherein each ring in the system contains 3 to 7 ring members. The term "aryl" may be used interchangeably with the term "aryl ring".

The term "heterocycle", "heterocyclyl", or "heterocyclic" as used herein means non-aromatic, monocyclic, bicyclic or tricyclic ring systems having five to fourteen ring members in which one or more ring members is a heteroatom, wherein each ring in the system contains 3 to 7 ring members.

The term "heteroaryl", used alone or as part of a larger moiety as in "heteroaralkyl" or "heteroarylalkoxy", refers to monocyclic, bicyclic and tricyclic ring systems having a total of five to fourteen ring members, wherein at least one ring in the system is aromatic, at least one ring in the system contains one or more heteroatoms, and wherein each ring in the system contains 3 to 7 ring members. The term "heteroaryl" may be used interchangeably with the term "heteroaryl ring" or the term "heteroaromatic".

An aryl (including aralkyl, aralkoxy, aryloxyalkyl and the like) or heteroaryl (including heteroaralkyl and heteroarylalkoxy and the like) group may contain one or more substituents. Suitable substituents on the unsaturated carbon atom of an aryl, heteroaryl, aralkyl, or heteroaralkyl group are selected from halogen, oxo, N₃, -R^{o}, -OR^{o}, -SR^{o}, 1,2-methylene-dioxy, 1,2-ethylenedioxy, protected OH (such as acyloxy), phenyl (Ph), Ph substituted with R^{o}, -O(Ph), O-(Ph) substituted with R^{o}, -CH₂(Ph), -CH₂(Ph) substituted with R^{o}, -CH₂CH₂(Ph), -CH₂CH₂(Ph) substituted with R^{o}, -NO₂, -CN, -N(R^{o})₂, -NR^{o}C(O)R^{o}, -NR^{o}C(O)N(R^{o})₂, -NR^{o}-CO₂R^{o}, -NR^{o}NR^{o}C(O)R^{o}, -NR^{o}NR^{o}C(O)N(R^{o})₂, -NR^{o}NR^{o}CO₂R^{o}, -C(O)C(O)R^{o}, -C(O)CH₂C(O)R^{o}, -CO₂R^{o}, -C(O)R^{o}, -C(O)N(R^{o})₂, -OC(O)N(R^{o})2, -S(O)₂R^{o}, -SO₂N(R^{o})₂, -S(O)R^{o}, -NR^{o}SO₂N(R^{o})₂, -NR^{o}SO₂R^{o}, -C(=S)N(R^{o})₂, -C(=NH)-N(R^{o})₂, or -(CH₂)yNHC(O)R^{o}, wherein y is 0-4, each R^{o} is independently selected from hydrogen, optionally substituted C₁₋₆ aliphatic, an unsubstituted 5-6 membered heteroaryl or heterocyclic ring having 0-4 heteroatoms independently selected from nitrogen, oxygen, or sulfur, phenyl (Ph), -O(Ph), or -CH₂(Ph)-CH₂(Ph). Substituents on the aliphatic group of R^{o} are selected from NH₂, NH(C₁₋₄ aliphatic), N(C₁₋₄ aliphatic)₂, halogen, C₁₋₄ aliphatic, OH, O-(C₁₋₄ aliphatic), NO₂, CN, CO₂H, CO₂(C₁₋₄ aliphatic), -O(halo C₁₋₄ aliphatic), or halo C₁₋₄ aliphatic.

An aliphatic group or a non-aromatic heterocyclic ring may contain one or more substituents. Suitable substituents on the saturated carbon of an aliphatic group or of a non-aromatic heterocyclic ring are selected from those listed above for the unsaturated carbon of an aryl or heteroaryl group and the following: =O, =S, =NNHR*, =NN(R*)₂, =N-, =NNHC(O)R*, =NNHCO₂(alkyl), =NNHSO₂(alkyl), or =NR*, where each R* is independently selected from hydrogen or an optionally substituted C₁₋₆ aliphatic. Substituents on the aliphatic group of R* are selected from NH₂, NH(C₁₋₄ aliphatic), N(C₁₋₄ aliphatic)₂, halogen, C₁₋₄ aliphatic, OH, O-(C₁₋₄ aliphatic), NO₂, CN, CO₂H, CO₂(C₁₋₄ aliphatic), -O(halo C₁₋₄ aliphatic), or halo C₁₋₄ aliphatic.

Substituents on the nitrogen of a non-aromatic heterocyclic ring are selected from -R⁺, -N(R⁺)₂, -C(O)R⁺, -CO₂R⁺, -C(O)C(O)R⁺, -C(O)CH₂C(O)R⁺, -SO₂R⁺, -SO₂N(R⁺)₂, -C(=S)N(R¹)₂, -C(=NH)-N(R⁺)₂, or -NR⁺SO₂R⁺; wherein R⁺ is hydrogen, an optionally substituted C₁₋₆ aliphatic, optionally substituted phenyl (Ph), optionally substituted -O(Ph), optionally substituted -CH₂(Ph), optionally substituted -CH₂CH₂(Ph), or an unsubstituted 5-6 membered heteroaryl or heterocyclic ring. Substituents on the aliphatic group or the phenyl ring of R⁺ are selected from NH₂, NH(C₁₋₄ aliphatic), N(C₁₋₄ aliphatic)₂, halogen, C₁₋₄ aliphatic, OH, O-(C₁₋₄ aliphatic), NO₂, CN, CO₂H, CO₂(C₁₋₄ aliphatic), -O(halo C₁₋₄ aliphatic), or halo C₁₋₄ aliphatic.

The term "alkylidene chain" refers to a straight or branched carbon chain that may be fully saturated or have one or more units of unsaturation and has two points of connection to the rest of the molecule.

The compounds of this invention are limited to those that are chemically feasible and stable. Therefore, a combination of substituents or variables in the compounds described above is permissible only if such a combination results in a stable or chemically feasible compound. A stable compound or chemically feasible compound is one in which the chemical structure is not substantially altered when kept at a temperature of 40 °C or less, in the absence of moisture or other chemically reactive conditions, for at least a week.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the invention. Unless otherwise stated, structures depicted herein are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon are within the scope of this invention.

Compounds of this invention may exist in alternative tautomeric forms. Unless otherwise indicated, the representation of either tautomer is meant to include the other.

Preferred Ring A moieties of formula **I** include an optionally substituted five to six membered aryl, heteroaryl or heterocyclic ring having 0-2 heteroatoms independently selected from nitrogen, oxygen or sulfur. More preferred Ring A moieties of formula **I** include an optionally substituted phenyl ring or an optionally substituted 6-membered heteroaryl or heterocyclic ring having 1-2 nitrogens. Examples of such preferred Ring A groups include rings **a** though **k** below:

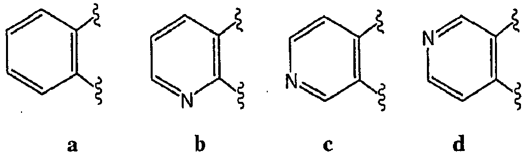

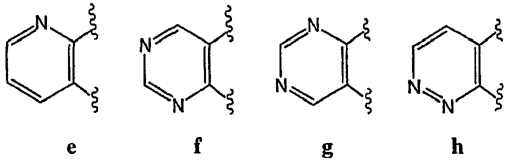

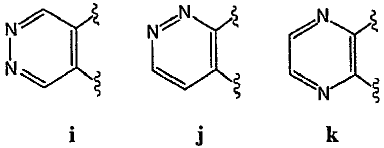

More preferably, Ring A is selected from rings **a, b,** or **f,** and most preferably Ring A is an optionally substituted benzo ring (**a**).

Preferred Ring B moieties of formula I include an optionally substituted 5-6 membered aromatic ring having 0-3 heteroatoms, independently selected from sulfur, oxygen and nitrogen. More preferred Ring B moieties of formula **I** are optionally substituted pyrazine, pyridine, pyrazole, phenyl, furazanyl, or thienyl rings.

Preferred R¹ groups of formula **I** include R, SO₂R, or -C(O)R, wherein each R is independently selected from hydrogen or an optionally substituted phenyl or C₁₋₄ aliphatic group. Accordingly, preferred R¹ groups of formula **I** include -C(O)CF₃, -C(O)CH₃, -C(O)CH₂CH₃, -SO₂Me, and methyl Preferred R¹ groups of Formula **I** also include those shown in Table 1 below.

Preferred substituents on Ring A of formula **I**, when present, are halogen, -NO₂, -R^{o}, -OR^{o}, -CO₂R^{o}, or -N(R^{o})₂. More preferred substituents on Ring A of formula **I** are chloro, bromo, methyl, -CF₃, nitro, *t*-butyl, methoxy, -CO₂Me, hydroxy, amino, - NH(Me), or -OCH₂CN.

Preferred rings fused to Ring A of formula **I**, when present, include optionally substituted benzo, 5-6 membered carbocyclo, or a 5-6 membered heterocyclo ring having 1-2 heteroatoms independently selected from nitrogen, oxygen, or sulfur, such as methylenedioxy, or pyrido ring.

Preferred R³ groups of formula **I** include T-CN or L-R, wherein T is a C₁₋₄ alkylidene chain, L is selected from a valence bond or a C₁₋₄ alkylidene chain wherein a methylene unit of L is optionally replaced by -CO₂, -C(O)NR-, -C(O)-, -N(R)-, or -O-, and wherein R is an optionally substituted C₁₋₄ aliphatic, 3-6 membered heterocyclyl ring having 1-2 heteroatoms independently selected from nitrogen, oxygen, or sulfur, optionally substituted phenyl, or an optionally substituted 5-6 membered heteroaryl ring having 1-4 heteroatoms independently selected from nitrogen, oxygen, or sulfur. Examples of such groups include those shown in Table 1 below, -CH₂CN, -CH₂C(O)NH₂, -CH₂CO₂H, propyl, -CH₂CH₂=CH₂, isopropyl, -(CH₂)₃CN, -CH₂OEt, -CH₂CF₃, isobutyl, cyclopropylmethyl, -CH₂CH₂N(Me)₂, -CH₂CH(OEt)₂, ethyl, -CH₂C(O)NH*t*-butyl, or an optionally substituted benzyl or -CH₂C(O)NHphenyl group. Examples of substituents on said benzyl or phenyl group include halogen, R^{o}, OR^{o}, CN, phenyl, and those shown below in Table 1.

According to one embodiment, the present invention relates to a compound of formula **Ia**: 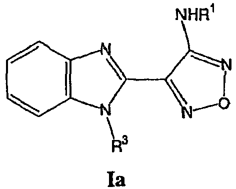 or a pharmaceutically acceptable salt thereof, wherein R¹ and R³ are as defined above and the benzo ring of formula **Ia** is optionally substituted.

Preferred substituents on the benzo ring of formula **Ia,** when present, include those set forth as preferred substituents on the Ring A moiety of formula **I.**

Preferred R¹ and R³ groups of formula **Ia** are those set forth as preferred R¹ and R³ groups of formula **I**, *supra.*

According to another embodiment, the present invention relates to a compound of formula **Ib:** 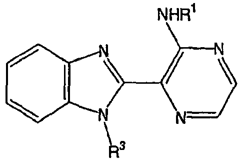 or a pharmaceutically acceptable salt thereof, wherein R¹ and R³ are as defined above and the benzo ring of formula **Ib** is optionally substituted.

Preferred substituents on the benzo ring of formula **Ib,** when present, include those set forth as preferred substituents on the Ring A moiety of formula **I**.

Preferred R¹ and R³ groups of formula **Ib** are those set forth as preferred R¹ and R³ groups of formula **I**, *supra.*

Representative compounds of formula **I** are shown below in Table 1.

**Table 1: Compounds of formula I**

| **No. I-** | **Structure** | **No. I-** | **Structure** |
|---|---|---|---|
| **25** | 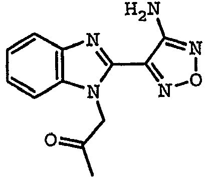 | **26** | 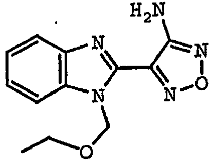 |
| **27** | 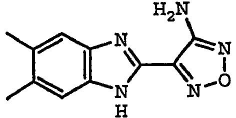 | **28** | 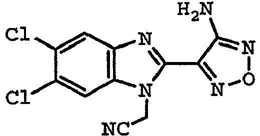 |
| **29** | 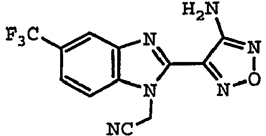 | **30** | 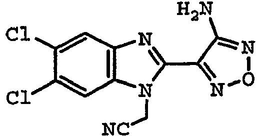 |
| **31** | 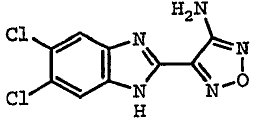 | **32** | 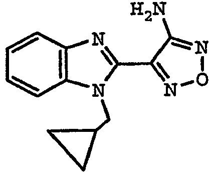 |
| | | **34** | 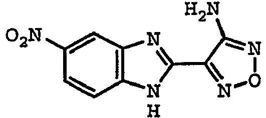 |
| **35** | 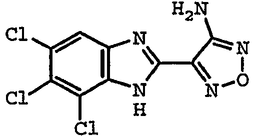 | **36** | 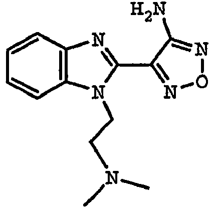 |
| **37** | 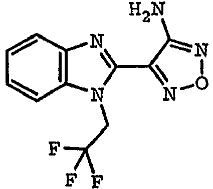 | | |
| **39** | 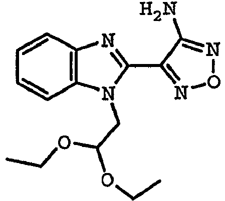 | **40** | 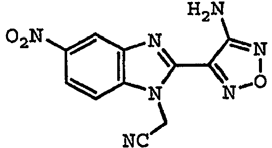 |
| **41** | 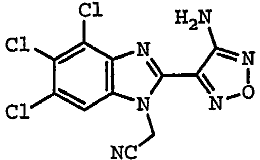 | **42** | 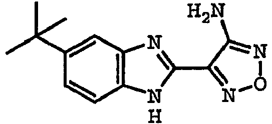 |
| **43** | 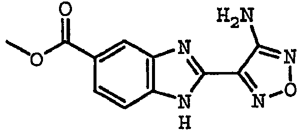 | **44** | 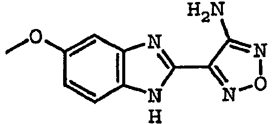 |
| **45** | 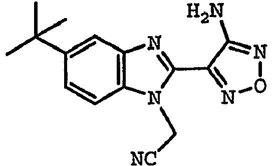 | **46** | 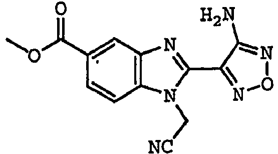 |
| **47** | 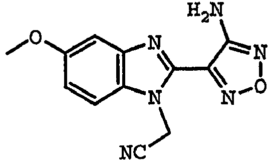 | **48** | 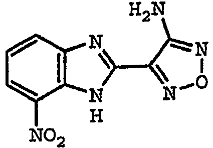 |
| **49** | 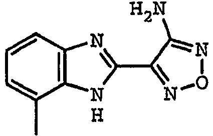 | **50** | 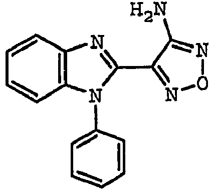 |
| **51** | 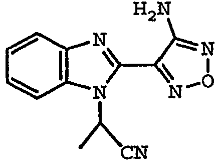 | **52** | 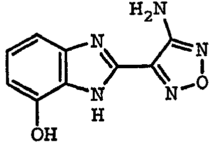 |
| **53** | 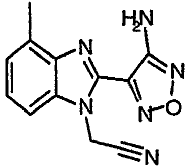 | **54** | 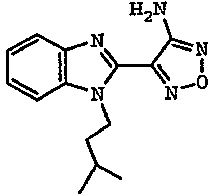 |
| | | **56** | 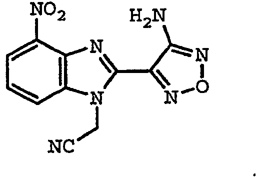 |
| **57** | 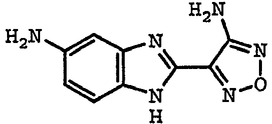 | **58** | 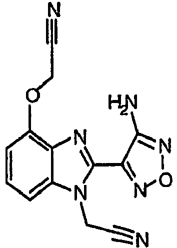 |
| **59** | 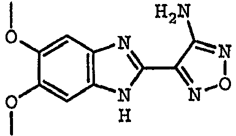 | **60** | 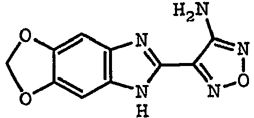 |
| | | | |
| | | | |
| | | | |
| **67** | 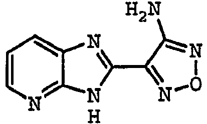 | **68** | 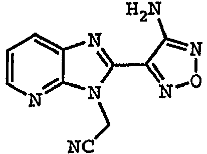 |
| **69** | 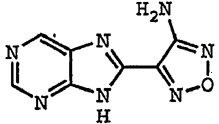 | | |
| | | | |
| | | | |
| | | **76** | 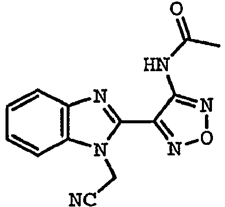 |
| | | | |
| | | **80** | 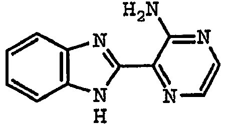 |
| | | | |
| **83** | 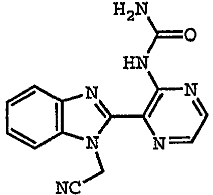 | | |
| **85** | 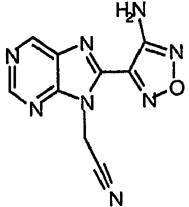 | | |
| | | **88** | 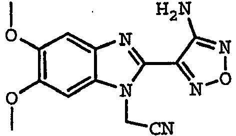 |
| | | **90** | 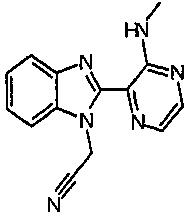 |
| | | **92** | 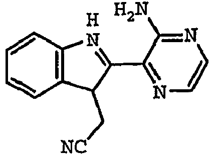 |
| **93** | 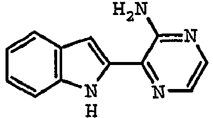 | **94** | 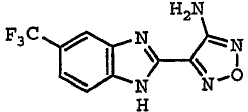 |
| **101** | 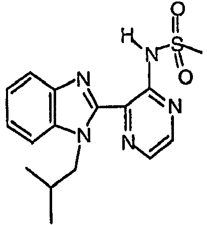 | **102** | 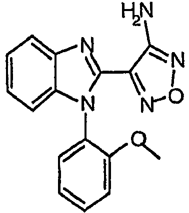 |
| **103** | 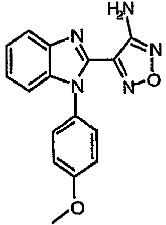 | **104** | 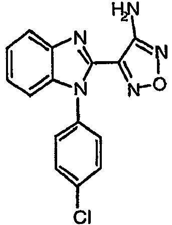 |
| **105** | 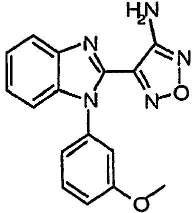 | | |
| **107** | 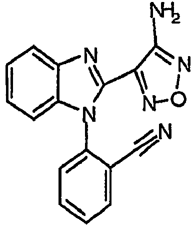 | **108** | 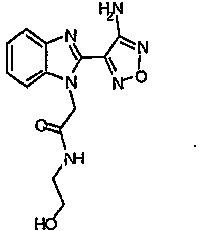 |
| **109** | 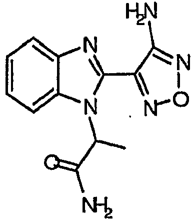 | | |
| **111** | 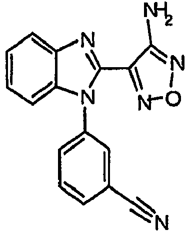 | | |
| **113** | 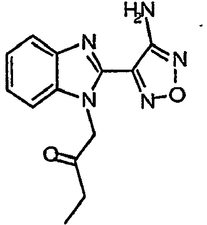 | **114** | 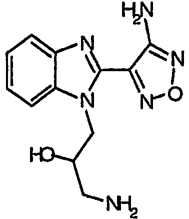 |
| **115** | 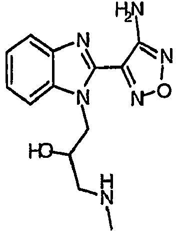 | **116** | 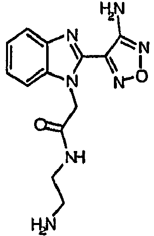 |
| **117** | 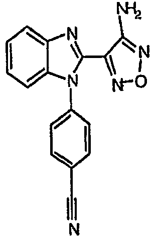 | **118** | 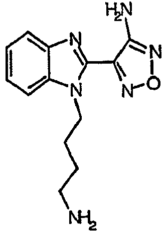 |
| **119** | 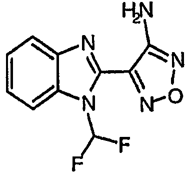 | **120** | 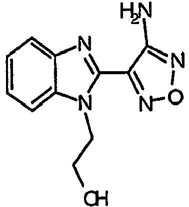 |
| **121** | 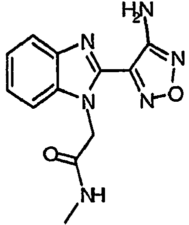 | **122** | 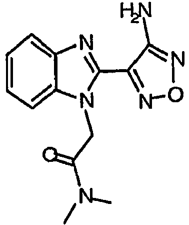 |
| **123** | 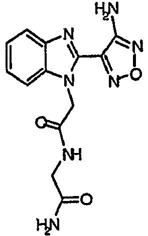 | **124** | 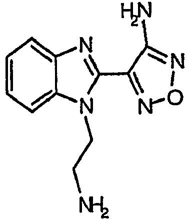 |
| **125** | 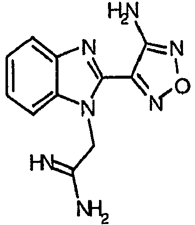 | **126** | 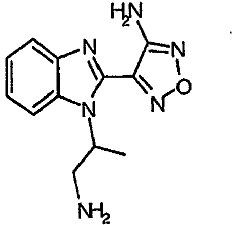 |
| **127** | 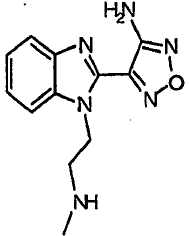 | **128** | 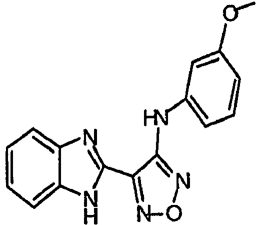 |
| **129** | 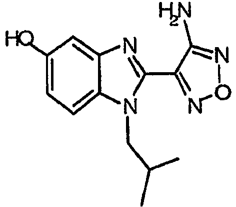 | **130** | 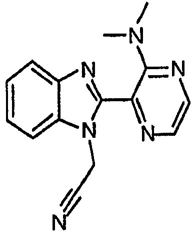 |
| **131** | 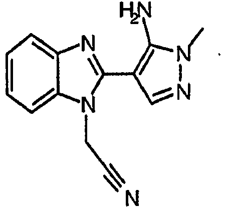 | **132** | 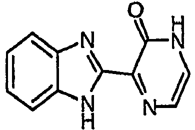 |
| **133** | 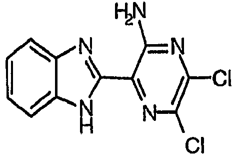 | **134** | 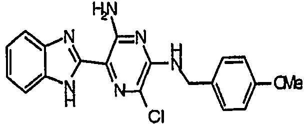 |
| **135** | 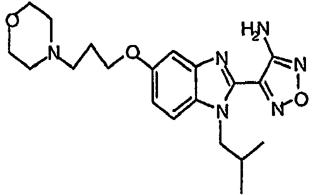 | | |

The compounds of this invention generally may be prepared from known starting materials, following methods known to those skilled in the art for analogous compounds, as illustrated by the following Schemes I through III and by the synthetic examples set forth below. Schemes I through III show a general approach for making the present compounds.

The activity of a compound utilized in this invention as an inhibitor of GSK3 or LCK protein kinase may be assayed *in vitro, in vivo* or in a cell line according to methods known in the art. *In vitro* assays include assays that determine inhibition of either the phosphorylation activity or ATPase activity of activated GSK3 or LCK. Alternate *in vitro* assays quantitate the ability of the inhibitor to bind to GSK3 or LCK. Inhibitor binding may be measured by radiolabelling the inhibitor prior to binding, isolating the inhibitor/GSK3 or inhibitor/LCK complex and determining the amount of radiolabel bound. Alternatively, inhibitor binding may be determined by running a competition experiment where compounds are incubated with GSK3 or LCK bound to known radioligands. Detailed conditions for assaying a compound utilized in this invention as an inhibitor of GSK3 or LCK kinase are set forth in the Examples below.

According to another embodiment, the invention provides a composition comprising a compound of this invention or a pharmaceutically acceptable derivative thereof and a pharmaceutically acceptable carrier, adjuvant, or vehicle. The amount of compound in the compositions of this invention is such that is effective to measurably inhibit a protein kinase, particularly GSK3 or LCK kinase, in a biological sample or in a patient. Preferably the composition of this invention is formulated for administration to a patient in need of such composition. Most preferably, the composition of this invention is formulated for oral administration to a patient.

The term "patient", as used herein, means an animal, preferably a mammal, and most preferably a human.

The term "pharmaceutically acceptable carrier, adjuvant, or vehicle" refers to a non-toxic carrier, adjuvant, or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyozypropylene-block polymers, polyethylene glycol and wool fat.

The term "measurably inhibit", as used herein means a measurable change in GSK3 or LCK activity between a sample comprising said composition and a GSK3 or LCK kinase and an equivalent sample comprising GSK3 or LCK kinase in the absence of said composition.

A "pharmaceutically acceptable salt" means any non-toxic salt or salt of an ester of a compound of this invention that, upon administration to a recipient, is capable of providing, either directly or indirectly, a compound of this invention or an inhibitorily active metabolite or residue thereof. As used herein, the term "inhibitorily active metabolite or residue thereof" means that a metabolite or residue thereof is also an inhibitor of a GSK3 or LCK family kinase.

Pharmaceutically acceptable salts of the compounds of this invention include those derived from pharmaceutically acceptable inorganic and organic acids and bases. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts.

Salts derived from appropriate bases include alkali metal (e.g., sodium and potassium), alkaline earth metal (e.g., magnesium), ammonium and N⁺(C₁₋₄ alkyl)₄ salts. This invention also envisions the quaternization of any basic nitrogen-containing groups of the compounds disclosed herein. Water or oil-soluble or dispersible products may be obtained by such quaternization.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutically acceptable compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, ceteatyl alcohol, 2-octyldodecanol, benzyl alcohol and water.

For ophthalmic use, the pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

The pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, the pharmaceutically acceptable compositions of this invention are formulated for oral administration.

The amount of the compounds of the present invention that may be combined with the carrier materials to produce a composition in a single dosage form will vary depending upon the host treated, the particular mode of administration. Preferably, the compositions should be formulated so that a dosage of between 0.01 - 100 mg/kg body weight/day of the inhibitor can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

Depending upon the particular condition, or disease, to be treated or prevented, additional therapeutic agents, which are normally administered to treat or prevent that condition, may also be present in the compositions of this invention. As used herein, additional therapeutic agents that are normally administered to treat or prevent a particular disease, or condition, are known as "appropriate for the disease, or condition, being treated".

For example, chemotherapeutic agents or other anti-proliferative agents may be combined with the compounds of this invention to treat proliferative diseases and cancer. Examples of known chemotherapeutic agents include, but are not limited to, Gleevec™, adriamycin, dexamethasone, vincristine, cyclophosphamide, fluorouracil, topotecan, taxol, interferons, and platinum derivatives.

Other examples of agents the inhibitors of this invention may also be combined with include, without limitation: treatments for Alzheimer's Disease such as Aricept^{®} and Excelon^{®}; treatments for Parkinson's Disease such as L-DOPA/carbidopa; entacapone, ropinrole, pramipexole, bromocriptine, pergolide, trihexephendyl, and amantadine; agents for treating Multiple Sclerosis (MS) such as beta interferon (e.g., Avonex^{®} and Rebif^{®}), Copaxone^{®}, and mitoxantrone; treatments for asthma such as albuterol and Singular^{®}; agents for treating schizophrenia such as zyprexa, risperdal, seroquel, and haloperidol; anti-inflammatory agents such as corticosteroids, TNF blockers, IL-1 RA, azathioprine, cyclophosphamide, and sulfasalazine; immunomodulatory and immunosuppressive agents such as cyclosporin, tacrolimus, rapamycin, mycophenolate mofetil, interferons, corticosteroids, cyclophophamide, azathioprine, and sulfasalazine; neurotrophic factors such as acetylcholinesterase inhibitors, MAO inhibitors, interferons, anti-convulsants, ion channel blockers, riluzole, and anti-Parkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders such as corticosteroids, anti-leukemic agents, and growth factors; and agents for treating immunodeficiency disorders such as gamma globulin.

The amount of additional therapeutic agent present in the compositions of this invention will be no more than the amount that would normally be administered in a composition comprising that therapeutic agent as the only active agent Preferably the amount of additional therapeutic agent in the presently disclosed compositions will range from about 50% to 100% of the amount normally present in a composition comprising that agent as the only therapeutically active agent.

According to another embodiment, the invention relates to an in vitro method of inhibiting GSK3 or LCK kinase activity in a biological sample comprising the step of contacting said biological sample with a compound of this invention, or a composition comprising said compound. Preferably, the method comprises the step of contacting said biological sample with a preferred compound of the present invention, as described herein *supra.*

The term "biological sample", as used herein, includes, without limitation, cell cultures or extracts thereof; biopsied material obtained from a mammal or extracts thereof; and blood, saliva, urine, feces, semen, tears, or other body fluids or extracts thereof.

Inhibition of GSK3 or LCK kinase activity in a biological sample is useful for a variety of purposes that are known to one of skill in the art. Examples of such purposes include, but are not limited to, blood transfusion, organ-transplantation, biological specimen storage, and biological assays.

Another aspect of this invention relates to the claimed compounds for use in treating a GSK3 or LCK -mediated disease in a patient, which method comprises administering to a patient in need thereof, a therapeutically effective amount of a compound of the present invention, or a pharmaceutically acceptable composition comprising said compound. According to another embodiment, the invention relates to administering a compound of formula Ia, or a pharmaceutically acceptable composition comprising said compound. Yet another embodiment relates to administering a preferred compound of formula Ia, as described herein *supra,* or a pharmaceutically acceptable composition comprising said compound.

Further described herein is a method for treating an GSK3 or LCK -mediated disease in a patient, which method comprises administering to a patient in need thereof, a therapeutically effective amount of a compound of formula **Ib,** or a pharmaceutically acceptable composition comprising said compound. According to another embodiment, said method comprises administering to a patient in need thereof, a therapeutically effective amount of a preferred compound of formula **Ib,** as described herein *supra*, or a pharmaceutically acceptable composition comprising said compound.

Further described herein is a method for treating or lessening the severity of a GSK-3-mediated disease or condition in a patient comprising the step of administering to said patient a composition according to the present invention.

The compound for treating or lessening the severity of a disease, disorder, or condition, selected from allergy, asthma, diabetes, Alzheimer's disease, Huntington's disease, Parkinson's disease, AIDS-associated dementia, amyotrophic lateral sclerosis (AML, Lou Gehrig's disease), multiple sclerosis (MS), schizophrenia, cardiomyocyte hypertrophy, reperfusion/ischemia, stroke, or baldness, comprising the step of administering to a patient in need thereof a composition according to the present invention..

According to a preferred embodiment, the invention is useful for treating or lessening the severity of stroke comprising the step of administering to a patient in need thereof a composition according to the present invention..

According to another preferred embodiment, the method of the present invention relates to treating or lessening the severity of a neurodegenerative or neurological disorder, comprising the step of administering to a patient in need thereof a composition according to the present invention..

According to another embodiment, the present invention relates to treating or lessening the severity of a disease, disorder, or condition, selected from autoimmune diseases, allergies, rheumatoid arthritis, or leukemia, comprising the step of administering to a patient in need thereof a composition according to the present invention.

According to another embodiment, the present invention relates to treating or lessening the severity of transplant rejection, comprising the step of administering to a patient in need thereof a composition according to the present invention.

In an alternate embodiment, the invention utilizes compositions that do not contain an additional therapeutic agent, comprise the additional step of separately administering to said patient an additional therapeutic agent. When these additional therapeutic agents are administered separately they may be administered to the patient prior to, sequentially with or following administration of the compositions of this invention.

In order that the invention described herein may be more fully understood, the following examples are set forth. It should be understood that these examples are for illustrative purposes only and are not to be construed as limiting this invention in any manner.

### EXAMPLES

¹H NMR spectra were recorded at 400MHz using a Bruker DPX 400 instrument. ¹³C NMR spectra were recorded at 100MHz using the same instrument. LC/MS data were obtained using a Micromass ZQ instrument with atmospheric pressure chemical ionisation. HPLC analysis were performed on a Phenomenex C₁₈₍₂₎ Luna column (30 x 4.6 mm) maintained at 40°C. Samples were prepared as solutions in acetonitrile with approximate concentration of 1mg/mL. Each sample of 1-5 µL was injected into the system. The compound was eluted using the following gradient at a flow rate of 2 mL/min:
0 min, 80% H₂O-20%MeCN,
2.5 min, O% H₂O-100%MeCN,
3.5 min, 0% H₂O-100%MeCN

The eluant mixture was then returned to the starting conditions and the column re-equilibrated for 1 minute. Detection was via a diode array detector, the chromatograms for 214 and 254 being extracted. In all cases the elution time was identical for the two wavelengths.

All reagents were obtained commercially and used directly. DMF was dried over 4Å molecular sieves (Fisher Scientific). Column chromatography employed Silica Gel 60 (Fluka). TLC analysis was carried out using pre-coated plastic sheets Polygram SIL G/UV₂₅₄ (Macherey-Nagel). 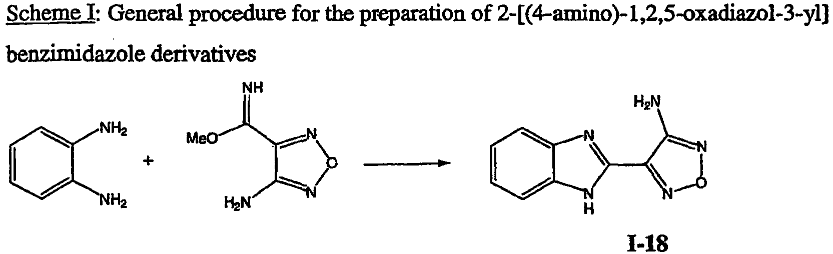 1,2-Diamine (2.7 mmol) was added to a solution of the amidate (4-amino-furazan-3-carboximidic acid methyl ester) (T.Ichikawa, T.Kato, T.Takenishi; J.Heterocycl.Chem., 1965, 2, 253-255. V.G.Andrianov, A.V.Eremeev, Chem.Heterocycl.Compd., 1994, 30, 608-611. I.V.Tselinskii, S.F.Mel'nikova, S.V.Pirogov, A.V.Sergievskii, Russ.J.Org. Chem. 1999, 35, 296-300) (2.7 mmol) in methanol (8 mL). Glacial acetic acid (4 mL) was then added to this solution and the reaction mixture heated at 65-70°C (an oil bath temperature) for 18 hours. The products crystallized from the mixture and were separated by filtration, washed with Et₂O or Et₂O/petroleum ether (40-60°C) and dried. The yields are given in Table 2, below.
**2-[(4-Amino)-1,2,5-oxadiazol-3-yl]benzimidazole (I-18):** Isolated as a pale yellow solid. LC/MS: 202 (M⁺+1), retention time: 1.46 min. δ_{H} (DMSO): 6.79 (2H, s, NH₂), 7.31 (2H, m, ArH), 7.53 (1H, d, ArH), 7.77 (1H, d, ArH and 13.65 (1H, s, NH).
**2-[(4-Amino)-1,2,5-oxadiazol-3-yl]-5-methoxy benzimidazole (I-44):** Isolated as a brown solid. LC/MS: 232 (M⁺+1), retention time: 1.52 min. δ_{H} (DMSO): 3.73 (3H, s, MeO), 6.72 (2H, s, NH₂), 6.87 (1H, d, ArH), 7.02 (1H, bs, ArH) and 7.51 (1H, bd, ArH). The N-H signal was not observed.
**2-[(4-Amino)-1,2,5-oxadiazol-3-yl]-4-nitro benzimidazole (I-48):** Isolated as an orange solid. LC/MS: 247 (M⁺+1), retention time: 1.52 min. δ_{H} (DMSO): 6.70 (2Ks, NH₂), 7.41 (1H, m, ArH), 8.09 (2H, m, ArH) and 14.20 (1H, bs, NH).
**2-[(4-Amino)-1,2,5-oxadiazol-3-yl]-4-hydroxy Benzimidazole (I-52):** Compound I-52 was prepared as described above, except was obtained after evaporation of the solvents. Isolated as a yellow solid. LC/MS: 218 (M⁺+1), retention time: 1.03 min. δ_{H} (DMSO): 6.88 (1H, m, ArH), 7.18-7.41 (4H, t+bs, 2 x ArR+NH₂), 11.99 (1H, bs, OH) and 13.78 (1H, bs, NH).

**Table 2: Yields for selected compounds**

| | | | | | | | |
|---|---|---|---|---|---|---|---|
| 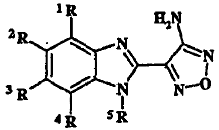 | | | | | | | |

| **#** | **entry** | **¹R** | **²R** | **³R** | **⁴R** | **⁵R** | **Yield (%)** |
|---|---|---|---|---|---|---|---|
| **I-18** | **a** | H | H | H | H | H | 76 |
| **I-44** | **b** | H | OMe | H | H | H | 72 |
| **I-48** | **c** | NO₂ | H | H | H | H | 56 |
| **I-52** | **d** | OH | H | H | H | H | 95 |
| **I-38** | **e** | H | H | H | H | 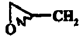 | 40^{c} |
| **I-32** | **f** | H | H | H | H | 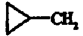 | 90 |
| **I-33** | **g** | H | H | H | H | 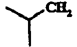 | 70 |
| **I-37** | **h** | H | H | H | H | CF₃CH₂ | 18 |
| **I-54** | **i** | H | H | H | H | 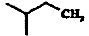 | 95 |
| **I-50** | **j** | H | H | H | H | Ph | 73 |
| **I-51** | **k** | H | H | H | H | 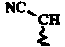 | 17^{c} |
| **I-56** | **l** | NO₂ | H | H | H | NCCH₂ | 14 |
| **I-47** | **m** | H | OMe | H | H | NCCH₂ | 95^{a} |
| | | H | H | OMe | H | | |
| **I-86** | **n** | H | OMe | H | H | 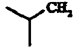 | 95^{a} |
| | | H | H | OMe | H | | |
| **I-58** | **o** | NCCH₂O | H | H | H | NCCH₂ | 28^{b} |
| | | H | H | H | NCCH₂O | | |

| | | | | | | | |
|---|---|---|---|---|---|---|---|
| ^{a}The product was isolated as a 1.5:1 mixture of the 5-methoxy and 6-methoxy derivatives. ^{b} The product was isolated as a 2: mixture of the 4-cyanomethoxy and 7-cyanomethoxy derivatives. ^{c}The product was isolated as a racemic mixture. | | | | | | | |

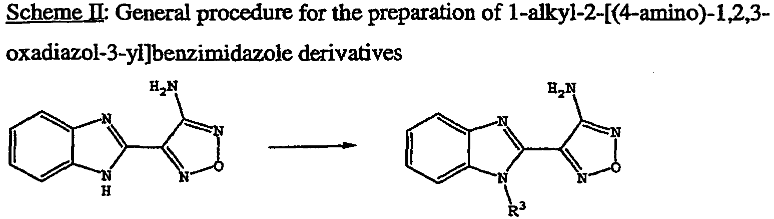

NaH (0.22 mmol, 60% in mineral oil) was added in portions at room temperature to a stirred solution of benzimidazole (or other hetero-fused imidazole) (0.2 mmol) in DMF (3 mL). After the addition, the mixture was stirred at room temperature for 45minutes. To this mixture the alkylating agent (0.44-0.66 mmol) was added dropwise and the mixture was then warmed to 60-65°C (oil bath temperature) and stirred for an additional 3h (when chloroacetonitrile was used as the alkylating agent) or 18h (when other alkylating agents were used). The reaction mixture was allowed to cool to room temperature, diluted with Et₂O (30-40 mL) and washed with water (3x5 mL). The ethereal layer was dried (MgSO₄), the solvent evaporated under reduced pressure and the residue purified either by crystallization or by flash chromatography to afford the product. The yields are given in Table 3.
The following alkylating agents (4) were used to prepare the present compounds: 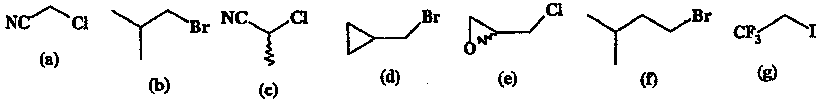
**1-Cyclopropylmethyl-2-[(4-amino)-1,2,5-oxadiazol-3-yl]benzimidazole (I-32):** Isolated as a colorless solid. LC/MS: 256 (M⁺+1), retention time: 2.14 min. δ_{H}(DMSO): 0.39 (4H, m, CH₂CH₂), 1.28 (1H, m, CH), 4.51 (2H, d, NCH₂), 6.98 (2H, S, NH₂), 7.28 (1H, t, ArH), 7.37 (1H, t, ArH) and 7.78 (d, 2H, ArH).
**1-(2-Methyl)propyl-2-[(4-amino)-1,2,5-oxadiazol-3-yl]benzimidazole (I-33):** Isolated as a colorless solid. LC/MS: 258 (M⁺+1), retention time: 2.24 min. δ_{H} (DMSO): 0.89 (6H, d, 2 x CH₃), 2.22 (1H, m, CH), 4.48 (2H, d, NCH₂), 7.01 (2H, s, NH₂), 7.29-7.47 (2H, m, ArH) and 7.82 (2H, t, ArH).
**1-(2,2,2-Tritfluoro)ethyl-2-[(4-amino)-1,2,5-oxadiazol-3-yl]benzimidazole (I-37):** Isolated as a colorless solid. LC/MS: 284 (M⁺+1), retention time: 2.01 min. δ_{H} @MSO): 5.52 (2H, m, NCH₂), 6.83 (2H, s, NH₂), 7.31-7.40 (2H, 2 x t, ArH) and 7.73 (2H, m, ArH).
**1-(3-Methyl)butyl-2-[(4-amino)-1,2,5-oxadiazol-3-yl]benzimidazole (I-54):** Isolated as a colorless solid. LC/MS: 272 (M⁺+1), retention time: 2.41 min. δ_{H}(DMSO): 0.98 (6H, d, 2 x CH₃), 1.69 (3H, m, CHCH₂), 4.70 (2H, dd, NCH₂), 6.99 (2H, s, NH₂), 7.34 (1H, t, ArH), 7.42 (1H, t, ArH), 7.73 (1H, d, ArH) and 7.80 (1H, d, ArH).
**1-(2-Cyano)propyl-2-[(4-amino)-1,2,5-oxadiazol-3-yl]benzimidazole (I-51):** Isolated as a colorless solid. LC/MS: 255 (M⁺+1), retention time: 1.28 min. δ_{H}(DMSO): 2.04 (3H, d, CH₃), 6.78 (1H, q, NCCH), 6.98 (2H, s, NH₂), 7.50 (1H, t, ArH), 7.59 (1H, t, ArH) and 7.98 (2H, m, ArH).
**1-Cyanomethyl-2-[(4-amino)-1,2,5-oxadiazol-3-yl]-4-nitro benzimidazole (I-56):** Isolated as a yellow solid. LC/MS: M⁺ ion not observed, retention time: 1.63 min. δ_{H} (DMSO): 5.88 (2H, s, NCCH₂), 6.92 (2H, s, NH₂), 7.63 (1H, t, ArH), 8.18 (1H, d, ArH) and 8.32 (1H, d, ArH).
**1-Cyanomethyl-2-[(4-amino)-1,2,5-oxadiazol-3-yl]-5/6-methoxy benzimidazole (I-47):** Isolated as a pale brown solid. LC/MS: 271 (M⁺+1), retention time: 1.65 min. δ_{H} (DMSO): 3.84 (2 x 3H, 2 x s, MeO, isomers A+B), 5.88 (2 x 2H, 2 x s, NCCH₂, isomers A+B), 6.90 (2 x 2H, 2 x s, NH₂, isomers A+B), 7.04 (1H, m, ArH, isomer A), 7.16 (1H, m, ArH, isomer B), 7.39 (1H, d, ArH, isomer B), 7.58 (1H, d, ArH, isomer A), 7.74 (1H, d, ArH, isomer A) and 7.82 (1H, d, ArH, isomer B).
**1-(2-Methyl)propyl-2-[(4-amino)-1,2,5-oxadiazol-3-yl]-5/6-methoxy benzimidazole (I-86):** Isolated as a yellow solid. LC/MS: 288 (M⁺+1), retention time: 2.19 min. δ_{H} (DMSO): 0.91 (2 x 6H, m, CH₃CHCH_{3,} isomers A+B), 2.22 (2 x 1H, m, CHCH₂, isomers A+B), 3.86 (2 x 3H, 2 x s, OMe, isomers A+B), 4.49 (2 x 2H, m, CH₂, isomers A+B), 6.96-7.09 (2 x 3H, m, NH₂ + ArH, isomers A+B), 7.31 (2 x 1H, m, ArH, isomers A+B) and 7.73 (2 x 1H, m, ArH, isomers A+B).
**1-Cyanomethyl-2-[(4-amino)-1,2,5-oxadiazol-3-yl]-4/7 - cyanomethoxy benzimidazole (I-58):** Isolated as a yellow solid. LC/MS: 296 (M⁺+1), retention time: 1.56 min. δ_{H} (DMSO): 5.36 (2 x 2H, 2 x s, NCH₂, isomers A+B), 5.79 (2 x 2H, 2 x s, OCH₂, isomers A+B), 6.88 (2 x 2H, 2 x s, NH₂, isomers A+B), 6.99 (1H, d, ArH, isomer A), 7.12 (1H, d, ArH, isomer B), 7.29 (1H, d, ArH, isomer B), 7.37 (1H, t, ArH, isomer A) 7.46 (1H, d, Arm isomer B) and 7.55 (1H, d, ArH, isomer A).
**1-Oxiranylmetllyl-2-[(4-amino)-1,2,5-oxadiazol-3-yl]benzimdazole (I-38):** A mixture of benzimidazole (I-18) (0.08 g, 0.4 mmol), (*R,S*) epichlorohydrin (0.11 g, 1.2 mmol), NaI (0.006 g, 0.04 mmol) and K₂CO₃ (0.17 g, 1.2 mmol) in DMF (5 mL) was heated at 70-80°C for 18hours. The mixture was then cooled to room temperature and the solid was separated by filtration and washed with Et₂O. The filtrate was diluted with Et₂O (~40 mL), washed with H₂O and dried (MgSO₄). The solvent was evaporated under reduced pressure and the residue purified by flash chromatography (petroleum ether:ether 1:1 v/v) to afford the product (0.04 g, 40%) as a colorless solid. LC/MS: 258 (M⁺+1), retention time: 1.67 min. δ_{H}(DMSO): 2.41 (1H, m), 2.60 (1H, m), 3.28 (1H, m), 4.56 (1H, dd), 4.98 (1H, m), 6.80 (2H, s, NH₂), 7.19-7.29 (2H, 2 x t, ArH) and 7.60-7.69 (2H, 2 x d, ArH).
**1-Phenyl-2-[(4-amino)-1,2,5-oxadiazol-3-yl]benzimidazole (I-50):** This compound was prepared according to the general procedure for the preparation of benzimidazole (I-18). N-phenyl-N-(2-amino)phenyl amine was used as the *bis*-amino component The product was isolated as a grey solid. LC/MS: 278 (M⁺+1), retention time: 2.14 min. δ_{H}(DMSO): 6.79 (2H, s, NH₂), 7.08 (1H, m, ArH), 7.28 (2H, m ArH), 7.41-7.50 (5H. m, ArH) and 7.80 (1H, m, ArH). 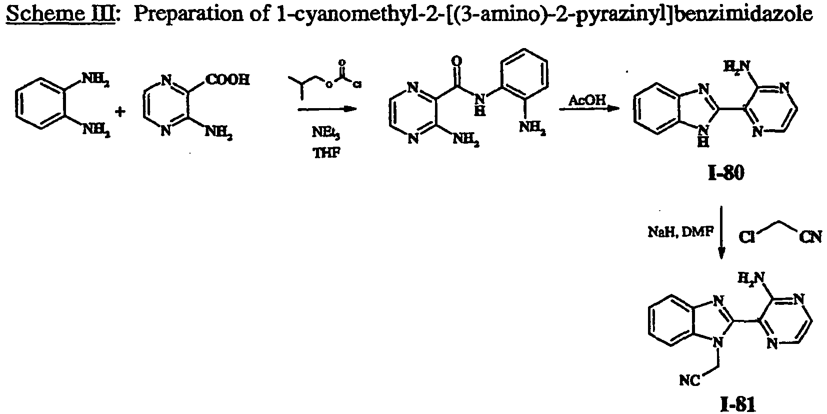
**N-(2-Aminophenyl)-3-aminopyrazine-2-carboxamide:** Triethylamine (0.22 g, 2.18 mmol) was added dropwise to a suspension of 3-amino-pyrazine-2-carboxylic acid (0.28 g, 2.0 mmol) in THF (20 mL). The mixture was cooled to 0-5°C using an ice bath and isobutylchloro formate (0.29 g, 2.12 mmol) was added dropwise over a period of 10-15min. The mixture was stirred for additional 3h at 0-5°C. 1,2-Diaminobenzene (0.22 g, 2.0 mmol) was then added in one portion and the mixture was slowly warmed to room temperature and stirred for 18hours. The reaction mixture was then diluted with CH₂Cl₂ (~50mL), washed with water, dried (MgSO₄) and the solvent evaporated under reduced pressure. The solidified residue was washed with a small amount of Et₂O to afford the product (0.34 g, 74%) as a yellow solid which was used in the next step without further purification. δ_{H}(DMSO): 5.01 (2H, s, NH₂), 6.78 (1H, t, ArH), 7.96 (1H, d, ArH), 7.09 (1H, t, ArH), 7.57 (1H, d, ArH) 7.70 (2H, bs, NH₂), 8.07 (1H, s, pyrazine-H), 8.42 (1H, s, pyrazine-H) and 10.01 (1H, s, NH).
**2-[(3-Amino)-2-pyrazinyl]benzimidazole (I-80):** A solution of N-(2-Aminophenyl)-3-aminopyrazine-2-carboxamide (0.15 g, 0.66 mmol) in acetic acid (6 mL) was heated at 100-110°C for 4h. The reaction mixture was cooled to room temperature and water (10mL) was added. The precipitated product was separated by filtration, washed with cold water and dissolved in EtOAc. The EtOAc solution was dried (MgSO₄) and the solvent evaporated to afford the product (I-80) (0.12 g, 87%) as a yellow solid. LC/MS: 212 (M⁺+1), retention time: 1.51 min. δ_{H}(DMSO): 7.13 (2H, m, ArH), 7.44 (1H, d, ArH), 7.63 (1H, d, ArH), 7.88 (1H, s, pyrazine-H), 8.06 (1H, s, pyrazine-H) and 12.99 (1H, s, NH). The NH₂ signal was not observed.
**1-Cyanomethyl-2-[(3-amino)-2-pyrazinyl]benzimidazole(I-81):** Alkylation of compound 2-[(3-Amino)-2-pyrazinyl]benzimidazole (I-80) (0.06 g, 0.29 mmol) under the standard conditions afforded (10) (0.04 g, 60%) as a yellow solid. LC/MS: 251 (M⁺+1), retention time: 1.63 min. δ_{H}(DMSO): 6.28 (2H, s, NCCH₂), 7.59-7.70 (2 x 1H, 2 x t, ArH) 8.07 (2H, m, ArH), 8.26 (1H, d, pyrazine-H) and 8.41 (1H, d, pyrazine-H). The NH₂ signal was not observed.

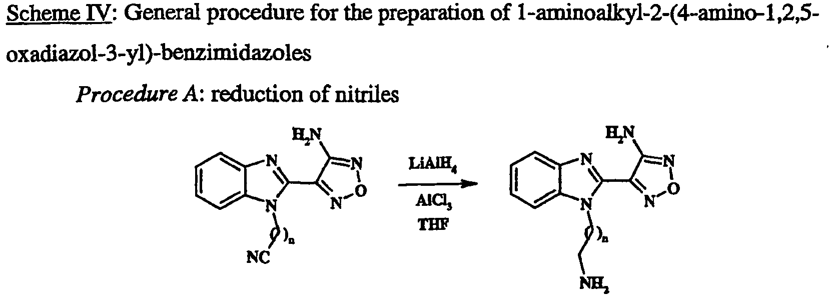

A solution of AlCl₃ (0.5mmol) in dry THF (2mL) was added dropwise under nitrogen atmosphere to a stirred solution of LiAlH₄ (0.5mL 1M solution in THF, 0.5mmol,) at room temperature and the mixture stirred for 5minutes. The nitrile starting material (0.25mmol) dissolved in dry THF (1mL) was then added dropwise, the mixture gently refluxed for 18h and then cooled to room temperature. H₂O (5ml) was carefully added, the mixture basified with aqueous NaOH (2M solution) and then extracted (Et₂O). The extract was dried (MgSO₄) and the solvent evaporated under reduced pressure. The residue was purified by flash chromatography (SiO₂, CH₂Cl₂/MeOH or Et₂O/NH₄OH) to afford the product amine.
The following compounds have been made by this procedure: 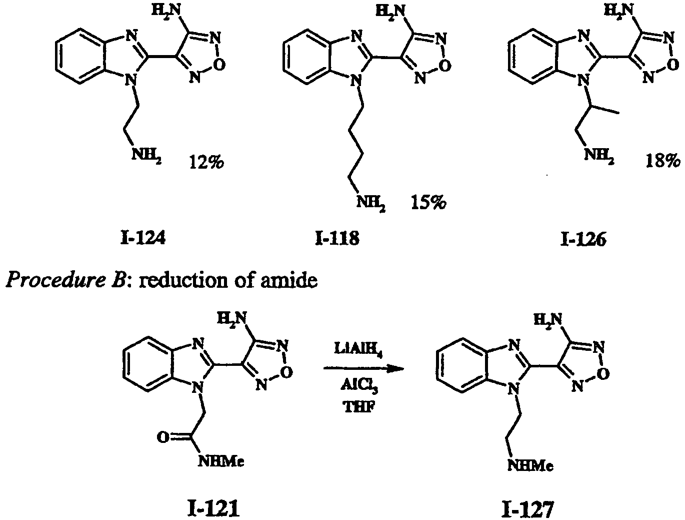 The above procedure (*Procedure A*) has been used for the reduction of amide **(I-121).** Compound **(I-127)** was isolated in 11% yield.

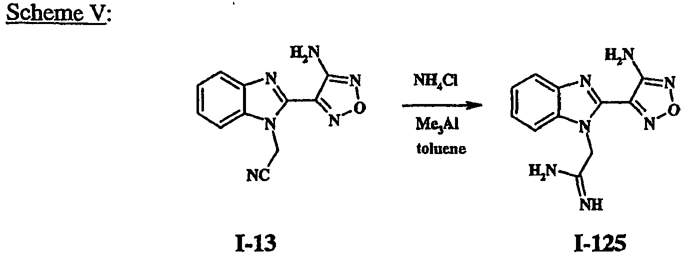

**2-(4-Amino)-1,2,5-oxadiazol-3-yl benzoimidazol-lyl acetamidine (I-125):** Me₃Al (0.42mL, 2M solution in hexanes, 0.84mmol) was added dropwise under N₂ atmosphere to a stirred suspension of NH₄Cl (0.044g, 0.84mmol) in toluene (4ml) at 0°C and the mixture stirred for additional 30minutes. Benzimidazole **(I-13)** (0.05g, 0.21mmol) was then added in several portions and the mixture was slowly warmed to room temperature and then refluxed for 18hours. The reaction mixture was cooled to room temperature and a suspension of SiO₂ (2g) in CH₂Cl₂ (3ml) added. SiO₂ was separated by filtration and washed with MeOH/CH₂Cl₂ (20mL, 50%). The filtrate was separated and the solvent evaporated under reduced pressure. The solid residue was suspended in water (2ml), the insoluble solid then separated by filtration, washed with Et₂O and dried to afford the product **(I-125),** (0.018g, 34%).

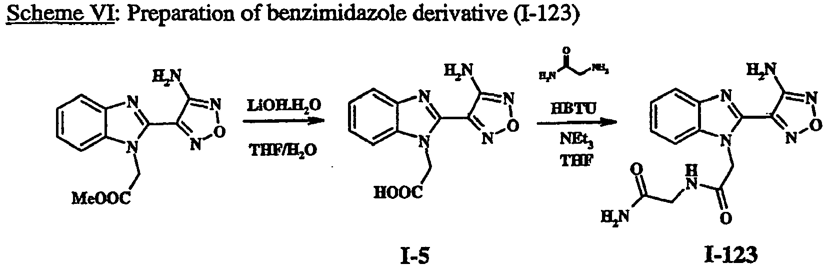

**[2-(4-Amino-furazan-3-yl)benzoimidazol-1-yl]-acetic acid (I-5): A solution of** LiOH.H₂O (0.032g, 0.77mmol) in H₂O (3ml) was added to a solution of [2-(4-amino-furazan-3-yl)-benzoimidazol-l-yl]-acetic acid methyl ester (0.2g, 0.73mmol) in THF (5ml) at 0°C. The mixture was warmed to room temperature and stirred for 18hours. The reaction mixture was then diluted with H₂O (10ml), acidified with citric acid (pH=3-4) and extracted (Et₂O). The extract was dried (MgSO₄ and the solvent evaporated under reduced pressure to afford the product **(I-5)** (0.14g, 74%).

**I-123:** Acid **(I-5)** (0.08g, 0.31mmol) was added to a suspension of glycine amide (0.038g, 0.34mmol) in dry THF under N₂ atmosphere (15mL) followed by NEt₃ (0.073g, 0.72mmol). The mixture was cooled to 0°C and HBTU (0.13g, 0.34mmol) was added in one portion. After being stirred at 0°C for 45 minutes, the mixture was slowly warmed to room temperature and then stirred for an additional 72hours. The solid precipitated from the mixture was separated by filtration and washed with a small amount of MeOH to afford the product **(I-123)** (0.05g 51%).

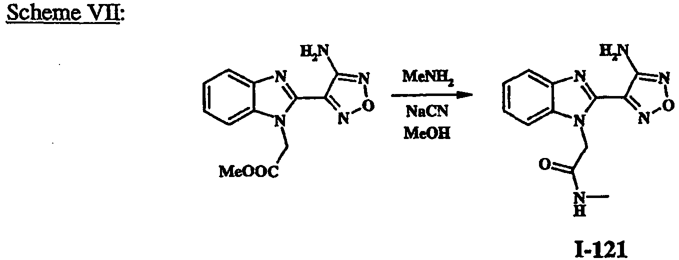

**2-(4-Amino-1,2,5-oxadiazol-3-yl)benzimidazol-1-yl N-methylacetamide (I-121):** To a solution of [2-(4-amino-furazan-3-yl)-benzoimidazol-1-yl]-acetic acid methyl ester (0.3g, 1.1mmol) in MeOH (3ml), methylamine (1.6mL 2M solution in MeOH, 0.032mol) was added followed by NaCN (5.5mg, 0.11mmol). The reaction mixture was heated at 50°C (oil bath temperature) for 18hour. The mixture was then cooled to room temperature, diluted with ether (30ml) and washed with 1M HCl. The organic phase was dried (MgSO₄), the solvent evaporated under reduced pressure and the residue crystallized (Et₂O/petroleum ether) to afford the product **(I-121)** (0.2g, 67%).

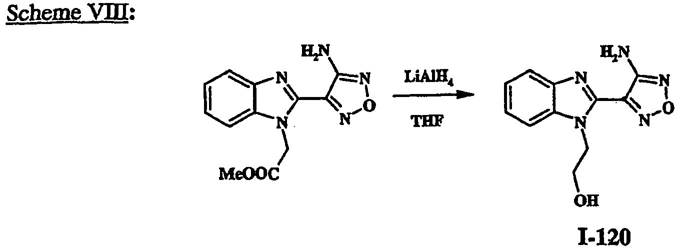

**2-(4-Amino-1,2,5-oxadiazol-3-yl)benzimidazol-1-yl ethanol (I-120):** To a solution of [2-(4-amino-furazan-3-yl)-benzoimidazol-1-yl]-acetic acid methyl ester (0.085g, 0.31mmol) in dry THF (5ml) under N₂ atmosphere, a solution of LiAlH₄ (0.31ml 1M solution in Et₂O, 0.31mmol) was added dropwise and the mixture was stirred for 18h at room temperature. A saturated solution of K,Na-tartarate (5ml) was added to the mixture and the solid formed separated by filtration. The filtrate was diluted with Et₂O (30ml) and washed with H₂O. The organic phase was dried (MgSO₄) the solvent evaporated under reduced pressure and the solid residue recrystalised (CH₂Cl₂/petroleum ether) to afford the product **(I-120)** (0.018g, 24%).

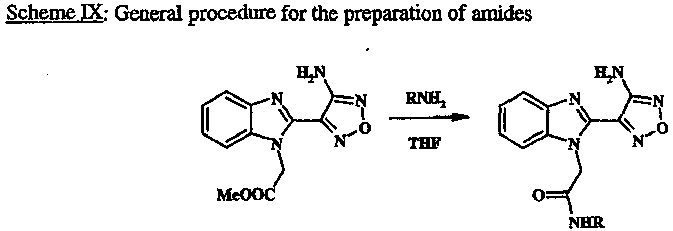

To a solution of [2-(4-amino-furazan-3-yl)-benzoimidazol-1-yl]-acetic acid methyl ester (0.18mmol) in MeOH (1ml), NaCN (0.018mmol) was added followed by the amine (5.9mmol) and the mixture heated in a sealed flask at 50°C (oil bath temperature) for 24hours. The solvent was then evaporated, the residue dissolved in CH₂Cl₂ and washed with brine. The organic phase was dried (MgSO₄) and the solvent evaporated under reduced pressure to afford the product amide.
The following compounds have been prepared by this procedure:

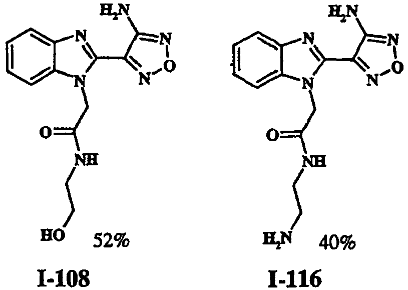

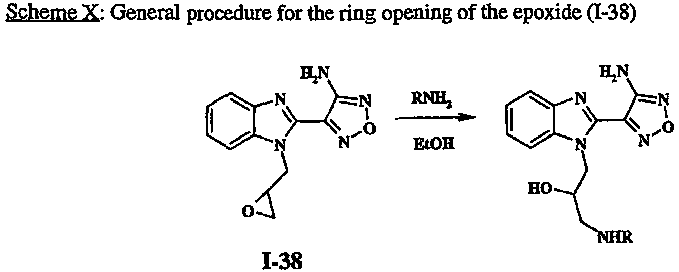

To a solution of epoxide (**I-38**) (0.19mmol) in EtOH (3ml), a solution of amine (3.8mmol of MeNH₂ or ~88mmol of NH₃) was added and the mixture stirred at room temperature for 24hours. The reaction mixture was concentrated under reduced pressure and then extracted (EtOAc). The extract was dried (MgSO₄) and the solvent evaporated under reduced pressure to afford the product amino alcohols.
The following compounds have been prepared by this procedure:

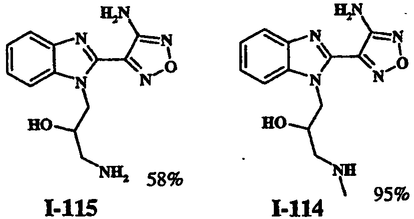

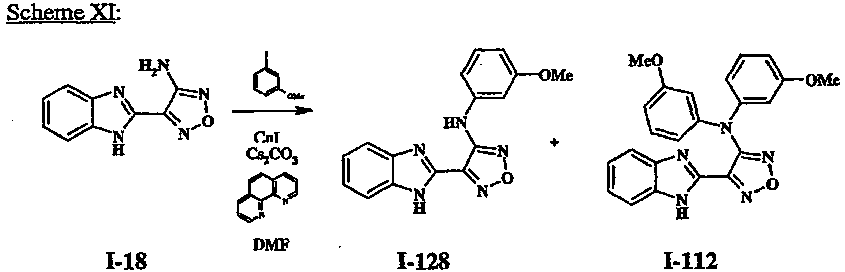

**2-[N-(3-Methoxyphenyl)-4-amino]-1,2,5-oxadiazol-3yl benzimidazole (I-128)**: To a solution of benzimidazole (**I-18**) (0.05g, 0.25mmol) in DMF (0.1mL) under N₂ atmosphere 3-iodoanisole (0.049g, 0.21mmol) was added followed by CuI (0.005g, 0.025mmol), phenanthroline (0.008g, 0.042mmol) and Cs₂CO₃ (0.142g, 0.44mmol), and the reaction mixture was then heated at 110°C (oil bath temperature) for 24hours. After being cooled to room temperature, the mixture was diluted with EtOAc (10ml), filtered through short plug of silica/celite and the plug washed (EtOAc). The solvent from the filtrate was evaporated under reduced pressure and the residue purified by flash chromatography (SiO₂, EtOAc/petroleum ether, v/v 5:95 to 20:80, a gradient elution) to afford the product (**I-128**) (0.038g, 50%). Further elution afforded the bisarylated product (**I-112**) in trace quantities.

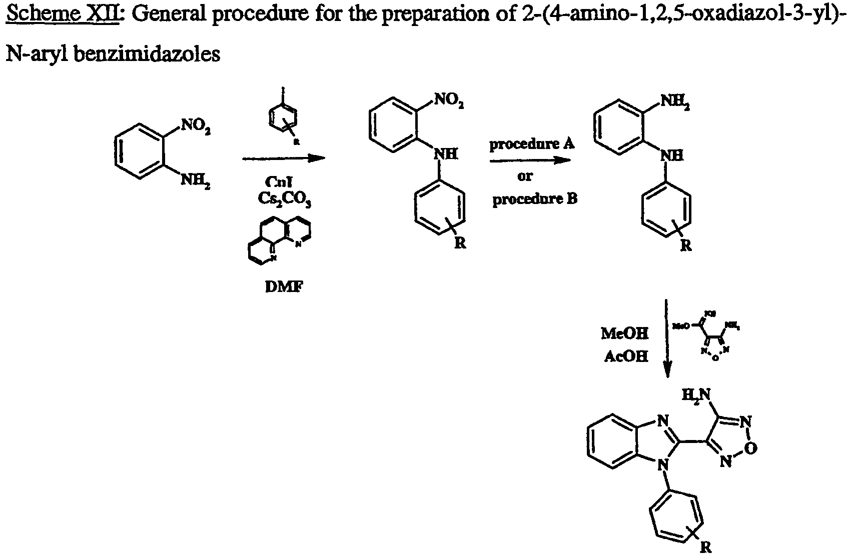

### Preparation of 2-(N-arylamino)-nitrobenzenes

To a solution of nitroaniline (5.8mmol) in DMF (2.5ml) under nitrogen atmosphere the iodobenzene derivative (4.8mmol) was added followed by CuI(0.48mmol), phenanthroline (0.96mmol) and Cs₂CO₃ (10.0mmol). The mixture was heated at 110°C (oil bath temperature) for 24hours. After being cooled to room temperature the mixture was diluted with EtOAc (15ml), filtered through a short plug of celite/silica and the plug washed with EtOAc. The solvent from the filtrate was evaporated under reduced pressure and the residue purified by flash chromatography (SiO₂, EtOAc/petroleum ether, v/v 1:9 to 2:8, a gradient elution) to afford the product 2-(N-arylamino)-nitrobenzenes.

### Procedure A: Preparation of N-Arylbenzene-1,2-diamines

To a solution of compound 2-(N-arylamino)-nitrobenzenes (1.0mol) in EtOH (10ml), Pd/C (0.13g) was added. The mixture was stirred under H₂ atmosphere at room temperature for 16hours. The reaction mixture was then filtered through celite and the celite plug was washed (EtOH). The solvent from the filtrate was evaporated under reduced pressure to afford the product, which was used in the next step without further purification.

### Procedure B

To a mixture of compound 2-(N-arylamino)-nitrobenzenes (0.6mmol) and conc. HCl (0.7ml), SnCl₂.2H₂O (3.33mmol) was added and the mixture stirred at 60°C (oil bath temperature) for 16hours. Most of the solvent was then evaporated under reduced pressure, the residue poured into ice/water, basified with aqueous NaOH (2M solution) and extracted (Et₂O). The extract was dried (MgSO₄) and the solvent evaporated under reduced pressure to afford the N-aylbenzene-1,2-diamine. The product was used in the next step without further purification.

### Preparation of compound (20)

Compound (**19**) was converted to the product (**20**) using the general procedure previously described for the preparation of benzimidazole derivatives.
The following compounds have been made by this procedure:

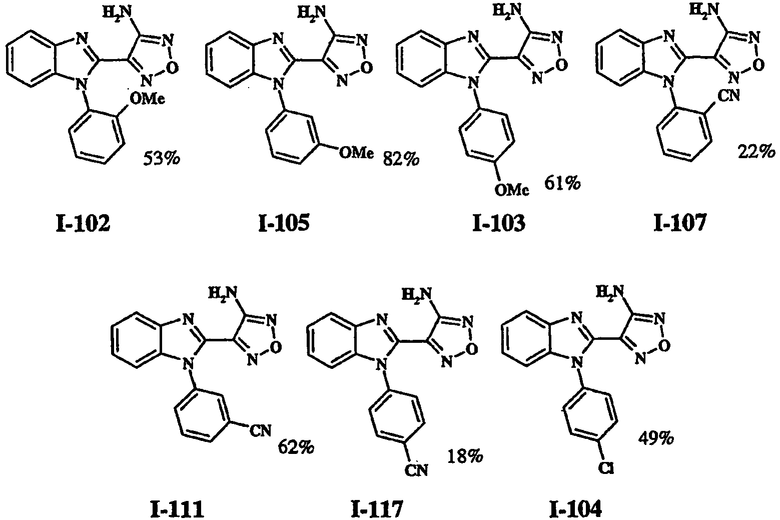

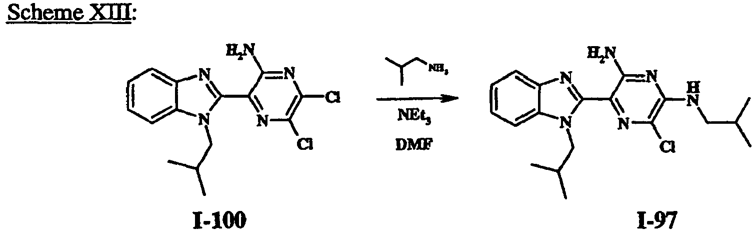

**2-(N-isobutyl)amino-3-chloro-5-(1-isobutyl)benzimidazol-2-yl- 2,6-diaminopyrazine** (**I-97**): To a solution of benzimidazole (**I-100**) (0.07g, 0.21mmol) in DMF (3ml) Et₃N (0.027g, 0.27mmol) was added followed by *iso*-butyl amine (0.015g, 0.21mmol) and the mixture was heated at 90°C (oil bath temperature) for 16hours. The reaction mixture was cooled to room temperature, diluted with Et₂O (30ml), washed with H₂O and dried (MgSO₄) The solvent was then evaporated under reduced pressure and the residue purified by flash chromatography (SiO₂, Et₂O/petroleum ether, v/v1:9) to afford the product (**I-97**) (0.039g, 50%).

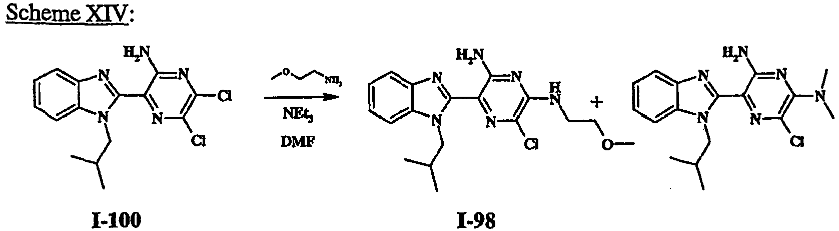

**2-(N-metboxyethyl)amino-3-chloro-5-(1-isobutyl)benzimidazol-2-yl-2,6-diaminopyrazine (I-98):** For the preparation of compound (**I-98**) the same experimental procedure as for the preparation of compound (**I-97**) was used. The crude reaction mixture was purified by flash chromatography (SiO₂, Et₂O/petroleum ether, 0:10 to 3:7, a gradient elution) to afford the product (**I-98**) (38%) and small amount of the dimethylamino by product (2%) (Scheme 15).

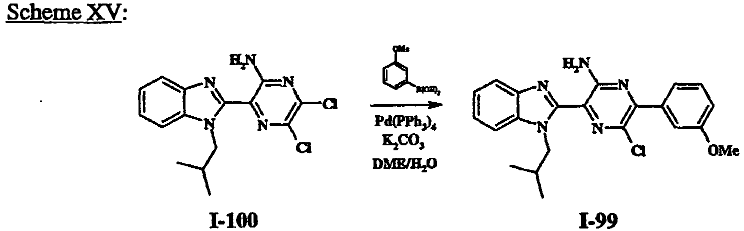

**2-(3-methoxy)phenyl-3-chloro-5-(1-isobutyl)benzimidazol-2-yl-6-aminopyrazine (I-99):** To a solution of the boronic acid (0.026g, 0.17mmol) in degassed DME (2ml) under N₂ atmosphere, benzimidazole derivative (**I-100**) (0.07g, 0.21mmol) was added followed by Pd(PPh₃)₄ (0.02g, 0:017mmol) and K₂CO₃ (0.072g, 0.52mmol) dissolved in degassed H₂O (1ml). The mixture was heated at 90°C (oil bath temperature) for 16hours. The reaction mixture was allowed to cool to room temperature and the organic layer separated. The water layer was extracted (EtOAc), the extract combined with the organic layer, dried (MgSO₄) and the solvent evaporated under reduced pressure. The residue was purified by flash chromatography (SiO₂, Et₂O/petroleum ether, v/v 5:95 to 20:80, a gradient elution) to afford the product (**I-99**) (0.03 1g, 44%).

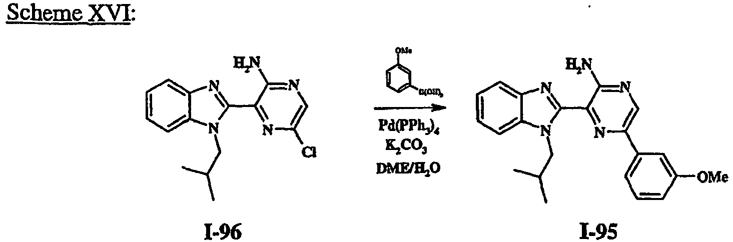

**5-(3-methoxy)phenyl-3-(1-isobutyl)benzimidazol-2-yl-2-aminopyrazine** (**I-95**) For the preparation of compound (**I-95**) the same experimental procedure as for the preparation of compound (**I-99**) was used. The crude reaction mixture was purified by flash chromatography (SiO₂, Et₂O/petroleum ether, v/v 1:9 to 2:8, a gradient elution) to afford the product (**I-95**) (33%).

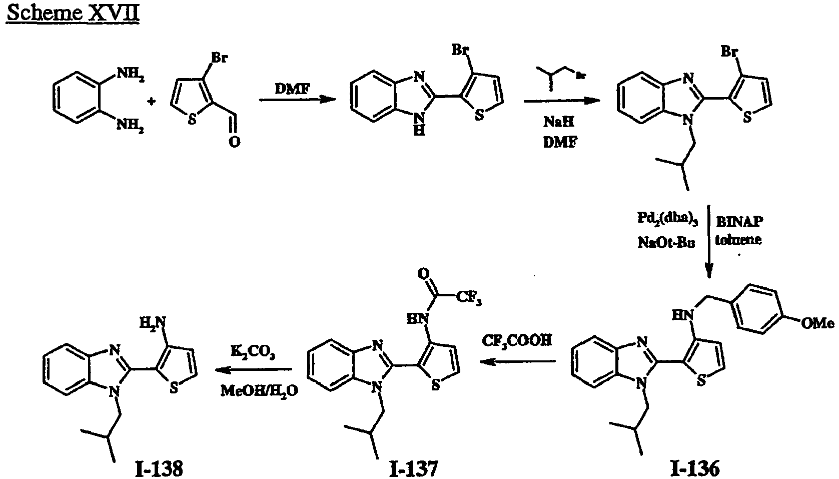

### Preparation of 2-(3-Bromo-thiophen-2-yl)-1H-benzimidazole

To a solution of 1,2-phenylenediamine(1.13g, 10.47mmol) in DMF (40ml), 3-bromothiophene carboxaldehyde (2g, 10.47mmol) was added and the mixture heated at 110°C for 16hours. The solvent was evaporated under reduced pressure and the reside partitioned between Et₂O (50ml) and brine (20ml). The Et₂O layer was separated and washed with H₂O. The combined brine and aqueous layers were extracted Et₂O), the extract combined with Et₂O layer, dried (MgSO₄) and the solvent evaporated under reduced pressure. The residue was purified by flash chromatography (SiO₂, Et₂O/petroleum ether, v/v 2:3) to afford 2-(3-bromo-thiophen-2-yl)-1H-benzimidazole (1.42g, 48%).

### Preparation of 2-(3-Bromo-thiophen-2-yl)-1-isobutyl-1H-benzimidazole

The alkylation of compound 2-(3-bromo-thiophen-2-yl)-1H-benzimidazole was carried out following the general procedure for the alkylation of benzimidazole derivatives. The product was isolated in 41% yield.

### Preparation of compound (I-136)

To a solution of compound 2-(3-bromo-thiophen-2-yl)-1-isobutyl-1H-benzoimidazole (0.404g, 1.21mmol) in toluene (16ml) 4-methoxybenzyl amine (0.198g, 1.45mmol) was added followed by NaO^{t}Bu (0.162g, 1.69mmol), BINAP (0.06g, 0.18mmol) and Pd₂(dba)₃ (0,03g, 0.06mmol) and the mixture was heated at reflux for 36hours. The reaction mixture was cooled to room temperature and the solvent evaporated under reduced pressure. The residue was partitioned between EtOAc and H₂O. The EtOAc layer was separated and the H₂O layer extracted (EtOAc). The extract was combined with the EtOAc layer, dried (MgSO₄) and the solvent evaporated under reduced pressure. The residue was purified by flash chromatography (SiO₂, EtOAc/petroleum ether, v/v 1:9) to afford the product (**I-136**) (0.117g, 25%).

### Preparation of compound (I-137)

Compound (**I-136**) (0.05g, 0.128mmol) was dissolved in TFA (1.5ml) and the mixture was heated at reflux for 1.5hours. After being cooled to room temperature, the mixture was diluted with H₂O (5ml), basified (aqueous NH₃) and then extracted (EtOAc). The extract was dried (MgSO₄), the solvent evaporated under reduced pressure and the residue purified by flash chromatography (SiO₂, EtOAc/petroleum ether, v/v1:4) to afford the product (**I-137**) (0.034g, 72%).

**1-Isobutyl-2-(3-amino)thiophen-2-yl benzimidazole (I-138):** To a mixture of K₂CO₃ (0.49g, 3.54mmol), H₂O (2ml) and MeOH (5ml), amide (**I-137**) (0.02g, 0.54mmol) was added and the mixture was heated at reflux for 2.5h. After being cooled to room temperature, the reaction mixture was concentrated under reduced pressure, diluted with H₂O (5ml) and then extracted (EtOAc). The extract was dried (MgSO₄) and the solvent evaporated under reduced pressure. The residue was purified by flash chromatography (SiO₂), EtOAc/petroleum ether, v/v 0: 10 to 4:6, gradient elution) to afford the product (**I-138**) (0.01g, 68%).

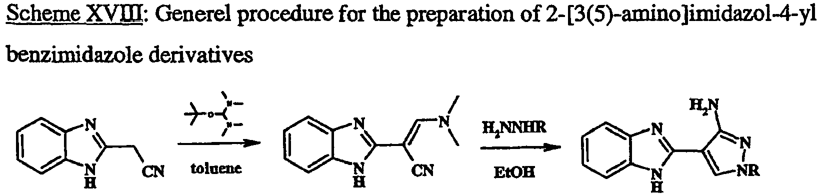

### Preparation of 2-(1H-Benzimidazol-2-yl)-3-dimethylamino-acrylonitrile

To a solution of (1H-Benzoimidazol-2-yl)-acetonitrile (0.25g, 1.6mmol) in toluene (5ml), Bredereck's reagent (0.33g, 1.92mmol) was added and the mixture was stirred at room temperature for 1hour 2-(1H-Benzoimidazol-2-yl)-3-dimethylamino-acrylo-nitrile (0.3g, 89%) precipitated from the reaction mixture and was separated by filtration, washed with petroleum ether and dried.

### Preparation of compounds 1-79 and I-131

To a solution of 2-(1H-Benzoimidazol-2-yl)-3-dimethylamino-acrylonitrile (0.99mmol) in EtOH (8ml) a hydrazine derivative (1.2-2mmol) was added and the mixture was heated at reflux for 16hours. The solvent was evaporated under reduced pressure and the residue purified by flash chromatography (SiO₂, CH₂Cl₂/MeOH) to afford the product.

The following compounds were prepared by this procedure:

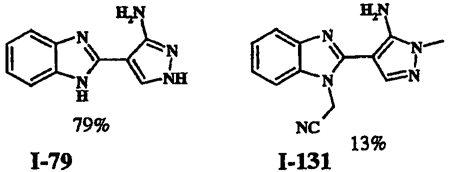

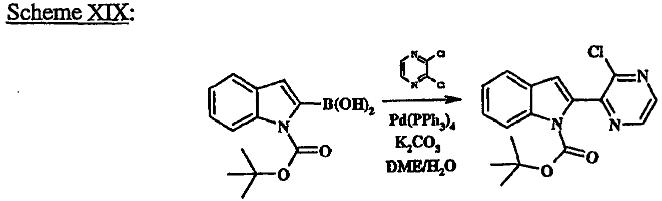

**1-(t-Butoxycarbonyl)-2-(3-chloro-pyrazin-2-yl)-indole:** To a solution of 1-(t-butoxycarbonyl)-indole-2-boronic acid (1.85mmol) in degassed DME (7ml) under N₂ atmosphere, pyrazine (2.22mmol) was added followed by K₂CO₃ (5.55mmol) dissolved in degassed H₂O (3ml) and Pd(PPh₃)₄ (0.185mmol) and the mixture heated at 90°C (oil bath temperature) for 16hours. The reaction mixture was then allowed to cool to room temperature, the organic layer separated and the H₂O layer extracted (EtOAc). The combined extract and organic layer were dried (MgSO₄) and the solvent evaporated under reduced pressure. The residue was purified by flash chromatography (SiO₂, Et₂O/petroleum ether) to afford 1-(t-butoxycarbonyl)-2-(3-chloro-pyrazin-2-yl)-indole.

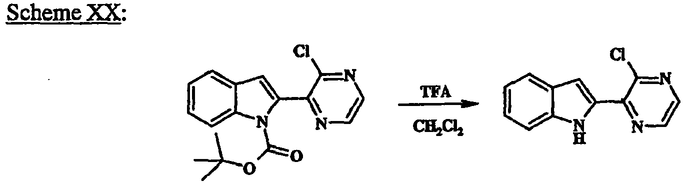

**2-(3-Chloro-pyrazin-2-yl)-indole:** To a solution of 1-(t-butoxycmbonyl)-2-(3-chloro-pyrazin-2-yl)-indole (0.62g, 1.9mmol) in CH₂Cl₂ (20ml), TFA (10ml) was added dropwise at 0°C. The mixture was allowed to warm to room temperature and then stirred for 16hours. The reaction mixture was diluted with CH₂Cl₂ (20ml), washed with the saturated aqueous NaHCO₃ solution and dried (MgSO₄). The solvent was then evaporated under reduced pressure to afford 2-(3-chloro-pyrazin-2-yl)-indole (0.41g, 95%)

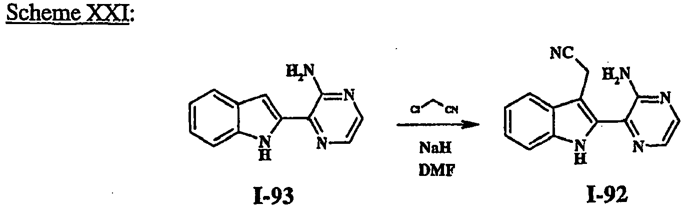

**[2-(3-Amino-pyrazin-2-yl)-1H-indol-3-yl]-acetonitrile (I-92):** To a solution of 2-(3-amino-pyrazin-2-yl-indole (0.03g, 0.19mmol)) in DMF (6ml), NaH (0.008g 60% in mineral oil, 0.21mmol) was added at room temperature. The mixture was stirred for an additional 45min and the chloride (0.028g, 0.38mmol) was added dropwise. The reaction mixture was stirred at room temperature for 16h and at 60°C (oil bath temperature) for 1hour, and then cooled to room temperature, diluted with Et₂O (30ml), washed with H₂O and dried (MgSO₄). The solvent was evaporated under reduced pressure and the residue purified by flash chromatography (SiO₂, ether) to afford the product (**I-92**) (0.006g, 17%/63% based on the recovered starting material).

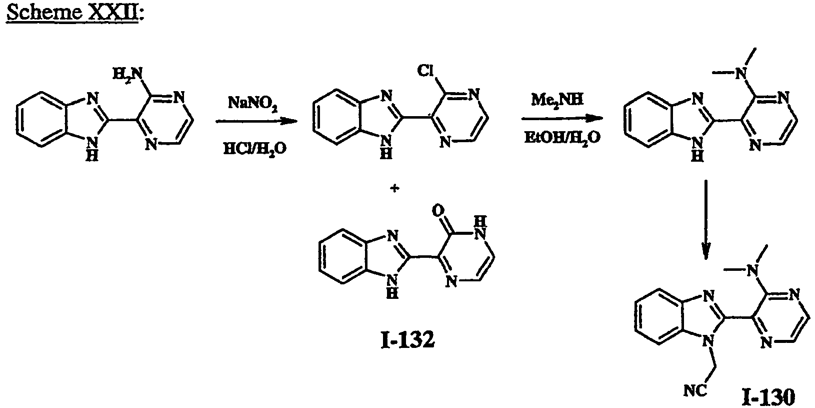

**2-(3-Chloro-pyrazin-2-yl)-1H-benzimidazole:** To a solution of 3-(1H-benzoimidazol-2-yl)-pyrazin-2-ylamine (0.15g, 0.71mmol) in a mixture of conc. HCl (3mL) and H₂O (3mL), a solution of NaNO₂ (0.059g, 0.85mmol) in H₂O (2mL) was added dropwise at 0°C. The mixture was stirred for 1hour at 0°C (yellow precipitate was formed), diluted with H₂O (5mL), basified with K₂CO₃ to pH 7 and extracted (EtOAc). The extract was dried (MgSO₄) and the solvent evaporated under reduced pressure. The residue was purified by flash chromatography (SiO₂, Et₂O) to afford the chloro pyrazine product (0.063mg, 39%). Further elution afforded the pyrazinone byproduct (**I-132**) (0.042g 28%).

**2-(3-Dimethylamino-pyrazin-2-yl)-1H-benzimidazole:** To a solution of 2-(3-chloro-pyrazin-2-yl)-1H-benzimidazole (0.039g, 0.17mmol) in EtOH (1ml), dimethylamine solution (5ml, 60% solution in H₂O, 53.0mmol) was added and the reaction mixture heated in sealed tube at 200°C for 45min under microwave conditions (300W, 300psi). After being cooled to room temperature, the mixture was extracted (EtOAc), the extract dried (MgSO₄) and the solvent evaporated under reduced pressure to afford 2-(3-dimethylamino-pyrazin-2-yl)-1H-benz-imidazole (0.05g, 100%).

**2-N,N-Dimethylamino-3-(1-cyanomethyl benzimidazol-2-yl)pyrazine (I-130):** 2-(3-Dimethylamino-pyrazin-2-yl)-1H-benz-imidazole was alkylated according to general procedure for the alkylation of benzimidazole derivatives.

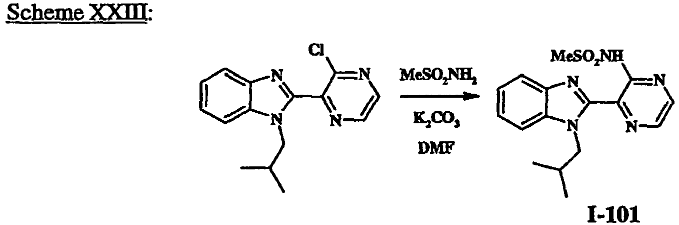

**3-(1-Isobutyl benzimidazol-2-yl)-pyrazin-2-ylamine methylsulfonamide (I-101):** To a solution of 2-(3-chloro-pyrazin-2-yl)-1-isobutyl-1H-benzoimidazole (0.11g, 0.384mmol) in DMF (8mL), MeSO₂NH₂ (0.033g, 0.346mmol) was added followed by K₂CO₃ (0.106g, 0.768mmol) and the mixture heated at 125°C (oil bath temperature) for 16 hours, cooled to room temperature and the solid separated by filtration. The solvent from the filtrate was evaporated under reduced pressure and the residue purified by flash chromatography (SiO₂, EtOAc/petroleum ether, v/v 2:8 to 3:7, gradient elution) to afford the product I-101 (0.01g, 9%/18% based on the recovered starting material).

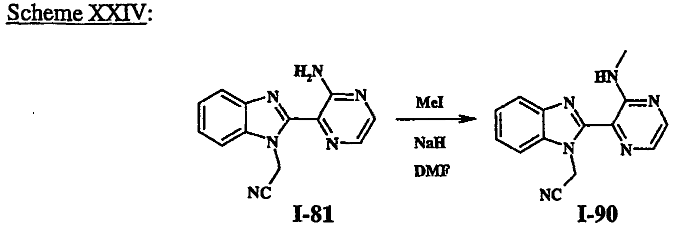

**3-(1-Cyanomethyl benzimidazol-2-yl)-pyrazin-2-yl methylamine (I-90):** To a solution of amine (**I-81**) (0.05g, 0.2mmol) in dry DMF (1.5ml) under N₂-atmosphere, NaH (0.01g, 0.24mmol, 60% in mineral oil) was added at room temperature and the mixture stirred for 30min. MeI (0.071g, 0.4mmol) was then added and the mixture stirred for additional 2hours. A saturated solution of NH₄Cl (4ml) was added and the mixture extracted (EtOAc). The extract was washed with H₂O, dried (MgSO₄) and the solvent evaporated under reduced pressure. The residue was purified by flash chromatography (SiO₂, EtOAc/petroleum ether, gradient elution) to afford the product (0.026g) contaminated by some byproducts (20% by LC-MS). Further HPLC purification afforded the product I-90 (0.009g, 17%).

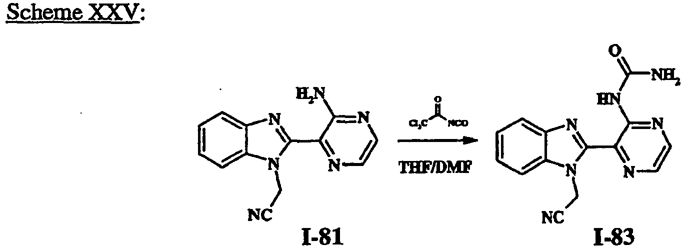

**3-(1-Cyanomethyl benzimidazol-2-yl)-pyrazin-2-yl urea (I-83):** To a solution of amine (**I-81**) (0.05g, 0.237mmol) in THF/DMF (2.5mL/2mL) under N₂-atmosphere, isocyanate (0,136g, 0.83mmol) was added dropwise at 0°C and the mixture stirred for 1hour. MeOH (1.5mL) and saturated aqueous solution of NaHCO₃ (1.5mL) were added and the mixture slowly warmed to room temperature. After being concentrated under reduced pressure, the crude reaction mixture was diluted with H₂O (5ml) and the solid formed separated by filtration, washed with EtOAc and dried to afford the product (**I-83**) (0.041g, 70%).

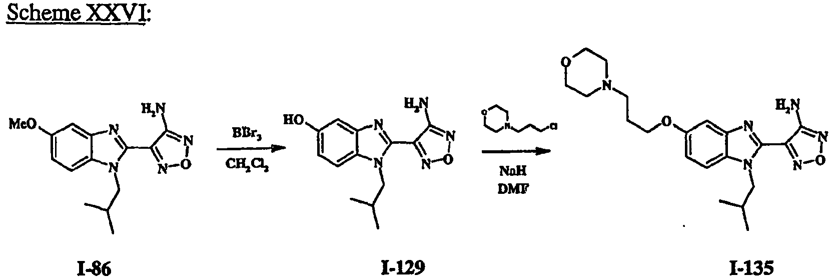

### Preparation of compound (I-129)

To a solution of benzimidazole (**I-86**) (0.212g, 0.738mmol) in dry CH₂Cl₂ (20ml) under N₂ atmosphere a solution of BBr₃ (5mL 1M solution in CH₂Cl₂, 5.0mmol) was added at - 50°C. The mixture was stirred at -50°C for 10minutes and then warmed to room temperature over a period of 1hour. Ice was added and the reaction mixture extracted (CH₂Cl₂), the extract dried (MgSO₄) and the solvent evaporated under reduced pressure. The residue was purified by flash chromatography (SiO₂, EtOAc/petroleum ether, v/v 1:1) to afford the product 1-86 (0.138g, 69%).

**1-Isobutyl-2-(4-amino)-1,2,5-oxadiazol-3-yl-5-(morpholino-N-propoxy) benzimidazole** (**I-135**)**:** To a solution of hydroxybenzimidazole (**I-129**) (0.047g, 0.17mmol) in dry DMF (4mL) under N₂ atmosphere, NaH (0.008g, 60% in mineral oil, 0.2mmol) was added at room temperature and the mixture stirred for 30min. The chloro alkylating agent (0.031g, 0.17mmol) was then added and the mixture heated at 90°C (oil bath temperature) for 16 hours. After being cooled to room temperature, the mixture was diluted with Et₂O (30ml) and washed with H₂O. The Et₂O layer was dried (MgSO₄) and the solvent evaporated under reduced pressure. In order to remove the staring hydroxy compound from the product, the residue was dissolved in CH₂Cl₂ (1ml), TBD-methyl polystyrene (0.01g, 2.39mmol/g) added and the mixture was shaken for 16 hours. The resin was separated by filtration, washed several times with CH₂Cl₂ and the solvent from the combined organic layers evaporated under reduced pressure to afford the product (**I-135**) (0.019g, 27%).

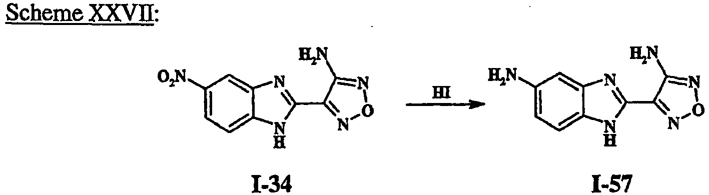

**2-(4-Amino)-1,2,5-oxadiazol-3-yl-5-amino benzimidazole (I-57):** Benzimidazole (**I-34**) (0.046g) was dissolved in HI (3ml, 57% solution in H₂O) and the mixture was heated at 90°C for 4 hours. After being cooled to room temperature, the reaction mixture was diluted with EtOAc (15ml), washed with aqueous Na₂S₂O₃ (10% solution), aqueous NaHCO₃ (saturated solution) and H₂O. The organic phase was then dried (MgSO₄), and the solvent evaporated to afford the product I-57 (0.015g, 38%).

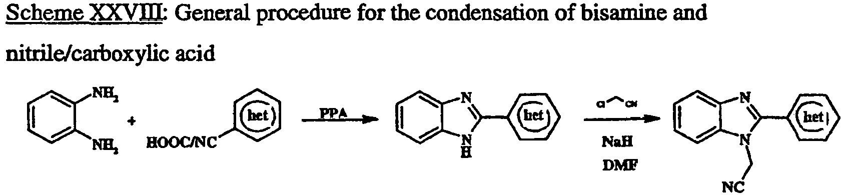

### Preparation of 2-(heteroayl)benzimidazoles

To a mixture of *bis*-amine (6.5mmol) and polyphosphoric acid (~15g) nitrile/ carboxylic acid (6.5mmol) was added and the mixture stirred at 170°C (oil bath temperature) for 6 hours. The reaction mixture was then cooled to ~50°C, dissolved in H₂O and extracted (EtOAc). The extract was dried (MgSO₄), the solvent evaporated under reduced pressure and the residue purified by flash chromatography (SiO₂, ether) to afford the product 2-(heteroaryl)benzimidazoles.

The following compounds have been prepared by this procedure:

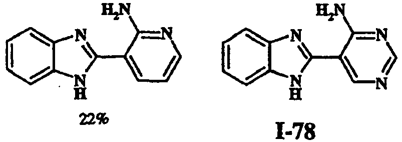

### Preparation of N-alkyl-2-(heteroaryl)benzimidazoles

The alkylation procedure was carried out following the general procedure for the alkylation of benzimidazole derivatives.

The following compounds have been prepared:

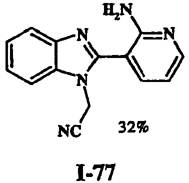

### CHARACTERIZATION DATA

We have prepared other compounds of formula **I** by methods substantially similar to those described in the schemes, general methods, and examples set forth herein, *supra.* The characterization data for these compounds is summarized in Table 3 below and includes HPLC, LC/MS (observed) and ¹H NMR data.

¹H NMR data is summarized in Table 3 below wherein ¹H NMR data was obtained in deuterated DMSO, unless otherwise indicated, and was found to be consistent with structure. Compound numbers correspond to the compound numbers listed in Table 1.

**Table 3. Characterization Data for Selected Compounds of Formula I**

| **Compound No I-** | **M+1 (obs)** | **Rₜ(min)** | **¹H NMR** |
|---|---|---|---|
| 13 | 241 | 1.69 | dH (DMSO): 5.90 (2H, s), 6.89 (2H, s), 7.41 (1H, t),7.53 (1 H, t), 7.90 (1H, d), 7.95 (1H, d). |
| 18 | 202 | 1.46 | 1H NMR dH (DMSO): 6.79 (2H, s, NH2), 7.31 (2H, m, ArH), 7.53 (1 H, d, ArH), 7.77 (1H, d, ArH and 13.65 (1H, s, NH) |
| 25 | 258 | 1.64 | dH (DMSO): 2.38 (3H, s), 5.75 (2H, s), 7.00 (2H, s), 7.41 (2H, m), 7.79 (1 H, d), 7.90 (1 H, d). |
| 26 | - | 1.98 | dH (DMSO): 1.05 13H, t), 3.53 (2H, q), 6.03 (2H, s), 6.95 (2H, s), 7.45 (2H, m), 7.85 (2H,m). |
| 27 | 230 | 1.75 | dH (DMSO): 2.40 (6H, s), 6.88 (2H, s), 7.35-7.70 (2H, br m), 13.50 (1H, s). |
| 28 | 269 | 1.98 | dH (DMSO): 2.40 (3H, s), 2.45 (3H, s), 5.85 (2H, s), 6.89 (2H, s), 7.62 (1 H, s), 7.70 (1H, s). |
| 29 | 309 | 1.99 | dH (DMSO): 5.93 (2H, s), 6.90 (2H, s), 7.7-8.5 (3H, m). |
| 30 | 309 | 2.07 | dH (DMSO): 5.90 (2H, s), 6.90 (2H, s), 8.23 (1H, s), 8.43 (1H, s). |
| 31 | 270 | 1.98 | dH (DMSO): 6.80 (2H, s), 7.95 (2H, s), 14.3 (1H, br). |
| 32 | 256 | 2.14 | 1H NMR dH (DMSO): 0.39 (4H, m, CH2CH2), 1.28 (1H, m, CH), 4.51 (2H, d, NCH2), 6.98 (2H, s, NH2), 7.28 (1 H, t, ArH), 7.37 (1H, t, ArH) and 7.78 .(d, 2H, ArH). |
| 33 | 258 | 2.24 | 1H NMR dH (DMSO): 0.89 (6H, d, 2 x CH3), 2.22 (1 H, m, CH), 4.48 (2H, d, NCH2), 7.01 (2H, s, NH2), 7.29-7.47 (2H, m, ArH) and 7.82 (2H, t, ArH). |
| 34 | 247 | 1.58 | dH (DMSO): 6.85 (2H, s), 7.87 (1H, m), 8.25 (1H, d), 8.60 (1H, s),14.4 (1 H, br). |
| 35 | 306 | 2.09 | dH (DMSO): 6.80 (2H, s), 7.85 (1H, s), 14.4 (1H, br). |
| 36 | 273 | 1.09 | 1H NMR dH (DMSO): 2.2 (6H, s), 2.7 (2H, m), 4.8 (2H, m), 7.0 (2H, s), 7.3-7.9 (4H, m). |
| 37 | 284 | 2.01 | 1H NMR dH (DMSO): 5.52 (2H, m, NCH2), 6.83 (2H, s, NH2), 7.31-7.40 (2H, 2 x t, ArH) and 7.73 (2H, m, ArH). |
| 38 | 258 | 1.67 | 1H NMR dH (DMSO): 2.41 (1H, m), 2.60 (1H, m), 3.28 (1H, m), 4.56 (1 H, dd), 4.98 (1H, m), 6.80 (2H, s, NH2), 7.19-7.29 (2H, 2 x t, ArH) and 7.60-7.69 (2H, 2 x d, ArH). |
| 39 | 318 | 2.15 | dH (DMSO): 0.92 (6H, t), 3.61 (2H, m), 4.80 (3H, m), 6.98 (2H, s), 7.37 (2H, m), 7.78 (2H, m). |
| 40 | - | 1.74 | dH (DMSO): 5.95, 6.00 (2H, 2s, CH2, isomers A+B), 6.93 (2H, s, isomers A+B), 8.0-9.1 (3H, m, isomers A+B). |
| 41 | 341 | 2.21 | dH (DMSO): 5.90 (2H, s), 6.90 (2H, s), 8.5 (1H, s). |
| 42 | 258 | 2.06 | dH (DMSO): 1.35 (9H, s), 6.77 (2H, s), 7.40 (1H, m), 7.65 (2H, br), 13.4 (1H, br). |
| 43 | 260 | 1.54 | dH (DMSO): 3.90 (3H, s), 6.80 (2H, s), 7.80 (1H, br), 7.90 (1H, m), 8.30 (1H, br), 14.1 (1H, br). |
| 44 | 232 | 1.52 | 1H NMR dH (DMSO): 3.73 (3H, s, MeO), 6.72 (2H, s, NH2), 6.87 (1H, d, ArH), 7.02 (1H, bs, ArH) and 7.51 (1 H, bd, ArH). The N-hydrogen signal was not observed. |
| 45 | 297 | 2.11 | dH (DMSO): 1.4 (9H, 2s, mixture of isomers), 5.9 (2H, 2s, mixture of isomers), 7.0 (2H, br s), 7.5-8.0 (3H, m). |
| 46 | 299 | 1.66 | dh (DMSO): 3.90 (3H, 2s, mixture of Isomers), 5.95, 6.05 (2H, 2s, nixture of isomers), 6.95 (2H, s), 7.95-8.65 (3H, m). |
| 47 | 271 | 1.65 | 1H NMR dH (DMSO): 3.84 (2 x 3H, 2 x s, MeO, isomers A+B), 5.88 (2 x 2H, 2 x s, NCCH2, isomers A+B), 6.90 (2 x 2H, 2 x s, NH2, isomers A+B), 7.04 (1H, m, ArH, isomer A), 7.16 (1H, m, ArH, isomer B), 7.39 (1H, d, ArH, Isomer B), 7.58 (1H, d, ArH, isomer... |
| 48 | 247 | 1.52 | 1H NMR dH (DMSO): 6.70 (2H,s, NH2), 7.41 (1H, m, ArH), 8.09 (2H, m, ArH) and 14.20 (1H, bs, NH). |
| 49 | 216 | 1.69 | dH (DMSO): 2.63 (3H, s), 6.88 (2H, s, NCCH2), 7.13 (1H, m), 7.33 (1H, t), 7.47 (1H, br) and 13.6 (1H, br). |
| 50 | 278 | 2.14 | 1H NMR dH (DMSO): 6.79 (2H, s, NH2), 7.08 (1H, m, ArH), 728 (2H, m ArH), 7.41-7.50 (5H, m, ArH) and 7.80 (1H, m, ArH). |
| 51 | 255 | 1.28 | 1H NMR dH (DMSO): 2.04 (3H, d, CH3), 6.78 (1H, q, NCCH), 6.98 (2H, s, NH2), 7.50 (1H, t, ArH), 7.59 (1 H, t, ArH) and 7.98 (2H, m, ArH). |
| 52 | 218 | 1.03 | 1H NMR dH (DMSO): 6.88 (1H, m, ArH), 7.18-7.41 (4H, t+bs, 2 x ArH+NH2), 11.99 (1H, bs, OH) and 13.78 (1H, bs, NH). |
| 53 | 256 | 1.82 | dH (DMSO): 2.65 (3H, s), 5.90 (2H, s), 6.95 (2H, s), 7.25 (1 H, m), 7.40 (1H, t), 7.70 (1H, m). |
| 54 | 272 | 2.41 | 1H NMR dH (DMSO): 0.98 (6H, d, 2 x CH3), 1.69 (3H, m, CHCH2), 4.70 (2H, dd, NCH2), 6.99 (2H, s, NH2), 7.34 (1H, t, ArH), 7.42 (1H, t, ArH), 7.73 (1H, d, ArH) and 7.80 (1H, d, ArH). |
| 56 | - | 1.63 | 1H NMR dH (DMSO): 5.88 (2H, s, NCCH2), 6.92 (2H, s, NH2), 7.63 (1H, t, ArH), 8.18 (1H, d, ArH) and 8.32 (1H, d, ArH). |
| 57 | 217 | 0.95 | dH (DMSO): 5.34 (2H, s), 6.67 (2H, br), 6.80 (2H, br), 7.45 (1H, d), 13.0 (1H, br). |
| 58 | 296 | 1.56 | 1H NMR dH (DMSO): 5.36 (2 x 2H, 2 x s, NCH2, isomers A+B), 5.79 (2 x 2H, 2 x s, OCH2, isomers A+B), 6.88 (2 x 2H, 2 x s, NH2, isomers A+B), 6.99 (1H, d, ArH, isomer A), 7.12 (1H, d, ArH, isomer B), 7.29 (1 H, d, ArH, isomer B), 7.37 (1H, t, ArH, isomer... |
| 59 | 262 | 1.22 | 1H NMR dH (DMSO): 3.85 (6H, s), 6.79 (2H, s), 7.18 (2H, br s). 13.4 (1H, br). |
| 60 | 246 | 1.47 | 1H NMR dH (DMSO): 6.08 (2H, s), 6.79 (2H, s), 7.05 (1H, br s), 7.28 (1H, br), 13.6 (1H, br). |
| 67 | 203 | 0.81 | dH (DMSO): 6.88 (2H, s), 7.38 (1H, m), 8.15 (1H, m), 8.52 (1H, s), 14.2 (1H, br s). |
| 68 | 242 | 1.38 | dH (DMSO): 5.80 (2H, s), 6.95 (2H, s), 7.60 (1H, m), 8.40 (1 H, m), 8.65 (1H, s). |
| 69 | 204 | 0.54 | 1H NMR dH (DMSO): 6.82 (2H, s), 9.08 (1H, s), 9.28 (1H, s) and 14.7 (1H, br s). |
| 77 | 250 | 1.16 | dH (DMSO): 5.48 (2H, s), 6.55 (2H, s), 6.75 (1H, m), 7.33 (2H, m), 7.70 (3H, m) and 8.12 (1H, d). |
| 80 | 212 | 1.51 | 1H NMR dH (DMSO): 7.13 (2H, m, ArH), 7.44 (1H, d, ArH), 7.63 (1H, d, ArH), 7.88 (1H, s, pyrazine-H), 8.06 (1H, s, pyrazine-H) and 12.99 (1H, s, NH). The NH2 signal was not observed. |
| 81 | 251 | 1.63 | 1 H NMR dH (DMSO): 6.28 (2H, s, NCCH2), 7.59-7.70 (2 x 1H, 2 x t, ArH) 8.07 (2H, m, ArH), 8.26 (1H, d, pyrazine-hydrogen) and 8.41 (1H, d, pyrazine-hydrogen). The NH2 signal was not observed. |
| 82 | 249 | 1.45 | 1H NMR dH (DMSO): 5.43 (2H, s), 5.75 (2H, s), 6.83 (1H, m), 6.90 (1H, m), 7.2-7.4 (4H, m), 7.75 (2H, m). |
| 83 | 294 | 1.36 | dH (DMSO): 6.0 (2H, s), 7.45 (3H, m), 7.90 (2H, m), 8.45 (3H, m), 11.6 (1H, br). |
| 84 | 211 | 1.37 | dH (DMSO): 7.35 (2H, br), 7.4 (2H, s), 7.75 (2H, br), 7.85 (1H, m), 7.95 (1H, m), 8.35 (1H, s), 13.2 (1H, br s). |
| 85 | 243 | 0.97 | 1H NMR dH (DMSO): 5.78 (2H, s), 6.90 (2H, s), 9.18 (1 H, s), 9.40 (1H, s). |
| 86 | 288 | 2.19 | 1H NMR dH (DMSO): 0.91 (2 x 6H, m, CH3CHCH3, isomers A+B), 2.22 (2 x 1H, m, CHCH2, isomers A+B), 3.86 (2 x 3H, 2 x s, OMe, isomers A+B), 4.49 (2 x 2H, m, CH2, isomers A+B), 6.96-7.09 (2 x 3H, m, NH2 + ArH, isomers A+B), 7.31 (2 x 1H, m, ArH, isomers A... |
| 87 | 211 | 1.64 | dH (DMSO): 7.2-7.4 (6H, m), 7.6 (1H, m), 7.7 (1H, m), 8.0 (1H, m), 12.9 (1H, s). |
| 88 | 301 | 1.48 | 1H NMR dH (DMSO): 3.9 (3H, s), 4.0 (3H, s), 5.9 (2H, s), 6.9 (2H, s), 7.4 (1H, s), 7.6 (1H, s). |
| 90 | 265 | 1.9 | 1H NMR dH (CDCl3): 3.20 (3H, s), 6.0 (2H, s), 7.45 (3H, m) 7.85 (2H, m), 8.20 (1H, s) and 9.45 (1 H, br s). |
| 91 | 214 | 1.05 | dH (DMSO): 3.60 (3H, s), 6.45 (2H, s), 7.1 (2H, m), 7.4 (2H, br m), 7.75 (1H, s), 12.3 (1H, s). |
| 92 | 250 | 1.28 | dH (DMSO): 4.1 (2H, s), 6.35 (2H, m), 7.15 (1H, m), 725 (1H, m), 7.5 (1H, d), 7.75 (1H, d), 8.0 (1H, s), 8.1 (1H, s), 11.6 (1H, s). |
| 93 | 211 | 1.49 | dH (DMSO): 6.45 (2H, s), 7.0 (1H. m), 7.15 (2H, m), 7.5 (1 H, d), 7.95 (2H, 2s), 11.5 (1H, s). |
| 94 | 270 | 1.88 | dH (DMSO): 6.85 (2H, s), 7.6-82 (3H, m), 142 (1H, s). |
| 95 | 374 | 2.72 | (DMSO): 0.9 (6H, m), 2.3 (1H, m), 3.9 (3H, s), 4.9 (2H, d), 6.9 (1H, m), 7.2-7.5 (6H, m), 7.8 (1H, m), 8.5 (1H, s). |
| 96 | 302 | 2.65 | dH (DMSO): 0.90 (6H, m), 2.2 (1H, m), 4.6 (2H, d), 7.3 (2H, m), 7.8 (2H, m), 8.2 (1H, s). |
| 97 | 373 | 3.02 | dH (DMSO): 0.95 (12H, m), 1.90 (1H, m), 2.3 (1H, m), 3.3 (2H, d), 4.5 (2H, d), 5.25 (1H, t), 7.2 (2H, m), 7.3 (1 H, m), 7.7 (1 H, d). |
| 98 | 375 | 2.69 | dH (DMSO): 0.95 (6H, m), 2.3 (1H, m), 3.4 (3H, s), 3.6 (2H, m), 3.7 (2H, m), 4.5 (2H, d), 5.5 (1 H, br t), 7.2 (2H, m), 7.3 (1H, m), 7.7 (1H, d). |
| 99 | 408 | 2.96 | dH (DMSO): 1.0 (6H, m), 2.4 (1H, m), 3.9 (3H, s), 4.6 (2H, d), 7.0 (1H, m), 7.3-7.5 (6H, m), 7.8 (1H, m). |
| 100 | 336 | 2.87 | dH (DMSO): 0.99 (6H, m), 2.3 (1H, m), 4.6 (2H, d), 7.2-7.5 (3H, m), 7.8 (1H, m), 8.2 (1H, s). |
| 101 | 346 | 2.21 | dH (DMSO): 0.90 (6H, m), 1.50 (3H, s), 4.7 (2H, d), 7.3-7.5 (3H, m), 7.85 (1H, m), 8.25 (2H, d), 13.7 (1H, br). |
| 102 | 308 | 1.98 | 1H NMR dH (CDCl3): 3.68 (3H, s), 5.88 (2H, s), 7.10-7.60 (7H, m), 7.90 (1H, m). |
| 103 | 308 | 3.15 | 1H NMR dH (CDCl3): 3.92 (3H, s), 5.88 (2H, s), 7.02-7.45 (7H, m), 7.88 (1H, m). |
| 104 | 312 | 3.31 | 1H NMR dH (CDCl3): 5.83 (2H, s), 7.20 (1H, d), 7.38 (4H, m), 7.67 (2H, m), 7.88 (1H, m). |
| 105 | 308 | 2.11 | 1H NMR dH (CDCl3): 3.86 (3H, s), 5.87 (2H, s), 6.90-7.55 (7H, m), 7.88 (1H, m). |
| 106 | 345 | 2.93 | dH (DMSO): 1.0 (6H, m), 2.3 (1H, m), 3.2 (6H, s), 4.6 (2H, d), 7.2 (2H, m), 7.4 (1H, m), 7.7 (1H, d). |
| 108 | 303 | 0.93 | dH (DMSO): 3.15 (2H, m), 3.5 (2H, m), 4.75 (1H, br), 5.4 (2H, s), 7.0 (2H, s), 7.4 (2H, m), 7.7 (1H, d), 7.8 (1H, d) 8.4 (1H, br). |
| 109 | 273 | 1.31 | dH (DMSO): 1.7 (3H, d), 6.0 (1H, q), 7.0 (2H, s), 7.3 (3H, m), 7.6 (2H, m) and 7.8 (1H, m). |
| 110 | 272 | 1.84 | dH (CDCl3): 1.78 (3H, d), 2.11 (3H, s), 5.96 (2H, br s), 6.35 (1H, m), 7.20-7.45 (3H, m) and 7.85 (1H, m). |
| 111 | 303 | 1.97 | dH (CDCl3): 5.80 (2H, broad s), 7.10 (1 H, m), 7.42 (2H, m), 7.55 (1H, m), 7.75 (1H, m) 7.88 (1H, m) and 7.97 (2H, m). |
| 112 | 414 | 2.09 | dH (CDCl3): 3.69 (6H, s), 6.6-6.75 (6H, m), 7.1-7.4 (5H, m, ArH) and 7.65 (1H, d), |
| 113 | 272 | 1.8 | dH (CDCl3): 1.17 (3H, t), 2.63 (2H, q), 5.50 (2H, s), 5.88 (2H, br s), 7.2-7.4 (3H, m) and 7.87 (1H, m). |
| 114 | 275 | 1.05 | dH (DMSO): 2.6 (2H, m), 3.8 (1H, br), 4.6 (2H, m), 4.9 (1H, br), 6.9 (2H, s), 7.3 (2H, m), 7.8 (2H, m). |
| 115 | 289 | 1.36 | dH (DMSO): 2.3 (3H, s), 2.6 (2H, m), 4.0 (1H, br), 4.7 (2H, m), 5.0 (1H, br), 7.0 (2H, s), 7.4 (2H, m), 7.8 (2H, m). |
| 116 | 302 | 1.09 | dH (DMSO): 2.6 (2H, m), 3.1 (2H, m), 5.4 (2H, s), 7.0 (2H, s), 7.4 (2H, m), 7.7 (1H, d), 7.8 (1 H, d) 8.3 (1 H, br). |
| 117 | 303 | 1.95 | dH (CDCl3): 5.85 (2H, broad s), 7.18 (2H, m), 7.40 (2H, m), 7.55 (2H, m) and 7.88 (2H, m). |
| 118 | 273 | 1.33 | dH (DMSO): 1.3 (2H, m), 1.7 (2H, m), 2.5 (m, obscured), 4.6 (2H, m), 7.0 (2H, s), 7.3 (2H, m) and 7.8 (2H, m). |
| 119 | 252 | 2.02 | dH (CDCl3): 5.82 (2H, br s), 7.48 (2H, m), 7.86 (2H, m), 8.47 (1H, t). |
| 120 | 246 | 1.28 | dH (DMSO): 3.80 (1H, m), 4.74 (1H, m), 4.94 (1H, t), 5.77 (2H, s), 6.98 (2H, s), 7.39 (2H, m), 7.80 (2H, dd). |
| 121 | 273 | 1.16 | dH (DMSO): 2.36 (3H, s), 5.20 (2H, s), 6.88 (2H, s, NH2), 7.28 (2H, m), 7.56 (1H, d), 7.73 (1 H, d), 8.11 (1 H, m). |
| 122 | 287 | 1.4 | dH (DMSO): 2.85 (3H, s), 3.18 (3H, s), 5.12 (2H, s), 6.98 (2H, s), 7.38 (2H, m), 7.75 (1H, d), 7.82 (1H, d). |
| 123 | 316 | 1.17 | dH (DMSO): 3.6 (2H, m), 5.4 (2H, s), 7.0 (2H, s), 7.2 (1H, s), 7.5 (3H, m), 7.7 (1H, d), 7.8 (1H, d), 8.6 (1H, t). |
| 124 | 245 | 1.34 | 1H NMR dH (DMSO): 3.2 (2H, m), 4.8 (2H, br), 7.0 (4 H, s and br), 5.92 (2H, br), 7.4 (2H, m), 7.9 (2H, m). |
| 125 | 258 | 0.88 | 1 H NMR dH (DMSO): 5.65 (2H, s), 7.0 (2H, s), 7.45 (2H, m), 7.9 (2H, 2d), 9.1 (3H, br). |
| 126 | 259 | 1.56 | dH (CDCl3): 1.70 (3H, d), 3.2 (1H, m), 3.5 (1H, m), 5.75 (1H, br), 5.95 (2H, br), 7.4 (2H, m), 7.7 (1H, m), 7.85 (1H, m). |
| 127 | 259 | 1.55 | 1H NMR dH (CDCl3): 2.48 (3H, s), 3.08 (2H, m), 4.82 (2H, t), 5.92 (2H, br), 7.37 (2H, m), 7.55 (1H, d), 7.82 (1H, d). |
| 128 | 308 | 2.37 | dH (DMSO): 3.90 (3H, s), 5.70 (1H, br), 6.65 (2H, d), 7.15-7.95 (6H, m), 9.47 (1H, br s) and 10.03 (1H, br s). |
| 129 | 274 | 1.79 | 1H NMR dH (DMSO): 0.89 (6H, d, 2 x CH3), 2.20 (1H, m, CH), 4.45 (2H, d, NCH2), 6.8-7.2 (4H, m), 7.63 (1H, m, ArH) 9.40 and 9.72 (1H, 2s isomers (2:1 ratio)). |
| 130 | 279 | 1.35 | 1H NMR dH (CDCl3): 2.95(6H, s), 5.18 (2H, s), 5.60 (2H, s), 7.35-7.50 (3H, m) 7.87 (1H, d), 8.06 (1H, s) and 8.20 (1H, s). |
| 131 | 253 | 0.62 | 1 H NMR dH (CDCl3): 3.78 (3H, s), 5.50 (2H, s), 7.35 (2H, m) 7.75 (2H, m). |
| 132 | 213 | 1.41 | 1H NMR dH (DMSO): 7.3 (2H, d), 7.7 (2H, d), 8.1 (2H, m), 13.4 (1H, br). |
| 133 | 280 | 2.19 | dH (DMSO): 7.2 (2H, br s), 7.5 (1H, br s), 7.8 (1H, br s), 8.1 (1H, br), 8.9 (1H, br), 13.1 (1H, br s). |
| 134 | 381 | 2.33 | dH (DMSO): 3.7 (3H, s), 4.5 (2H, d), 6.9 (2H, m), 7.1 (2H, m), 7.3 (2H, m), 7.45 (1H, m), 7.6 (1 H, d), 7.8 (1H, t), 12.4 (1H, s). |
| 136 | 392 | 2.78 | 1H NMR dH (CDCl3): 0.98 (6H, m), 2.38 (1H, m), 3.79 (3H, s), 4.28 (2H, d), 4.57 (2H, d), 6.75 (1H, d), 6.88 (2H, m), 7.15-7.35 (6H, m), 7.67 (1 H, m) 8.96 (1H, br t). |
| 137 | 368 | 2.73 | 1H NMR dH (CDCl3): 1.03 (6H, d), 2.35 (1H, m), 3.80 (2H, m), 4.32 (2H, d), 7.32 (2H, m), 7.40 (1H, m), 7.50 (1H, d), 7.75 (1H, d), 8.30 (1H, d), 14.40 (1H, br s). |
| 138 | 272 | 1.15 | 1H NMR dH (CDCl3): 1.05 (6H, m), 2.35 (1H, m), 4.2 (2H, d), 6.1 (2H, br), 6.7 (1H, s), 7.2-7.4 (4H, m), 7.8 (1H, m). |

### BIOLOGICAL ASSAYS

### Determination for the Inhibition of GSK-3

Compounds were screened for their ability to inhibit GSK-3β (AA 1-420) activity using a standard coupled enzyme system (Fox et al. (1998) *Protein Sci.* 7, 2249). Reactions were carried out in a solution containing 100 mM HEPES (pH 7.5), 10 mM MgCl₂, 25 mM NaCl, 300 µM NADH, 1 mM DTT and 1.5% DMSO. Final substrate concentrations in the assay were 20 µM ATP (Sigma Chemicals, St Louis, MO) and 300 µM peptide (HSSPHQS(PO₃H₂)EDEEE, American Peptide, Sunnyvale, CA). Reactions were carried out at 30°C and 20 nM GSK-3□. Final concentrations of the components of the coupled enzyme system were 2.5 mM phosphoenolpyruvate, 300 µM NADH, 30 µg/ml pyruvate kinase and 10 µg/ml lactate dehydrogenase.

An assay stock buffer solution was prepared containing all of the reagents listed above with the exception of ATP and the test Compound of interest. The assay stock buffer solution (175 µl) was incubated in a 96 well plate with 5 µl of the test compound of interest at final concentrations spanning 0.002 µM to 30 µM at 30°C for 10 minutes. Typically, a 12 point titration was conducted by preparing serial dilutions (from 10 mM compound stocks) with DMSO of the test compounds in daughter plates. The reaction was initiated by the addition of 20 µl of ATP (final concentration 20 µM). Rates of reaction were obtained using a Molecular Devices Spectramax plate reader (Sunnyvale, CA) over 10 minutes at 30 °C. The Kᵢ values were determined from the rate data as a function of inhibitor concentration.

The following compounds were shown to have a Kᵢ of less than 1 µM for GSK-3 (Compound numbers correspond to the compound numbers listed in Table 1.): **I**-2, **I**-7, **I**-9, **I**-11, **I**-12, **I**-13, **I**-19, **I**-32, **I**-33, **I**-37, **I**-38, **I**-47, **I**-50, **I**-51, **I**-54, **I**-55, **I**-56, **I**-58, **I**-81, **I**-86, **I**-87, **I**-90, **I**-96, **I**-109, **I**-128, and **I**-129.

The compounds were evaluated as inhibitors of human Lck kinase using either a radioactivity-based assay or spectrophotometric assay.

### Lck Inhibition Assay A: Radioactivity-based Assay

The compounds were assayed as inhibitors of full-length bovine thymus Lck kinase (from Upstate Biotechnology, cat. no. 14-106) expressed and purified from baculo viral cells. Lck kinase activity was monitored by following the incorporation of ³³P from ATP into the tyrosine of a random poly Glu-Tyr polymer substrate of composition, Glu:Tyr = 4:1 (Sigma, cat. no. P-0275). The following were the final concentrations of the assay components: 0.025 M HEPES, pH 7.6, 10 mM MgCl₂, 2 mM DTT, 0.25 mg/ml BSA, 10 µM ATP (1-2 µCi ³³P-ATP per reaction), 5 mg/ml poly Glu-Tyr, and 1-2 units of recombinant human Lck kinase. In a typical assay, all the reaction components with the exception of ATP were pre-mixed and aliquoted into assay plate wells. Inhibitors dissolved in DMSO were added to the wells to give a final DMSO concentration of 2.5%. The assay plate was incubated at 30 C for 10 min before initiating the reaction with ³³P-ATP. After 20 min of reaction, the reactions were quenched with 150 µl of 10% trichloroacetic acid (TCA) containing 20 mM Na₃PO₄. The quenched samples were then transferred to a 96-well filter plate (Whatman, UNI-Filter GF/F Glass Fiber Filter, cat no. 7700-3310) installed on a filter plate vacuum manifold. Filter plates were washed four times with 10% TCA containing 20 mM Na₃PO₄ and then 4 times with methanol. 200µl of scintillation fluid was then added to each well. The plates were sealed and the amount of radioactivity associated with the filters was quantified on a TopCount scintillation counter. The radioactivity incorporated was plotted as a function of the inhibitor concentration. The data was fitted to a competitive inhibition kinetics model to get the Kᵢ for the compound.

### Lck Inhibition Assay B: Spectrophotometric Assay

The ADP produced from ATP by the human recombinant Lck kinase-catalyzed phosphorylation of poly Glu-Tyr substrate was quanitified using a coupled enzyme assay (Fox et al (1998) Protein Sci 7, 2249). In this assay one molecule of NADH is oxidised to NAD for every molecule of ADP produced in the kinase reaction. The disappearance of NADH can be conveniently followed at 340 nm.

In a typical assay, all the reaction components with the exception of ATP were pre-mixed and aliquoted into assay plate wells. Inhibitors dissolved in DMSO were added to the wells to give a final DMSO concentration of 2.5%. The assay plate was incubated at 30 °C for 10 min before initiating the reaction with 150 µM ATP. The absorbance change at 340 nm with time, the rate of the reaction, was monitored on a molecular devices plate reader. The data of rate as a function of the inhibitor concentration was fitted to competitive inhibition kinetics model to get the Kᵢ for the compound.

The following compounds were shown to have a Kᵢ of less than 5 µM for Lck (Compound numbers correspond to the compound numbers listed in Table 1.): **I**-56, **I**-57, **I**-58, **I**-59, **I**-70, **I**-71, **I**-80, **I**-81, **I**-86, **I**-87, **I**-89, **I**-90, **I**-96, **I**-104, **I**-109, **I**-110, **I**-117, **I**-128, and **I**-129.

## Claims

1. A compound of formula **Ia** or **Ib**: 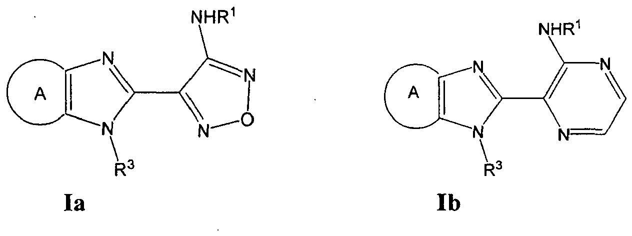 or a pharmaceutically acceptable salt thereof, wherein:
Ring A is a 5-7 membered, partially unsaturated or fully unsaturated ring having 0-3 heteroatoms independently selected from nitrogen, oxygen or sulfur, and wherein Ring A is optionally fused to an saturated, partially unsaturated or fully unsaturated 5-8 membered ring having 0-3 heteroatoms independently selected from nitrogen, oxygen or sulfur, wherein said Ring A and the ring optionally fused to Ring A are optionally substituted on the unsaturated carbon atom of an aryl, heteroaryl, aralkyl, or heteroaralkyl group with halogen, oxo, N₃, -R°, -OR°, SR°, 1,2-methylene-dioxy, 1,2-ethylenedioxy, phenyl (Ph), Ph substituted with R°,-O(Ph), O-(Ph) substituted with R°, -CH₂(Ph), -CH₂(Ph) substituted with R°, -CH₂CH₂ (Ph), -CH₂CH₂ (Ph) substituted with R°, NO₂, -CN, -N(R°)₂, -NR°C(O)R°,-NR°C(O)N(R°)₂, -NR°CO₂R°, -NR°NR°C(O)R°, -NR°NR°C(O)N(R°)₂,-NR°NR°CO₂R°, -C(O)C(O)R°, -C(O)CH₂C(O)R°, -CO₂R°, -C(O)R°, -C(O)N(R°)₂,-OC(O)N(R°)₂, -S(O)₂R°, -SO₂N(R°)₂, -S(O)R°, -NR°SO₂N(R°)₂, -NR°SO₂R°,-C(=S)N(R°)₂, -C(=NH)-N(R°)₂, or -(CH₂)_{Y}NHC(O)R°, wherein y is 0-4, each R° is independently selected from hydrogen, optionally substituted C₁₋₆ aliphatic, an unsubstituted 5-6 membered heteroaryl or heterocyclic ring having 0-4 heteroatoms independently selected from nitrogen, oxygen or sulfur, phenyl (Ph), -O(Ph), or-CH₂(Ph)-CH₂(Ph), and wherein each optional substituent on the aliphatic group of R° is independently selected from NH₂, NH(C₁₋₄ aliphatic), N(C₁₋₄ aliphatic)₂, halogen, C₁₋₄ aliphatic, OH, O-(C₁₋₄ aliphatic), NO₂, CN, CO₂H, CO₂(C₁₋₄ aliphatic), -O(haloC₁₄ aliphatic), or halo C₁₋₄ aliphatic; and wherein said Ring A and the ring optionally fused to Ring A are optionally substituted on the saturated carbon atom of an aliphatic group or of a non-aromatic heterocyclic ring with the substituents being selected from those listed above for the unsaturated carbon of an aryl or heteroaryl group and =O, =S, =NNHR*, NN(R*)₂, =N-, =NNHC(O)R*, =NNHCO₂ (alkyl), =NNHSO₂ (alkyl), or =NR*, where each R* is independently selected from hydrogen or an optionally substituted C1-6 aliphatic with the substituents on the aliphatic group of R* are selected from NH₂, NH(C₁₋₄ aliphatic), N(C₁₋₄ aliphatic)₂, halogen, C₁₋₄ aliphatic, OH, O-(C₁₋₄ aliphatic), NO₂, CN, CO₂H, CO₂ (C₁₋₄ aliphatic)-O(haloC₁₋₄ aliphatic, or haloC₁₋₄ aliphatic;
R¹ is selected from R or R²;
R² is selected from -SO₂R, -SO₂N(R)₂, -CN, -C(O)R, -CO₂R, or -CON(R)₂;
R is independently selected from hydrogen or an optionally substituted group selected from C₁₋₆ aliphatic, a 3-6 membered saturated, partially unsaturated, or aryl ring having 0-4 heteroatoms independently selected from nitrogen, oxygen, or sulfur, or:
two R groups on the same nitrogen are taken together with the nitrogen bound thereto to form a 3-7 membered heterocyclic or heteroaryl ring having 1-4 heteroatoms independently selected from nitrogen, oxygen, or sulfur, wherein the optional substituent is selected as listed above;
R³ is selected from T-CN or L-R;
T is a valence bond or an optionally substituted C₁₋₆ alkylidene chain with the optional substituent being selected as listed before for the substituents on the saturated carbon of an aliphatic group, ; and
L is a valence bond or a C₁₋₄ alkylidene chain, wherein up to two methylene units of L are optionally, and independently, replaced by, -S-, -NR-, -NRC(O)-, -NRC(O)NR-, -OC(O)NR-, , -CO₂-, -NRCO₂-, -C(O)NR-, -SO₂NR-, -NRSO₂-, or -NRSO₂NR-;
provided that said compound is other than one of the group consisting of:
4-[1-(4-Chloro-benzyl)-1H-benzoimidazol-2-yl]-furazan-3-ylamine (**I**-1);
4-(1-Prop-2-ynyl-1H-benzoimidazol-2-yl)-furazan-3-ylamine (**I**-2);
4-(5-Methyl-1H-benzoimidazol-2-yl)-furazan-3-ylamine (**I**-3);
4-[1-(2-Chloro-6-fluoro-benzyl)-1H-benzoimidazol-2-yl]-furazan-3-ylamine (**I**-4);
[2-(4-Amino-furazan-3-yl)-benzoimidazol-1-yl]-acetic acid (**I**-5);
2-[2-(4-Amino-furazan-3-yl)-benzoimidazol-1-yl]-acetamide (**I**-6);
4-(1-Propyl-1H-benzoimidazol-2-yl)-furazan-3-ylamine (**I**-7);
4-[1-(2,6-Dichloro-benzyl)-1H-benzoimidazol-2-yl]-furazan-3-ylamine (**I**-8);
4-(1-Allyl-1H-benzoimidazol-2-yl)-furazan-3-ylamine (**I**-9);
4-[1-(4-Methyl-benzyl)-1H-benzoimidazol-2-yl]-furazan-3-ylamine (**I**-10);
4-(1-Isopropyl-1H-benzoimidazol-2-yl)-furazan-3-ylamine (**I**-11);
4-[1-(2-Methyl-benzyl)-1H-benzoimidazol-2-yl]-furazan-3-ylamine (**I**-12);
[2-(4-Amino-furazan-3-yl)-benzoimidazol-1-yl]-acetonitrile (**I**-13);
4-[1-(1H-Tetrazol-5-ylmethyl)-1H-benzoimidazol-2-yl]-furazan-3-ylamine (**I**-14);
4-[1-(2,4-Dichlorobenzyl)-1H-benzoimidazol-2-yl]-furazan-3-ylamine (**I**-15);
4-[1-(3,4-Dichloro-benzyl)-1H-benzoimidazol-2-yl]-furazan-3-ylamine (**I**-16);
2-[2-(4-Amino-furazan-3-yl)-benzoimidazol-1-yl]-N-(3,4-dimethoxy-phenyl)-acetamide (**I**-17);
4-(1H-Benzoimidazol-2-yl)-furazan-3-ylamine (**I**-18);
2-[2-(4-Amino-furazan-3-yl)-benzoimidazol-1-yl]-N-(3,4-difluoro-phenyl)-acetamide (**I-**19);
2-[2-(4-Amino-furazan-3-yl)-benzoimidazol-1-ylmethyl]-benzonitrile (**I**-20);
2-[2-(4-Amino-furazan-3-yl)-benzoimidazol-1-yl]-N-(2-trifluoromethyl-phenyl)-acetamide (**I**-21);
4-[1-(3-Bromo-benzyl)-1H-benzoimidazol-2-yl]-furazan-3-ylamine (**I**-22);
4-[2-(4-Amino-furazan-3-yl)-benzoimidazol-1-yl]-butyronitrile (**I**-23);
4-(1-Ethyl-1H-benzoimidazol-2-yl)-furazan-3-ylamine (**I**-55);
2-[2-(4-Amino-furazan-3-yl)-benzoimidazol-1-yl]-N-(2-fluoro-phenyl)-acetamide (**I**-61);
4-(1-Methyl-1H-benzoimidazol-2-yl)-furazan-3-ylamine (**I**-62);
2-[2-(4-Amino-furazan-3-yl)-benzoimidazol-1-yl]-N-biphenyl-2-yl-acetamide (**I**-63);
2-[2-(4-Amino-furazan-3-yl)-benzoimidazol-1-yl]-N-(2,6-dimethyl-phenyl)-acetamide (**I-**64);
2-[2-(4-Amino-furazan-3-yl)-benzoimidazol-1-yl]-N-tert-butyl-acetamide (**I**-65);
2-[2-(4-Amino-furazan-3-yl)-benzoimidazol-1-yl]-N-(3-fluoro-phenyl)-acetamide (**I**-66);
2-[2-(4-Amino-furazan-3-yl)-benzoimidazol-1-yl]-N-(2-fluoro-phenyl)-acetamide (**I**-70);
N-[4-(1-Ethyl-1H-benzoimidazol-2-yl)-furazan-3-yl]-2,2,2-trifluoro-acetamide (**I**-72);
N-[4-(1-Cyanomethyl-1H-benzoimidazol-2-yl)-furazan-3-yl]-acetamide (**I**-76);
2-(4-Amino-1,2,5-oxadiazol-3-yl)-1H-benzimidazole-1-acetonitrile;
2-(4-Amino-1,2,5-oxadiazol-3-yl)-N-(2,4-difluorophenyl)-1H-benzimidazole-1-acetamide;
4-[1-(Cyclopropylmethyl)-1H-benzimidazol-2-yl-1,2,5-oxadiazol-3-amine;
N-[4-[1-(Cyclopropylmethyl)-1H-benzimidazol-2-yl]-1,2,5-oxadiazol-3-yl]-acetamide;
4-[1-(2-Methylpropyl)-1H-benzimidazol-2-yl]-1,2,5-oxadiazol-3-amine;
4-[1-(Phenylmethyl)-1H-benzimidazol-2-yl-1,2,5-oxadiazol-3-amine;
2-(4-Amino-1,2,5-oxadiazol-3-yl)-N-[4-(trifluoromethoxy)phenyl]-1H-benzimidazole-1-acetamide;
2-(4-Amino-1,2,5-oxadiazol-3-yl)-1H-benzimidazole-1-acetic acid ethyl ester;
N-[4-[1-[(5-Methyl-1,3,4-oxadiazol-2yl)-methyl]-1H-benzimidazol-2-yl]-1,2,5-oxadiazol-3yl-acetamide;
4-(1-Methyl-5-nitro-1H-benzimidazol-2-yl)-1,2,5-oxadiazol-3-amine[,] ;
3-Amino-4-(1H-benzimidazol-2-yl)-1,2,5-oxadiazole;
3-Amino-4-(1-ethyl-1H-benzimidazol-2-yl)-1,2,5-oxadiazole;
4-(1H-benzo[d]imidazol-2-yl)-1,2,5-oxadiazol-3-amine; and
Methyl-2-[2-(4-amino-1,2,5-oxadiazol-3yl)-(1H-benzimidazol-1-yl)]-acetate.

2. The compound according to claim 1, wherein:
R¹ is selected from R, -SO₂R, or -C(O)R;
R³ is selected from T-CN or L-R;
L is a valence bond or a C₁₋₄ alkylidene chain wherein a methylene unit of L is optionally replaced by -CO₂-, -C(O)NR-, -C(O)-, -N(R)-, or -O-;
R is hydrogen, or an optionally substituted group selected from C₁₋₄ aliphatic, 3-6 membered heterocyclyl having 1-2 heteroatoms independently selected from nitrogen, oxygen, or sulfur, phenyl, or a 5-6 membered heteroaryl ring having 1-4 heteroatoms independently selected from nitrogen, oxygen, or sulfur, with the optional substituent as defined in claim 1; and
T is a C₁₋₄ alkylidene chain.

3. The compound according to claim 2, wherein:
R¹ is selected from R, -SO₂R, or -C(O)R;
R is hydrogen or an optionally substituted C₁₋₄ aliphatic group;
R³ is selected from hydrogen, -CH₂CN, -CH₂C(O)NH₂, -CH₂CO₂H, propyl, -CH₂CH₂=CH₂, isopropyl, -(CH₂)₃CN, -CH₂OEt, -CH₂CF₃, isobutyl, cyclopropylmethyl, -CH₂CH₂N(Me)₂,-CH₂CH(OEt)₂, ethyl, -CH₂C(O)NH*t*-butyl, or an optionally substituted benzyl or-CH₂C(O)NHphenyl group; and
any substitutable carbon on the benzo ring is independently and optionally substituted with chloro, bromo, methyl, -CF₃, nitro, *t*-butyl, methoxy, -CO₂Me, hydroxy, amino, or -OCH₂CN.

4. The compound according to claim 1, wherein said compound is selected from the following Table 1 compounds:
| **No. I-** | **Structure** | **No. I-** | **Structure** |
|---|---|---|---|
| **25** | 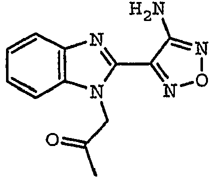 | **26** | 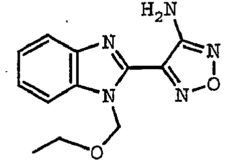 |
| **27** | 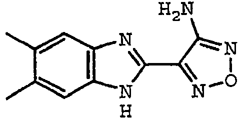 | **28** | 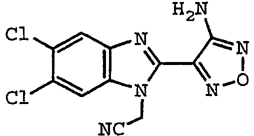 |
| **29** | 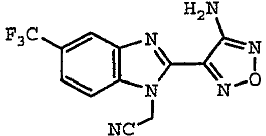 | **30** | 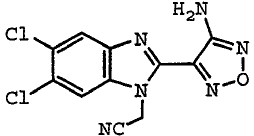 |
| **31** | 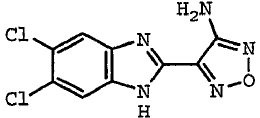 | **32** | 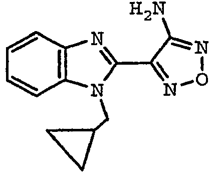 |
| | | **34** | 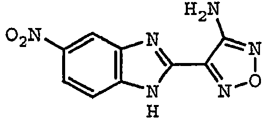 |
| **35** | 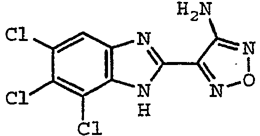 | **36** | 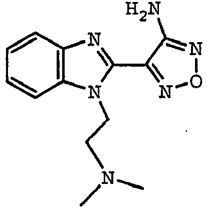 |
| **37** | 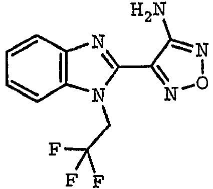 | | |
| **39** | 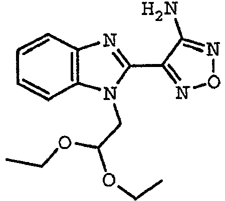 | **40** | 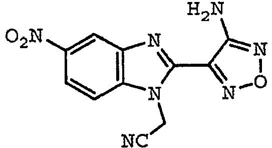 |
| **41** | 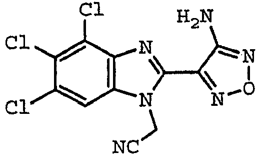 | **42** | 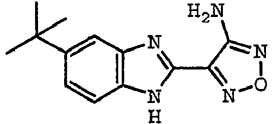 |
| **43** | 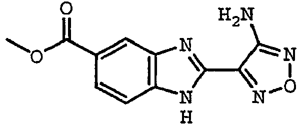 | **44** | 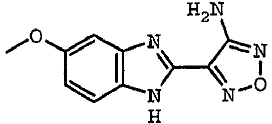 |
| **45** | 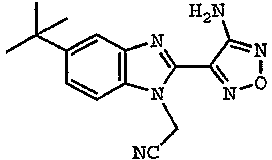 | **46** | 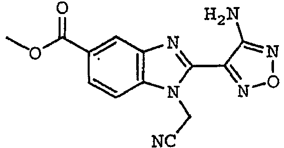 |
| **47** | 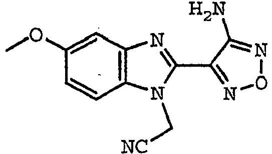 | **48** | 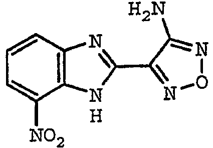 |
| **49** | 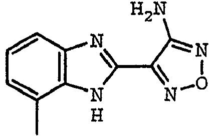 | **50** | 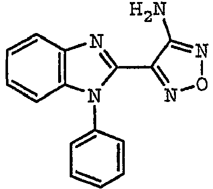 |
| **51** | 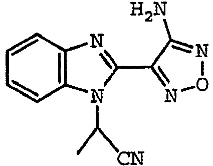 | **52** | 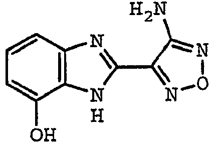 |
| **53** | 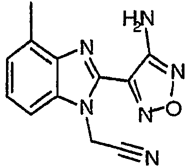 | **54** | 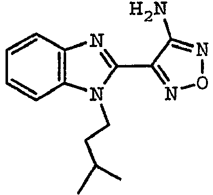 |
| | | **56** | 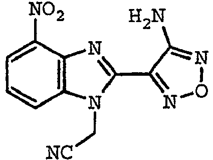 |
| **57** | 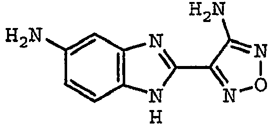 | **58** | 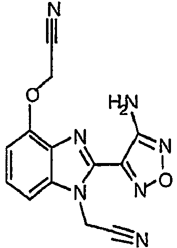 |
| **59** | 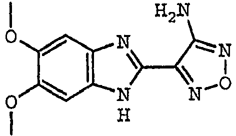 | **60** | 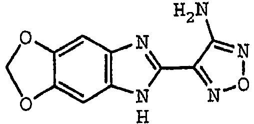 |
| | | | |
| | | | |
| | | | |
| **67** | 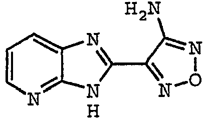 | **68** | 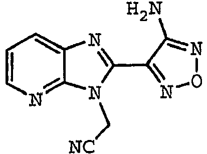 |
| **69** | 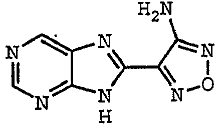 | | |
| | | | |
| | | | |
| | | **76** | 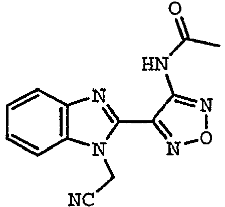 |
| | | | |
| | | **80** | 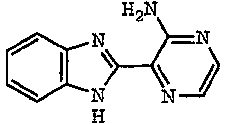 |
| | | | |
| **83** | 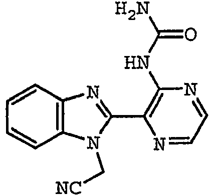 | | |
| **85** | 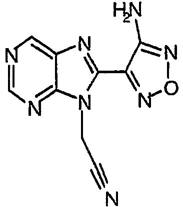 | | |
| | | **88** | 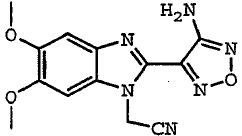 |
| | | **90** | 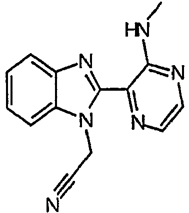 |
| | | **92** | 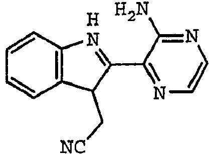 |
| **93** | 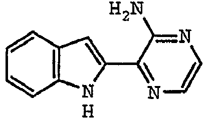 | **94** | 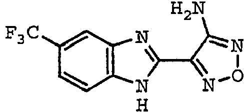 |
| | | | |
| | | | |
| | | | |
| **101** | 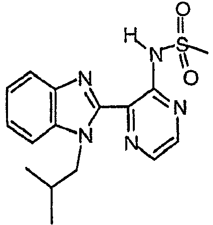 | **102** | 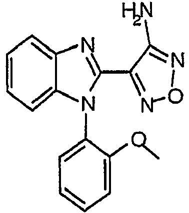 |
| **103** | 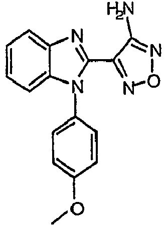 | **104** | 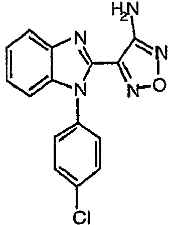 |
| **105** | 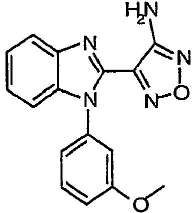 | | |
| **107** | 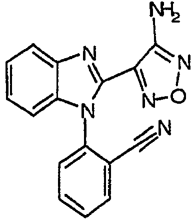 | **108** | 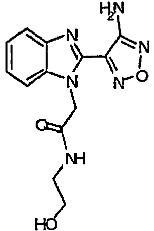 |
| **109** | 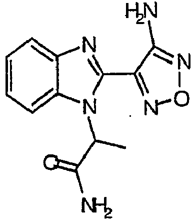 | | |
| **111** | 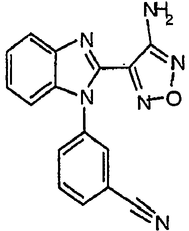 | | |
| **113** | 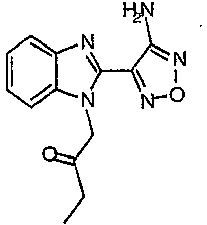 | **114** | 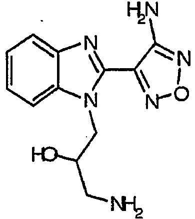 |
| **115** | 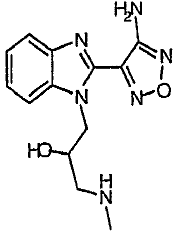 | **116** | 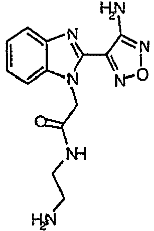 |
| **117** | 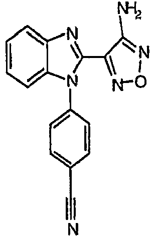 | **118** | 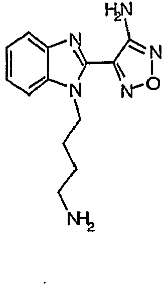 |
| **119** | 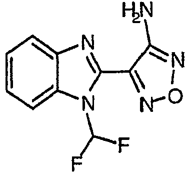 | **120** | 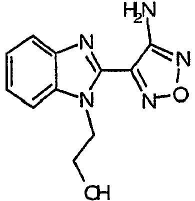 |
| **121** | 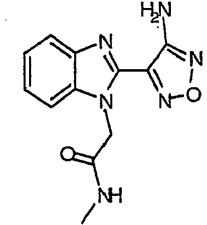 | **122** | 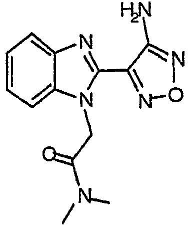 |
| **123** | 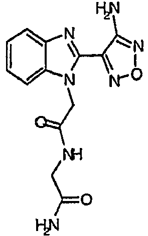 | **124** | 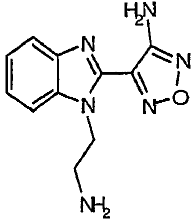 |
| **125** | 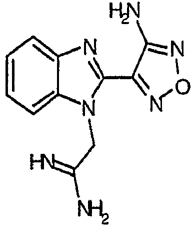 | **126** | 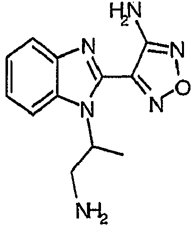 |
| **127** | 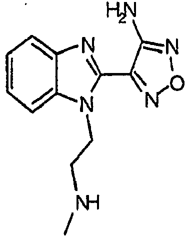 | **128** | 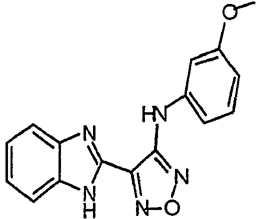 |
| **129** | 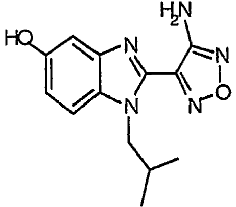 | **130** | 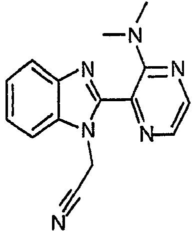 |
| **131** | 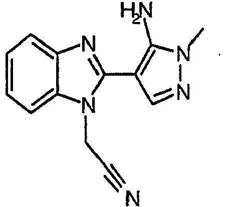 | **132** | 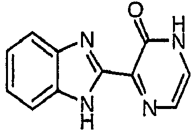 |
| **133** | 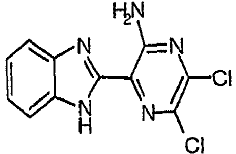 | **134** | 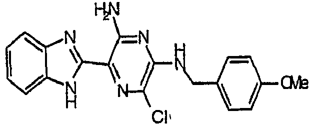 |
| **135** | 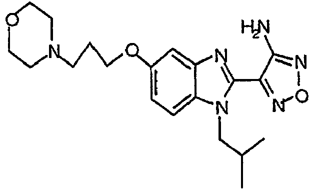 | | |

5. A composition comprising an effective amount of a compound according to claim 1, and a pharmaceutically acceptable carrier.

6. The composition according to claim 5, further comprising an additional therapeutic agent.

7. The composition of claims 5 or 6 or the compound of claims 1-4 for use in treating or lessening the severity of a disease, disorder, or condition selected from a neurological disorder, allergy, asthma, diabetes, Alzheimer's disease, Huntington's disease, Parkinson's disease, AIDS-associated dementia, amyotrophic lateral sclerosis (AML, Lou Gehrig's disease), multiple sclerosis (MS), schizophrenia, cardiomyocyte hypertrophy, reperfusion/ischemia, stroke, or baldness.

8. The compound or composition for use according to claim 7, wherein said disease is selected from a neurological disorder.

9. The compound or composition for use according to claim 7, wherein said disease is stroke.

10. The compound or composition for use according to claim 7, wherein said disease is selected from transplant rejection, allergies, rheumatoid arthritis, or leukemia.

11. The compound or composition for use according to claim 10, wherein said disease is selected from transplant rejection or rheumatoid arthritis.

12. An in-vitro method of inhibiting GSK-3 or Lck kinase in a biological sample with a compound of claims 1-4.

## Patentansprüche

1. Verbindung mit der Formel **Ia** oder **Ib:** 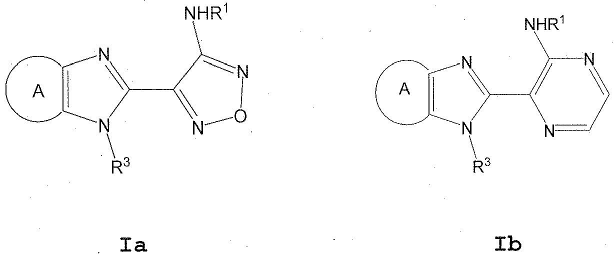 oder ein pharmazeutisch verträgliches Salz davon, wobei:
- Ring A einen 5- bis 7-gliedrigen teilweise ungesättigten oder vollständig ungesättigten Ring mit 0 bis 3 Heteroatomen, die unabhängig voneinander aus Stickstoff, Sauerstoff oder Schwefel ausgewählt sind, bedeutet und Ring A wahlweise mit einem gesättigten, teilweise ungesättigten oder vollständig ungesättigten 5- bis 8-gliedrigen Ring mit 0 bis 3 Heteroatomen, die unabhängig voneinander aus Stickstoff, Sauerstoff oder Schwefel ausgewählt sind, anelliert ist, wobei dieser Ring A und der wahlweise mit Ring A anellierte Ring an dem ungesättigten Kohlenstoffatom einer Aryl-, Heteroaryl-, Aralkyl- oder Heteroaralkylgruppe mit Halogen, Oxo, N₃, -R°, -OR°, SR°, 1,2-Methylendioxy, 1,2-Ethylenedioxy, Phenyl (Ph), mit R° substituiertem Ph, -O(Ph), mit R° substituiertem O-(Ph), -CH₂(Ph), mit R° substituiertem -CH₂(Ph), -CH₂CH₂(Ph), mit R° substituiertem -CH₂CH₂(Ph), NO₂, -CN, -N(R°)₂, -NR°C(O)R°, -NR°C(O)N(R°)₂, -NR°CO₂R°, -NR°NR°C(O)R°, -NR°NR°C(O)N(R°)₂, -NR°NR°CO₂R°, -C(O)C(O)R°, -C(O)CH₂C(O)R°, -CO₂R°, -C(O)R°, -C(O)N(R°)₂, -OC(O)N(R°)₂, -S(O)₂R°, -SO₂N(R°)₂, -S(O)R°, -NR°SO₂N(R°)₂, -NR°SO₂R°, -C(=S)N(R°)2, -C(=NH)-N(R°)₂ oder -(CH₂)_{Y}NHC(O)R° wahlweise substituiert sind, wobei y 0 bis 4 ist und jeder R° unabhängig voneinander aus Wasserstoff, wahlweise substituiertem C₁- bis C₆-aliphatisch, einem unsubstituierten 5- bzw. 6-gliedrigen heterocyclischen oder Heteroarylring mit 0 bis 4 Heteroatomen, die unabhängig voneinander aus Stickstoff, Sauerstoff oder Schwefel ausgewählt sind, Phenyl (Ph), -O(Ph) oder -CH₂(Ph)-CH₂(Ph) ausgewählt ist, wobei jeder optionale Substituent an der aliphatischen Gruppe von R° unabhängig voneinander aus NH₂, NH(C₁- bis C₄-aliphatisch), N(C₁- bis C₄-aliphatisch)₂, Halogen, C₁- bis C₄-aliphatisch, OH, O-(C₁- bis C₄-aliphatisch), NO₂, CN, CO₂H, CO₂(C₁- bis C₄-aliphatisch), -O(HalogenC₁- bis C₄-aliphatisch) oder HalogenC₁- bis C₄-aliphatisch ausgewählt ist, wobei dieser Ring A und der wahlweise mit Ring A anellierte Ring an dem gesättigten Kohlenstoffatom einer aliphatischen Gruppe oder eines nichtaromatischen heterocyclischen Rings wahlweise mit den Substituenten substituiert sind, die aus denjenigen, die weiter oben für das ungesättigte Kohlenstoffatom einer Aryl- oder Heteroarylgruppe aufgezählt sind, und =O, =S, =NNHR*, N(R*)₂, =N-, =NNHC(O)R*, =NNHCO₂(Alkyl), =NNHSO₂(Alkyl) oder =NR* ausgewählt sind, wobei jeder R* unabhängig voneinander aus Wasserstoff oder wahlweise substituiertem C₁- bis C₆-aliphatisch ausgewählt ist, wobei die Substituenten an der aliphatischen Gruppe von R* aus NH₂, NH(C₁- bis C₄-aliphatisch), N(C₁- bis C₄-aliphatisch)₂, Halogen, C₁- bis C₄-aliphatisch, OH, O-(C₁- bis C₄-aliphatisch), NO₂, CN, CO₂H, CO₂(C₁- bis C₄-aliphatisch)-O(HalogenC₁- bis C₄-aliphatisch oder HalogenC₁- bis C₄-aliphatisch ausgewählt sind,
- R¹ aus R oder R²,
- R² aus -SO₂R, -SO₂N(R)₂, -CN, -C(O)R, -CO₂R oder -CON(R)₂ und
- R unabhängig voneinander aus Wasserstoff oder einer wahlweise substituierten Gruppe, die aus C₁- bis C₆-aliphatisch und einem 3- bis 6-gliedrigen gesättigten, teilweise ungesättigten oder Arylring mit 0 bis 4 Heteroatomen, die unabhängig voneinander aus Stickstoff, Sauerstoff oder Schwefel ausgewählt sind, ausgewählt ist, oder - zwei R-Gruppen an demselben Stickstoff mit dem Stickstoff, an welchen sie gebunden sind, zusammengenommen werden, um einen 3- bis 7-gliedrigen heterocyclischen oder Heteroarylring mit 1 bis 4 Heteroatomen, die unabhängig voneinander aus Stickstoff, Sauerstoff oder Schwefel ausgewählt sind, zu bilden, wobei der optionale Substituent wie weiter oben aufgezählt ausgewählt ist,
- R³ aus T-CN oder L-R ausgewählt ist,
- T eine Valenzbindung oder eine optional substituierte C₁- bis C₆-Alkylidenkette bedeutet, wobei der optionale Substituent wie weiter oben für die Substituenten an dem gesättigten Kohlenstoffatom einer aliphatischen Gruppe aufgezählt ausgewählt ist, und
- L eine Valenzbindung oder eine C₁- bis C₄-Alkylidenkette, wobei bis zu zwei Methyleneinheiten von L wahlweise und unabhängig voneinander durch -S-, -NR-, -NRC(O)-, -NRC(O)NR-, -OC(O)NR-, -CO₂-, -NRCO₂-, -C(O)NR-, -SO₂NR-, -NRSO₂- oder -NRSO₂NR-ersetzt sind, bedeutet,
unter der Voraussetzung, dass diese Verbindung keine aus der Gruppe ist, die besteht aus:
- 4-[1-(4-Chlorbenzyl)-1H-benzimidazol-2-yl]-furazan-3-ylamin (**I**-1),
- 4-(1-Prop-2-inyl-1H-benzimidazol-2-yl)-furazan-3-ylamin (**I**-2),
- 4-(5-Methyl-1H-benzimidazol-2-yl)-furazan-3-ylamin (**I**-3),
- 4-[1-(2-Chlor-6-fluorbenzyl)-1H-benzimidazol-2-yl]-furazan-3-ylamin (**I**-4),
- [2-(4-Aminofurazan-3-yl)-benzimidazol-1-yl]-essigsäure (**I**-5),
- 2-[2-(4-Aminofurazan-3-yl)-benzimidazol-1-yl]-acetamid (**I**-6),
- 4-(1-Propyl-1H-benzimidazol-2-yl)-furazan-3-ylamin (**I**-7),
- 4-[1-(2,6-Dichlorbenzyl)-1H-benzimidazol-2-yl]-furazan-3-ylamin (**I**-8),
- 4-(1-Allyl-1H-benzimidazol-2-yl)-furazan-3-ylamin (**I**-9),
- 4-[1-(4-Methylbenzyl)-1H-benzimidazol-2-yl]-furazan-3-ylamin (**I**-10),
- 4-(1-Isopropyl-1H-benzimidazol-2-yl)-furazan-3-ylamin (**I**-11),
- 4-[1-(2-Methylbenzyl)-1H-benzimidazol-2-yl]-furazan-3-ylamin (**I**-12),
- [2-(4-Aminofurazan-3-yl)-benzimidazol-1-yl]-acetonitril (**I**-13),
- 4-[1-(1H-Tetrazol-5-ylmethyl)-1H-benzimidazol-2-yl]-furazan-3-ylamin (**I**-14),
- 4-[1-(2,4-Dichlorbenzyl)-1H-benzimidäzol-2-yl]-furazan-3-ylamin (**I**-15),
- 4-[1-(3,4-Dichlorbenzyl)-1H-benzimidazol-2-yl]-furazan-3-ylamin (**I**-16),
- 2-[2-(4-Aminofurazan-3-yl)-benzimidazol-1-yl]-N-(3,4-dimethoxyphenyl)-acetamid (**I**-17),
- 4-(1H-Benzimidazol-2-yl)-furazan-3-ylamin (**I**-18),
- 2-[2-(4-Aminofurazan-3-yl)-benzimidazol-1-yl]-N-(3,4-difluorphenyl)-acetamid (**I**-19),
- 2-[2-(4-Aminofurazan-3-yl)-benzimidazol-1-ylmethyl]-benzonitril (**I**-20),
- 2-[2-(4-Aminofurazan-3-yl)-benzimidazol-1-yl]-N-(2-trifluormethylphenyl)-acetamid (**I**-21),
- 4-[1-(3-Brombenzyl)-1H-benzimidazol-2-yl]-furazan-3-ylamin (**I**-22),
- 4-[2-(4-Aminofurazan-3-yl)-benzimidazol-1-yl]-butyronitril (**I**-23),
- 4-(1-Ethyl-1H-benzimidazol-2-yl)-furazan-3-ylamin (**I**-55),
- 2-[2-(4-Aminofurazan-3-yl)-benzimidazol-1-yl]-N-(2-fluorphenyl)-acetamid (**I**-61),
- 4-(1-Methyl-1H-benzimidazol-2-yl)-furazan-3-ylamin (**I**-62),
- 2-[2-(4-Aminofurazan-3-yl)-benzimidazol-1-yl]-N-biphenyl-2-yl-acetamid (**I**-63),
- 2-[2-(4-Aminofurazan-3-yl)-benzimidazol-2-yl]-N-(2,6-dimethylphenyl)-acetamide (**I**-64),
- 2-[2-(4-Aminofurazan-3-yl)-benzimidazol-1-yl]-N-tert.-butylacetamid (**I**-65),
- 2-[2-(4-Aminofurazan-3-yl)-benzimidazol-1-yl]-N-(3-fluorphenyl)-acetamid (**I**-66),
- 2-[2-(4-Aminofurazan-3-yl)-benzimidazol-1-yl]-N-(2-fluorphenyl)-acetamid (**I**-70),
- N-[4-(1-Ethyl-1H-benzimidazol-2-yl)-furazan-3-yl]-2,2,2-trifluoracetamid (**I**-72),
- N-[4-(1-Cyanomethyl-1H-benzimidazol-2-yl)-furazan-3-yl]-acetamid (**I**-76),
- 2-(4-Amino-1,2,5-oxadiazol-3-yl)-1H-benzimidazol-1-acetonitril,
- 2-(4-Amino-1,2,5-oxadiazol-3-yl)-N-(2,4-difluorphenyl)-1H-benzimidazol-1-acetamid,
- 4-[1-(Cyclopropylmethyl)-1H-benzimidazol-2-yl-1,2,5-oxadiazol-3-amin,
- N-[4-[1-(Cyclopropylmethyl)-1H-benzimidazol-2-yl]-1,2,5-oxadiazol-3-yl]-acetamid,
- 4-[1-(2-Methylpropyl)-1H-benzimidazol-2-yl]-1,2,5-oxadiazol-3-amin,
- 4-[1-(Phenylmethyl)-1H-benzimidazol-2-yl-1,2,5-oxadiazol-3-amin,
- 2-(4-Amino-1,2,5-oxadiazol-3-yl)-N-[4-(trifluormethoxy)phenyl]-1H-benzimidazol-1-acetamid,
- 2-(4-Amino-1,2,5-oxadiazol-3-yl)-1H-benzimidazol-1-essigsäureethylester,
- N-[4-[1-[(5-Methyl-1,3,4-oxadiazol-2-yl)-methyl]-1H-benzimidazol-2-yl]-1,2,5-oxadiazol-3-yl-acetamid,
- 4-(1-Methyl-5-nitro-1H-benzimidazol-2-yl)-1,2,5-oxadiazol-3-amin,
- 3-Amino-4-(1H-benzimidazol-2-yl)-1,2,5-oxadiazol,
- 3-Amino-4-(1-ethyl-1H-benzimidazol-2-yl)-1,2,5-oxadiazol,
- 4-(1H-Benzo[d]imidazol-2-yl)-1,2,5-oxadiazol-3-amin, und
- Methyl-2-[2-(4-amino-1,2,5-oxadiazol-3-yl)-(1H-benzimidazol-1-yl)]-acetat.

2. Verbindung nach Anspruch 1, wobei:
- R¹ aus R, -SO₂R oder -C(O)R und
- R³ aus T-CN oder L-R ausgewählt ist,
- L eine Valenzbindung oder eine C₁- bis C₄-Alkylidenkette, wobei eine Methyleneinheit von L wahlweise durch -CO₂-, -C(O)NR-, -C(O)-, -N(R)- oder -O- ersetzt ist,
- R Wasserstoff oder eine wahlweise substituierte Gruppe, die aus C₁- bis C₄-aliphatisch, 3- bis 6-gliedrigem Heterocyclyl mit 1 bzw. 2 Heteroatomen, die unabhängig voneinander aus Stickstoff, Sauerstoff oder Schwefel ausgewählt sind, Phenyl oder einem 5- bzw. 6-gliedrigen Heteroarylring mit 1 bis 4 Heteroatomen, die unabhängig voneinander aus Stickstoff, Sauerstoff oder Schwefel ausgewählt sind, ausgewählt ist, wobei der optionale Substituent wie in Anspruch 1 definiert ist, und
- T eine C₁- bis C₄-Alkylidenkette bedeutet.

3. Verbindung nach Anspruch 2, wobei:
- R¹ aus R, -SO₂R oder -C(O)R ausgewählt ist,
- R Wasserstoff oder eine wahlweise substituierte C₁- bis C₄-aliphatische Gruppe bedeutet,
- R³ aus Wasserstoff, -CH₂CN, -CH₂C(O)NH₂, -CH₂CO₂H, Propyl, -CH₂CH₂=CH₂, Isopropyl, -(CH₂)₃CN, -CH₂OEt, -CH₂CF₃, Isobutyl, Cyclopropylmethyl, -CH₂CH₂N(Me)₂, -CH₂CH(OEt)₂, Ethyl, -CH₂C(O)NH*t-*butyl oder einer wahlweise substituierten Benzyl- oder -CH₂C(O)NHphenylgruppe ausgewählt ist und
jedes substituierbare Kohlenstoffatom an dem Benzoring unabhängig voneinander und wahlweise mit Chlor, Brom, Methyl, -CF₃, Nitro, *tert*.-Butyl, Methoxy, -CO₂Me, Hydroxy, Amino oder -OCH₂CN substituiert ist.

4. Verbindung nach Anspruch 1, wobei diese Verbindung aus den Verbindungen der folgenden Tabelle 1 ausgewählt ist:
| **Nr. I-** | **Strukturformel** | **Nr. I-** | **Strukturformel** |
|---|---|---|---|
| **25** | 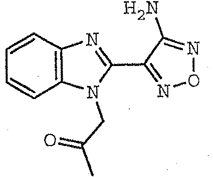 | **26** | 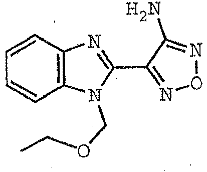 |
| **27** | 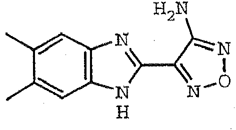 | **28** | 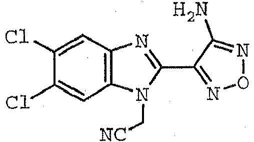 |
| **29** | 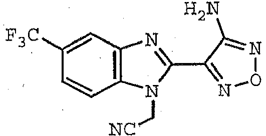 | **30** | 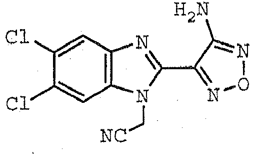 |
| **31** | 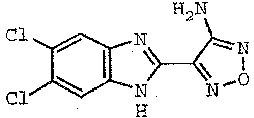 | **32** | 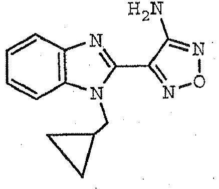 |
| | | **34** | 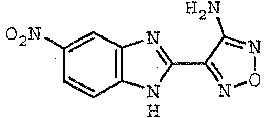 |
| **35** | 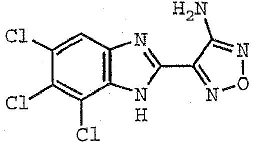 | **36** | 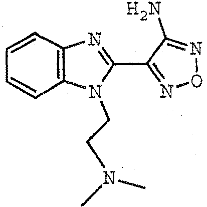 |
| **37** | 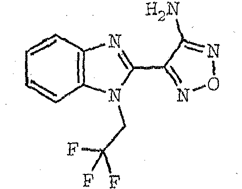 | | |
| **39** | 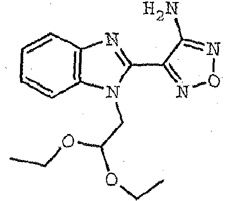 | **40** | 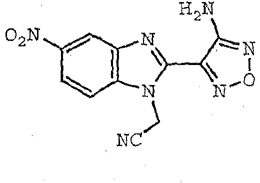 |
| **41** | 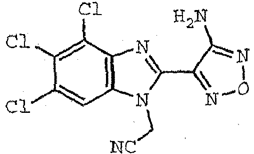 | **42** | 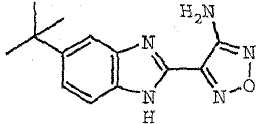 |
| **43** | 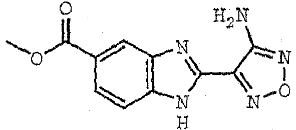 | **44** | 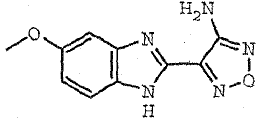 |
| **45** | 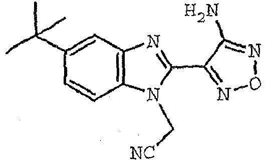 | **46** | 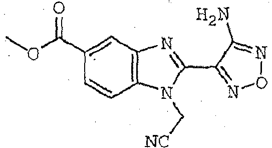 |
| **47** | 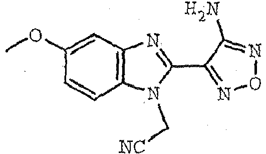 | **48** | 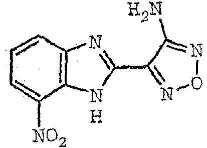 |
| **49** | 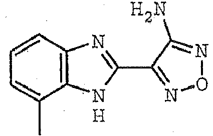 | **50** | 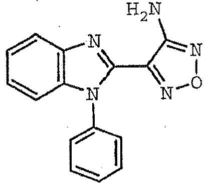 |
| **51** | 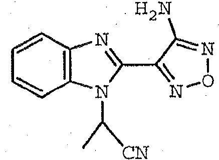 | **52** | 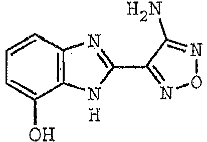 |
| **53** | 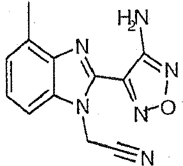 | **54** | 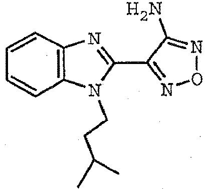 |
| | | **56** | 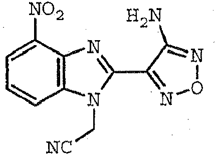 |
| **57** | 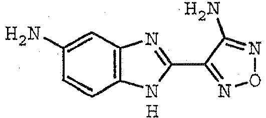 | **58** | 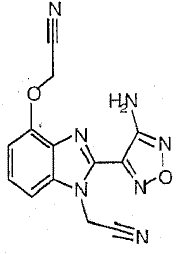 |
| **59** | 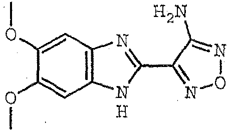 | **60** | 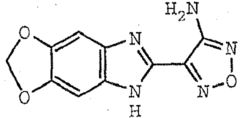 |
| | | | |
| | | | |
| | | | |
| **67** | 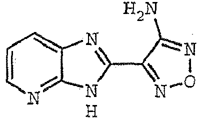 | **68** | 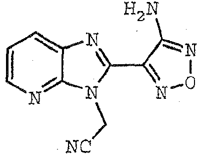 |
| **69** | 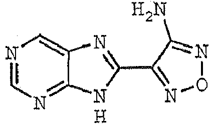 | | |
| | | | |
| | | | |
| | | **76** | 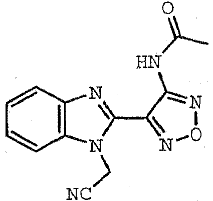 |
| | | | |
| | | **80** | 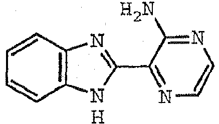 |
| | | | |
| **83** | 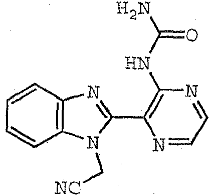 | | |
| **85** | 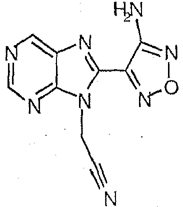 | | |
| | | **88** | 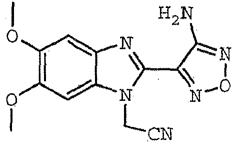 |
| | | **90** | 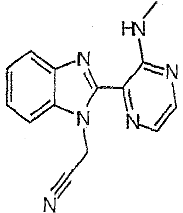 |
| | | **92** | 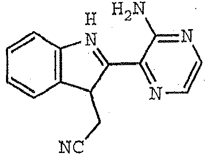 |
| **93** | 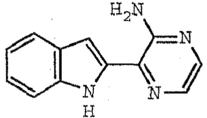 | **94** | 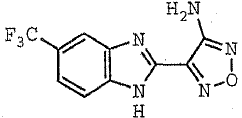 |
| | | | |
| | | | |
| | | | |
| **101** | 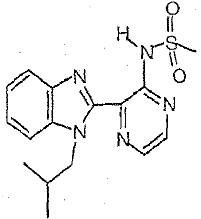 | **102** | 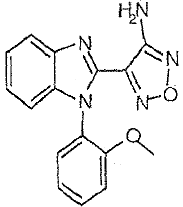 |
| **103** | 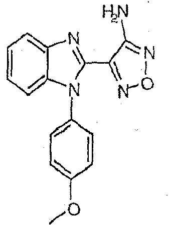 | **104** | 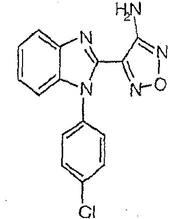 |
| **105** | 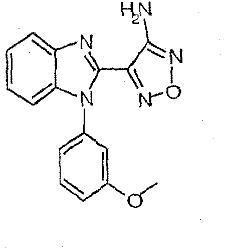 | | |
| **107** | 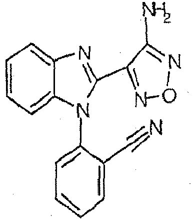 | **108** | 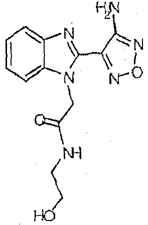 |
| **109** | 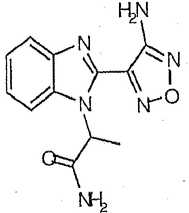 | | |
| **111** | 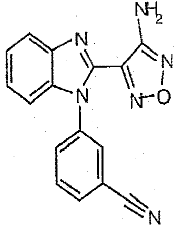 | | |
| **113** | 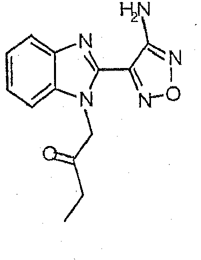 | **114** | 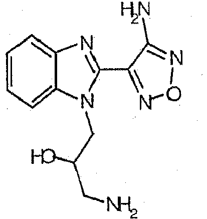 |
| **115** | 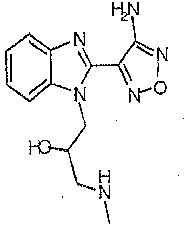 | **116** | 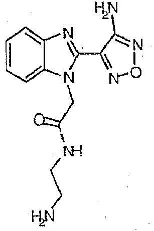 |
| **117** | 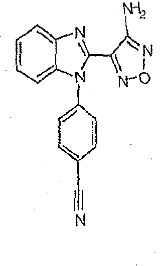 | **118** | 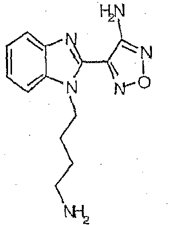 |
| **119** | 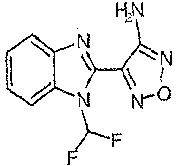 | **120** | 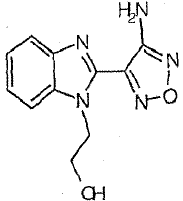 |
| **121** | 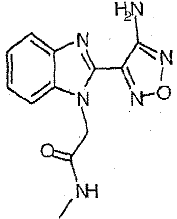 | **122** | 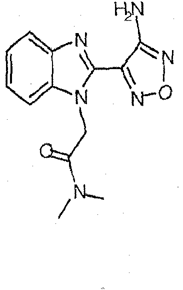 |
| **123** | 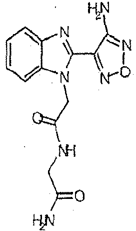 | **124** | 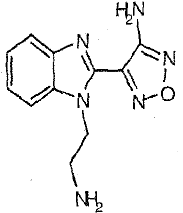 |
| **125** | 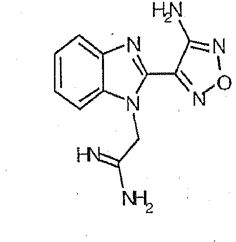 | **126** | 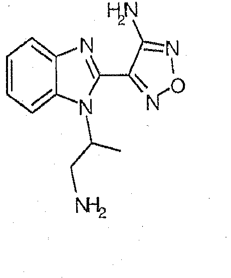 |
| **127** | 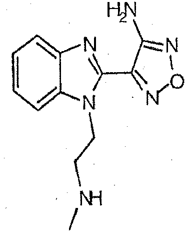 | **128** | 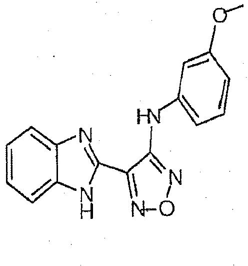 |
| **129** | 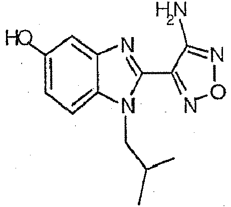 | **130** | 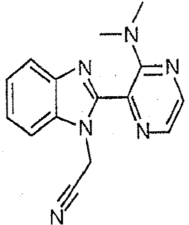 |
| **131** | 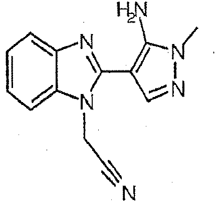 | **132** | 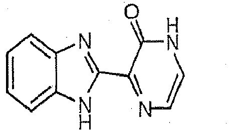 |
| **133** | 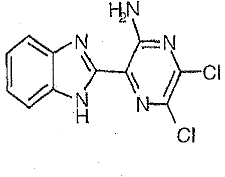 | **134** | 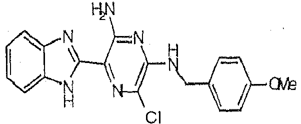 |
| **135** | 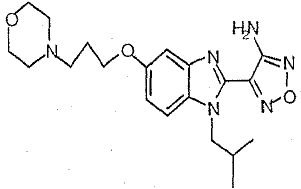 | | |

5. Zubereitung, die einen wirksamen Anteil einer Verbindung nach Anspruch 1 und einen pharmazeutisch verträglichen Träger umfasst.

6. Zubereitung nach Anspruch 5, die ferner ein zusätzliches therapeutisches Mittel umfasst.

7. Zubereitung nach Anspruch 5 oder 6 bzw. Verbindung nach den Ansprüchen 1 bis 4 für eine Verwendung bei der Behandlung oder Milderung der Schwere einer Krankheit, einer Störung oder eines Zustands, die/der aus einer neurologischen Störung, Allergie, Asthma, Diabetes, Alzheimer-Krankheit, Chorea Huntington, Parkinson-Syndrom, AIDS-assoziierter Demenz, amyotropher Lateralsklerose (AML, Lou-Gehrig-Krankheit), Multipler Sklerose (MS), Schizophrenie, Kardiomyozythypertrophie, Reperfusion/Ischämie, Schlaganfall oder Kahlköpfigkeit ausgewählt ist.

8. Verbindung oder Zubereitung für eine Verwendung nach Anspruch 7, wobei die Krankheit aus einer neurologischen Störung ausgewählt ist.

9. Verbindung oder Zubereitung für eine Verwendung nach Anspruch 7, wobei die Krankheit ein Schlaganfall ist.

10. Verbindung oder Zubereitung für eine Verwendung nach Anspruch 7, wobei die Krankeit aus Transplantatabstoßung, Allergien, rheumatoider Arthritis oder Leukämie ausgewählt ist.

11. Verbindung oder Zubereitung für eine Verwendung nach Anspruch 10, wobei die Krankheit aus Transplantatabstoßung oder rheumatoider Arthritis ausgewählt ist.

12. In-vitro-Verfahren zum Inhibieren der GSK-3 oder Lck-Kinase in einer biologischen Probe mit einer Verbindung nach den Ansprüchen 1 bis 4.

## Revendications

1. Composé de formule Ia ou Ib : 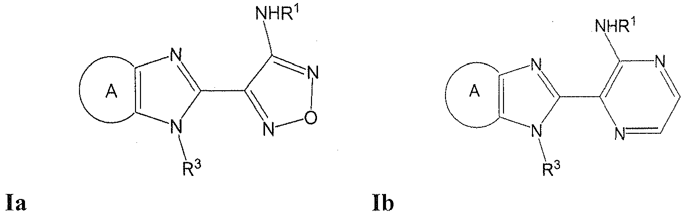 ou sel pharmaceutiquement acceptable de celui-ci, où :
le cycle A est un cycle partiellement insaturé ou complètement insaturé de 5-7 membres, ayant 0-3 hétéroatomes choisis indépendamment parmi azote, oxygène et soufre, et où le cycle A est le cas échéant condensé à un cycle saturé, partiellement insaturé ou complètement insaturé de 5-8 membres, ayant 0,-3 hétéroatomes choisis indépendamment parmi azote, oxygène et soufre, où le cycle A et le cycle le cas échéant condensé au cycle A sont le cas échéant substitués sur le carbone insaturé d'un radical aryle, hétéroaryle, aralkyle ou hétéroaralkyle par halogène, oxo, N₃, -R°, -OR°, SR°, 1,2-méthylènedioxy, 1,2-éthylènedioxy, phényle (Ph), Ph substitué par R°, - O(Ph), O-(Ph) substitué par R°, -CH₂(Ph), -CH₂(Ph) substitué par R°, -CH₂CH₂ (Ph), -CH₂CH₂ (Ph) substitué par R°, N0₂, -CN, -N(R°)₂, -NR°C(O)R°, -NR°C(O)N(R°)₂, - NR°CO₂R°, -NR°NR°C(O)R°, -NR°NR°C(O)N(R°)₂, -NR°NR°CO₂R°, -C(O)C(o)R)), -C(O)CH₂C(O)R°, -CP₂R°, -C(O)R°, - C(O)N(R°)₂, -OC(O)N(R°)₂, -S(O)₂R°, -SO₂N(R°)₂, -S(O)R°, -NR°SO₂N(R°)₂, -NR°SO₂R°, -C(=S)N(R°)₂, -C(=NH)-N(R°)_{2,} ou -(CH₂)_{Y}NHC(O)R°, où y est 0-4, chaque R° est choisi indépendamment parmi hydrogène, aliphatique en C₁₋₆ le cas échéant substitué, hétéroaryle ou hétérocyclyle de 5-6 membres non substitué, ayant 0-4 hétéroatomes choisis indépendamment parmi azote, oxygène et soufre, phényle (Ph), -O(Ph) ou -CH₂(Ph)-CH₂(Ph), et où chaque substituant facultatif sur le radical aliphatique de R° est indépendamment choisi parmi NH₂, NH(aliphatique en C₁₋₄), N (aliphatique en C₁₋₄)₂ halogène, aliphatique en C₁-₄, OH, 0- (aliphatique en C₁₋₄), NO₂, CN, CO₂H, CO₂(aliphatique en C₁₋₄), -O(haloaliphatique en C₁₋₄), ou haloaliphatique en C₁₋₄ ; et où le cycle A et le cycle le cas échéant condensé au cycle A sont le cas échéant substitués sur l'atome de carbone saturé d'un radical aliphatique ou d'un hétérocycle non aromatique par les substituants choisis parmi ceux mentionnés ci-dessus pour le carbone insaturé d'un radical aryle ou hétéroaryle et =O, =S, =NNHR*, NN(R*)₂, =N-, =NNHC(O)R*, =NNHCO₂(alkyl), =NNHSO₂(alkyl), ou =NR*, où chaque R* est indépendamment choisi parmi hydrogène ou un aliphatique en C₁₋₆ le cas échéant substitué, les substituants sur le radical aliphatique de R* étant choisis parmi NH₂, NH (aliphatique en C₁₋₄), N (aliphatique en C₁₋₄)₂, halogène, aliphatique en C₁₋₄, OH, O-(aliphatique en C₁₋₄), NO₂, CN, CO₂H, CO₂(aliphatique en C₁₋₄), -O(haloaliphatique en C₁₋₄), ou haloaliphatique en C₁₋₄ ;
R¹ est choisi parmi R ou R² ;
R² est choisi parmi -SO₂R, -SO₂N(R)₂, -CN, - C(O)R, -CO₂R, ou -CON(R)₂ ;
R est choisi indépendamment parmi hydrogène ou un radical le cas échéant substitué, choisi parmi : aliphatique en C₁₋₆, cycle saturé, partiellement insaturé ou aryle de 3-6 membres, ayant 0-4 hétéroatomes indépendamment choisis parmi azote, oxygène et soufre, ou
deux radicaux R sur le même azote sont pris avec l'azote lié pour former un hétérocycle ou hétéroaryle de 3-7 membres, ayant 1-4 hétéroatomes choisis indépendamment parmi azote, oxygène et soufre, où le substituant facultatif est choisi comme mentionné ci-dessus ;
R³ est choisi parmi T-CN ou L-R ;
T est une liaison de valence ou une chaine alkylidène en C₁₋₆ le cas échéant substituée, le substituant facultatif étant choisi comme mentionné ci-dessus pour les substituants du carbone saturé d'un radical aliphatique, et
L est une liaison de valence ou une chaine alkylidène en C₁₋₄, où jusqu'à deux unités méthylène de L sont le cas échéant et indépendamment remplacées par -S-, -NR-, -NRC(O)-, -NRC(O)NR-, -OC(O)NR-, , -CO₂-,-NRCO₂-, -C (0) NR-, -SO₂NR-, -NRSO₂-, ou -NRSO₂NR- ;
pourvu que ledit composé est autre que l'un du groupe consistant en :
la 4-[1-(4-chlorobenzyl)-1H-benzoimidazol-2-yl]-furazan-3-ylamine (**I**-1) ;
la 4-(1-prop-2-ynyl-1H-benzoimidazol-2-yl)furazan-3-ylamine (**I**-2) ;
la 4-(5-méthyl-1H-benzoimidazol-2-yl)furazan-3-ylamine (**I**-3) ;
la 4-[1-(2-chloro-6-fluorobenzyl)-1H-benzoimidazol-2-yl]-furazan-3-ylamine (**I**-4) ;
l'acide [2-(4-aminofurazan-3-yl)benzoimidazol-1-yl]acétique (**I**-5) ;
le 2-[2-(4-aminofurazan-3-yl)benzoimidazol-1-yl]-acétamide (**I**-6) ;
la 4-(1-propyl-1H-benzoimidazol-2-yl)furazan-3-ylamine (**I**-7) ;
la 4-[1-(2,6-dichlorobenzyl)-1H-benzoimidazol-2-yl]-furazan-3-ylamine (**I**-8) ;
la 4-(1-allyl-1H-benzoimidazol-2-yl)furazan-3-ylamine (**I**-9) ;
la 4-[1-(4-méthylbenzyl)-1H-benzoimidazol-2-yl]-furazan-3-ylamine (**I**-10) ;
la 4-(1-isopropyl-1H-benzoimidazol-2-yl)furazan-3-ylamine (**I**-11) ;
la 4-[1-(2-méthylbenzyl)-1H-benzoimidazol-2-yl]-furazan-3-ylamine (**I**-12) ;
le [2-(4-aminofurazan-3-yl)benzoimidazol-1-yl]-acétonitrile (**I**-13) ;
la 4-[1-(1H-tétrazol-5-ylméthyl)-1H-benzoimidazol-2-yl]furazan-3-ylamine (**I**-14) ;
la 4-[1-(2,4-dichlorobenzyl)-1H-benzoimidazol-2-yl]-furazan-3-ylamine (**I**-15) ;
la 4-[1-(3,4-dichlorobenzyl)-1H-benzoimidazol-2-yl]-furazan-3-ylamine (**I**-16) ;
le 2-[2-(4-aminofurazan-3-yl)benzoimidazol-1-yl]-N-(3,4-diméthoxyphényl)acétamide (**I**-17) ;
la 4-(1H-benzoimidazol-2-yl)furazan-3-ylamine (**I-**18) ;
le 2-[2-(4-aminofurazan-3-yl)benzoimidazol-1-yl]-N-(3,4-difluorophényl)acétamide (**I**-19) ;
le 2-[2-(4-aminofurazan-3-yl)benzoimidazol-1-ylméthyl]benzonitrile (**I**-20) ;
le 2-[2-(4-aminofurazan-3-yl)benzoimidazol-1-yl]-N-(2-trifluorométhylphényl)acétamide (**I**-21) ;
la 4-[1-(3-bromobenzyl)-1H-benzoimidazol-2-yl]-furazan-3-ylamine (**I**-22) ;
le 4-[2-(4-aminofurazan-3-yl)benzoimidazol-l-yl]-butyronitrile (**I**-23) ;
la 4-(1-éthyl-1H-benzoimidazol-2-yl)furazan-3-ylamine (**I**-55) ;
le 2-[2-(4-aminofurazan-3-yl)benzoimidazol-1-yl]-N-(2-fluorophényl)acétamide (**I**-61) ;
la 4-(1-méthyl-1H-benzoimidazol-2-yl)furazan-3-ylamine (**I**-62) ;
le 2-[2-(4-aminofurazan-3-yl)benzoimidazol-1-yl]-N-biphényl-2-ylacétamide (**I**-63) ;
le 2-[2-(4-aminofurazan-3-yl)benzoimidazol-1-yl]-N-(2,6-diméthylphényl)acétamide (**I**-64) ;
le 2-[2-(4-aminofurazan-3-yl)benzoimidazol-1-yl]-N-tert-butylacétamide (**I**-65) ;
le 2-[2-(4-aminofurazan-3-yl)benzoimidazol-1-yl]-N-(3-fluorophényl)acétamide (**I**-66) ;
le 2-[2-(4-aminofurazan-3-yl)benzoimidazol-1-yl]-N-(2-fluorophényl)acétamide (**I**-70) ;
le N-[4-(1-éthyl-1H-benzoimidazol-2-yl)furazan-3-yl]-2,2,2-trifluoroacétamide (**I**-72) ;
le N-[4-(1-cyanométhyl-1H-benzoimidazol-2-yl)-furazan-3-yl]acétamide (**I**-76) ;
le 2-(4-amino-1,2,5-oxadiazol-3-yl)-1H-benz-imidazole-1-acétonitrile ;
le 2-(4-amino-1,2,5-oxadiazol-3-yl)-N-(2,4-difluorophényl)-1H-benzimidazole-1-acétamide ;
la 4-[1-(cyclopropylméthyl)-1H-benzimidazol-2-yl-1,2,5-oxadiazol-3-amine ;
le N-[4-[1-(cyclopropylméthyl)-1H-benzimidazol-2-yl]-1,2,5-oxadiazol-3-yl]acétamide ;
la 4-[1-(2-méthylpropyl)-1H-benzimidazol-2-yl]-1,2,5-oxadiazol-3-amine ;
la 4-[1-(phénylméthyl)-1H-benzimidazol-2-yl-1,2,5-oxadiazol-3-amine ;
le 2-(4-amino-1,2,5-oxadiazol-3-yl)-N-[4-(trifluorométhoxy)phényl]-1H-benzimidazole-1-acétamide ;
l'ester éthylique de l'acide 2-(4-amino-1,2,5-oxadiazol-3-yl)-1H-benzimidazole-1-acétique ;
le N-[4-[1-[(5-méthyl-1,3,4-oxadiazol-2yl)méthyl]-lH-benzimidazol-2-yl]-1,2,5-oxadiazol-3ylacétamide ;
la 4-(1-méthyl-5-nitro-1H-benzimidazol-2-yl)-1,2,5-oxadiazol-3-amine ;
le 3-amino-4-(1H-benzimidazol-2-yl)-1,2,5-oxadiazole ;
le 3-amino-4-(1-éthyl-1H-benzimidazol-2-yl)-1,2,5-oxadiazole ;
la 4-(1H-benzo[d]imidazol-2-yl)-1,2,5-oxadiazol-3-amine ; et
le 2-[2-(4-amino-1,2,5-oxadiazol-3yl)-(1H-benzimidazol-1-yl)]acétate de méthyle.

2. Composé selon la revendication 1, où :
R¹ est choisi parmi R, -SO₂R, ou -C(O)R ;
R³ est choisi parmi T-CN ou L-R ;
L est une liaison de valence ou une chaine alkylidène en C₁₋₄ où une unité méthylène de L est le cas échéant remplacée par -CO₂-, -C(O)NR-, -C(O)-, - N(R)-, ou -O-;
R est hydrogène, ou un radical le cas échéant substitué, choisi parmi aliphatique en C₁₋₄, hétérocyclyle de 3-6 membres ayant 1-2 hétéroatomes indépendamment choisis parmi azote, oxygène et soufre, phényle, ou hétéroaryle de 5-6 membres, ayant 1-4 hétéroatomes indépendamment choisis parmi azote, oxygène et soufre, le substituant facultatif étant défini à la revendication 1, et
T est une chaine alkylidène en C₁₋₄.

3. Composé selon la revendication 2, où :
R¹ est choisi parmi R, -SO₂R, ou -C(O)R ;
R est hydrogène, ou un radical aliphatique en C₁₋₄ le cas échéant substitué ;
R³ est choisi parmi hydrogène, -CH₂CN, - CH₂C(O)NH₂, -CH₂CO₂H, propyle, -CH₂CH₂=CH₂, isopropyle, - (CH₂) ₃CN, -CH₂OEt, -CH₂CF₃, isobutyle, cyclopropylméthyle, -CH₂CH₂N(Me)₂, -CH₂CH(OEt)₂, éthyle, -CH₂C(O)NH-t-butyl, ou benzyle le cas échéant substitué ou -CH₂C(O)NH-phényle ;
tout carbone substituable sur le cycle benzo est indépendamment et le cas échéant substitué par chloro, bromo, méthyle, -CF₃, nitro, t-butyle, méthoxy, -CO₂Me, hydroxy, amino ou -OCH₂CN.

4. Composé selon la revendication 1, où ledit composé est choisi parmi les composés du tableau 1 suivant :
| **No. I-** | **Structure** | **No. I-** | **Structure** |
|---|---|---|---|
| **25** | 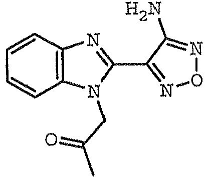 | **26** | 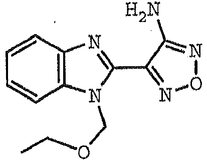 |
| **27** | 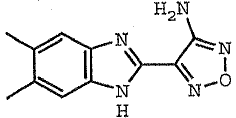 | **28** | 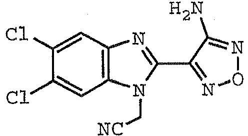 |
| **29** | 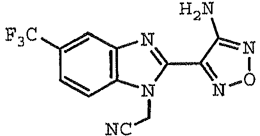 | **30** | 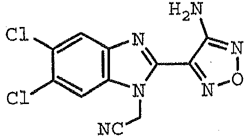 |
| **31** | 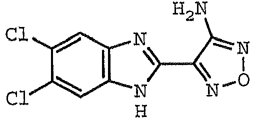 | **32** | 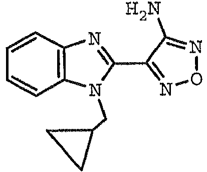 |
| | | **34** | 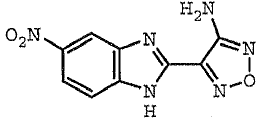 |
| **35** | 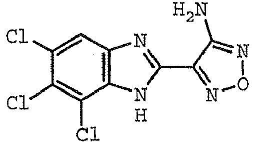 | **36** | 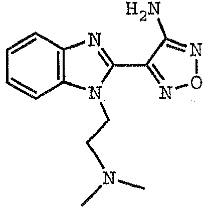 |
| **37** | 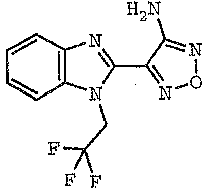 | | |
| **39** | 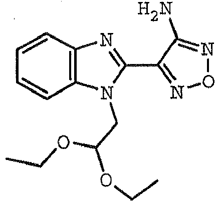 | **40** | 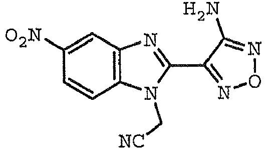 |
| **41** | 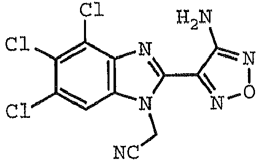 | **42** | 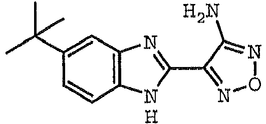 |
| **43** | 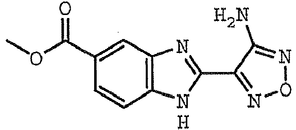 | **44** | 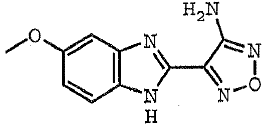 |
| **45** | 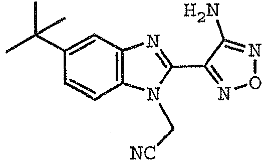 | **46** | 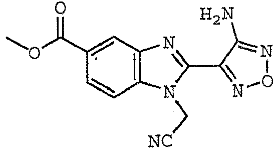 |
| **47** | 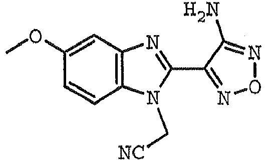 | **48** | 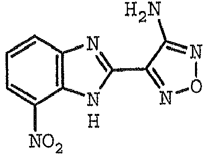 |
| **49** | 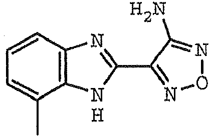 | **50** | 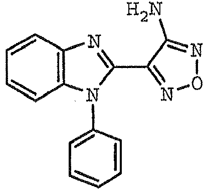 |
| **51** | 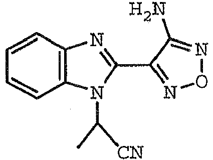 | **52** | 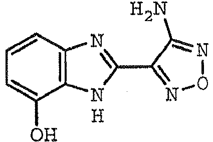 |
| **53** | 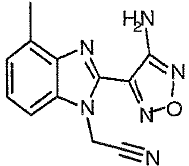 | **54** | 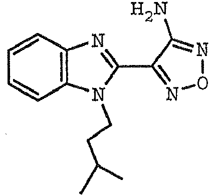 |
| | | **56** | 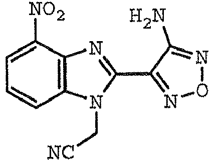 |
| **57** | 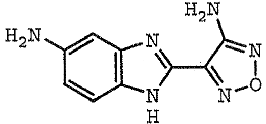 | **58** | 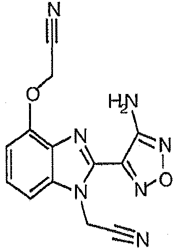 |
| **59** | 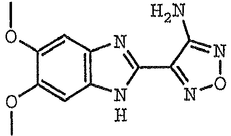 | **60** | 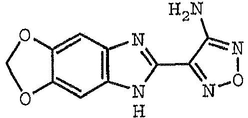 |
| **67** | 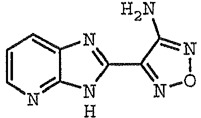 | **68** | 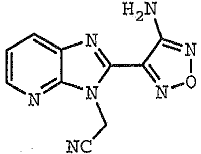 |
| **69** | 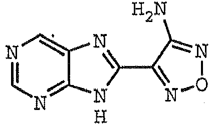 | | |
| | | **76** | 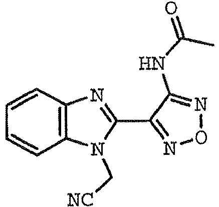 |
| | | **80** | 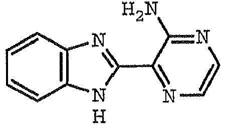 |
| **83** | 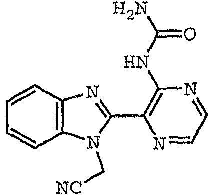 | | |
| **85** | 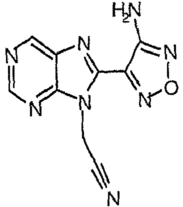 | | |
| | | **88** | 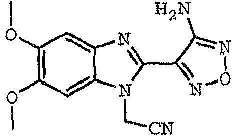 |
| | | **90** | 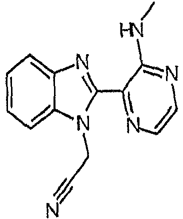 |
| | | **92** | 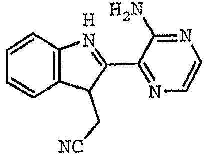 |
| **93** | 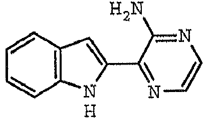 | **94** | 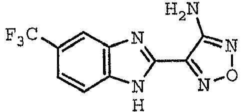 |
| **101** | 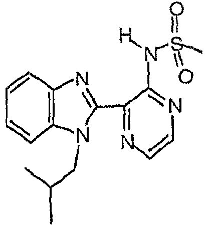 | **102** | 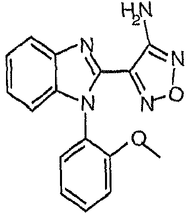 |
| **103** | 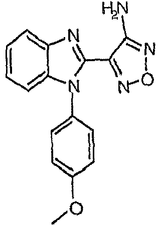 | **104** | 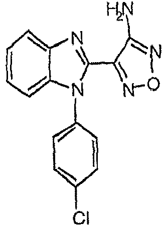 |
| **105** | 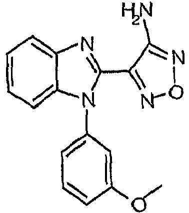 | | |
| **107** | 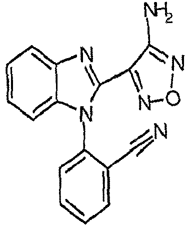 | **108** | 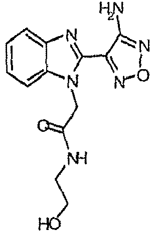 |
| **109** | 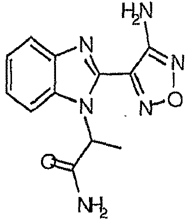 | | |
| **111** | 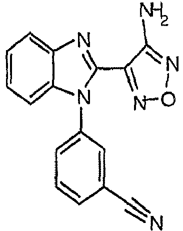 | | |
| **113** | 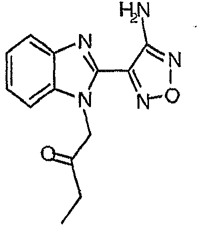 | **114** | 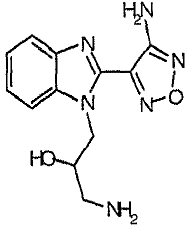 |
| **115** | 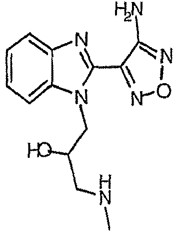 | **116** | 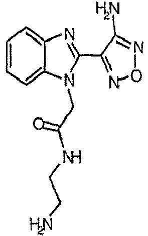 |
| **117** | 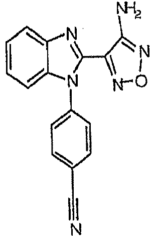 | **118** | 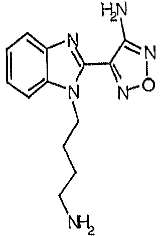 |
| **119** | 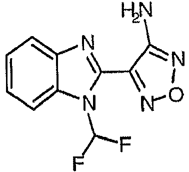 | **120** | 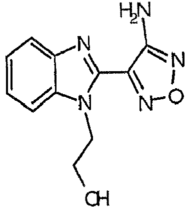 |
| **121** | 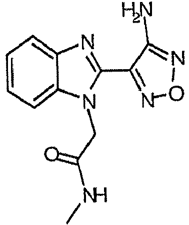 | **122** | 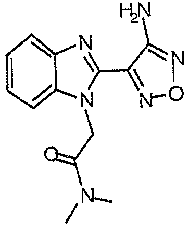 |
| **123** | 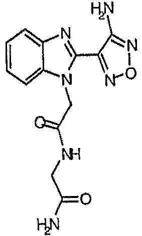 | **124** | 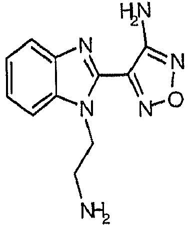 |
| **125** | 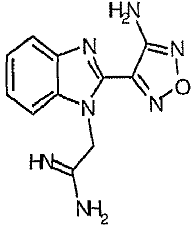 | **126** | 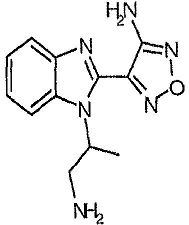 |
| **127** | 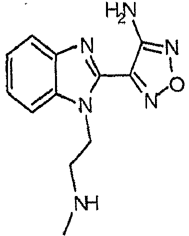 | **128** | 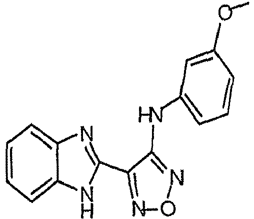 |
| **129** | 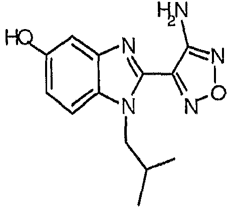 | **130** | 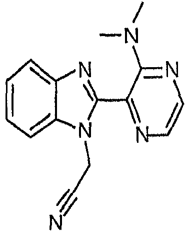 |
| **131** | 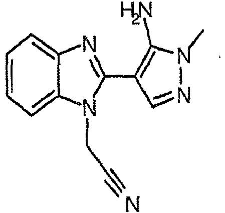 | **132** | 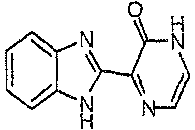 |
| **133** | 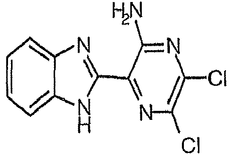 | **134** | 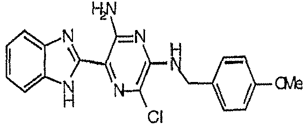 |
| **135** | 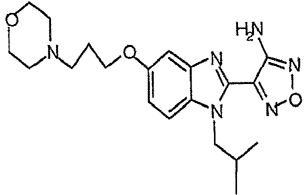 | | |

5. Composition comprenant une quantité efficace d'un composé selon la revendication 1, et un support pharmaceutiquement acceptable.

6. Composition selon la revendication 5, comprenant en outre, un agent thérapeutique supplémentaire.

7. Composition selon la revendication 5 ou 6 ou composé selon les revendications 1 à 4, à utiliser dans le traitement ou pour diminuer le sévérité d'une maladie, un désordre ou un état choisi parmi un désordre neurologique, l'allergie, l'asthme, le diabète, la maladie d'Alzheimer, la maladie de Huntington, la maladie de Parkinson, la démence associée au SIDA, la sclérose latérale amyotrophique (AML ou maladie de Lou Gehrig), la sclérose en plaques, la schizophrénie, l'hypertrophie cardiomyocytaire, la reperfusion/ ischémie, l'accident vasculaire cérébral ou la calvitie.

8. Composé ou composition à utiliser selon la revendication 7, où ladite maladie est choisie parmi un désordre neurologique.

9. Composé ou composition à utiliser selon la revendication 7, où ladite maladie est l'accident vasculaire cérébral.

10. Composé ou composition à utiliser selon la revendication 7, où ladite maladie est choisie parmi un rejet de transplantation, des allergies, la polyarthrite rhumatoïde ou la leucémie.

11. Composé ou composition à utiliser selon la revendication 10, où ladite maladie est choisie parmi un rejet de transplantation ou la polyarthrite rhumatoïde.

12. Procédé *in vitro* d'inhibition de la kinase GSK-3 ou Lck dans un échantillon biologique avec un composé selon les revendications 1 à 4.
